# EUROPEAN PATENT SPECIFICATION

(11) **EP 3 148 574 B1**
(45) Date of publication and mention of the grant of the patent: **14.04.2021**
(21) Application number: 15799654.7
(22) Date of filing: 28.05.2015
(51) Int. Cl.: A61K 39/00, A61K 39/29, A61P 1/16, A61P 31/20, A61K 35/16

(54) **PURIFIED COMPOSITIONS OF IVIG AND KH PROTEINS FOR MODULATING LYMPHOCYTES AND TREATING HEPATITIS B VIRUS**
GEREINIGTE ZUSAMMENSETZUNGEN VON IVIG- UND KH-PROTEINEN ZUR MODULATION VON LYMPHOZYTEN UND ZUR BEHANDLUNG VON HEPATITIS-B-VIRUS
COMPOSITIONS PURIFIÉES DE PROTÉINES IVIG ET KH POUR LA MODULATION DES LYMPHOCYTES ET LE TRAITEMENT CONTRE LE VIRUS DE L'HÉPATITE B

(30) Priority: 28.05.2014 US 201462003664 P
(43) Date of publication of application: 05.04.2017
(73) Proprietor: Rare Antibody Antigen Supply, Inc., Agoura Hills, CA 91301 (US)
(72) Inventor: HOANG, Kieu, Westlake Village, CA 91361 (US)
(74) Representative: AWA Denmark A/S
(86) International application number: PCT/US2015/032807
(87) International publication number: WO 2015/184050

(56) References cited:
- WO-A1-2009/117085
- US-A- 5 190 752
- US-A1- 2007 083 334
- US-A1- 2010 047 249
- US-A1- 2010 074 885
- US-A1- 2013 190 477
- US-A1- 2014 141 488
- ECHEVARRIA J M ET AL: "Laboratory diagnosis and molecular epidemiology of an outbreak of hepatitis C virus infection among recipients of human intravenous immunoglobulin in Spain", TRANSFUSION, AMERICAN ASSOCIATION OF BLOOD BANKS, BETHESDA, MD, US, vol. 36, no. 8, 1 August 1996 (1996-08-01) , pages 725-730, XP009193488, ISSN: 0041-1132
- BREUER G S ET AL: "A pregnant woman with hepatitis A and Guillain-Barré", JOURNAL OF CLINICAL GASTROENTEROLOGY, RAVEN PRESS LTD., NEW YORK, NY, US, vol. 32, no. 2, 1 February 2001 (2001-02-01), pages 179-180, XP009193483, ISSN: 0192-0790
- WU, W ET AL.: 'Fibrinogen Beta Chain Isoform 1 Preproprotein [Homo Sapiens].', [Online] 10 May 2014, XP055238992 Retrieved from the Internet: <URL:http://www.ncbi.nlm.nih.gov/protein/70 906435?sat=18&satkey=12940820.>
- HEWING, B ET AL.: 'Apolipoprotein A1 Preproprotein [Homo Sapiens].', [Online] 24 May 2014, XP055238994 Retrieved from the Internet: <URL:http://www.ncbi.nlm.nih.gov/protein/45 57321?sat=18&satkey=18606193.>

## Description

### Field of the Invention:

The invention relates to isolated purified IVIG plasma products comprising an isolated purified protein complex and isolated purified IVIG plasma products for use in the treatment of hepatitis B.

### Background of the Invention:

The practice of administering antibodies or antitoxins acquired from exposed or vaccinated individuals or animals to a patient susceptible to the disease in question has been the underlying medical practice involved in passive immunotherapy since the late 1800s. Since the 1940s human albumin and other therapeutic proteins have been extracted from plasma to address specific clinical needs.

In comparison to most antibiotics, antibody-based therapies that use human antibodies have low toxicity and high specificity. The high specificity means that the antibody targets only the disease-causing microorganism that causes disease without affecting the host's endogenous organisms, therefore minimizing adverse reactions and the chance of the development of resistant organisms. This also means, however, that more than one antibody preparation may be required to target micro-organisms with high antigenic variation. Combination plasma products containing antibodies specific for a variety of diseases and afflictions, as well as therapies for administering the same, are therefore desired for addressing a range of diseases while minimizing damage to healthy cells.

The identification of various fractions of human plasma as well as the proteins residing therein and the unique characteristics of those proteins, has resulted in new life saving treatments for a variety of chronic and acute, hereditary and acquired diseases. Improvements in the manufacturing processes that ensure product safety and efficacy, and the identification of specific clinical applications require new advances in the technology.

Various techniques to remove protein aggregates, such as chromatography, have made some products more tolerable when administered intravenously and the addition of virus removal/inactivation steps has made the products essentially free of lipid and nonlipid enveloped viruses.

Intravenous immunoglobin (IVIG) is a blood product generally administered intravenously. IVIG is administered to patients with immunodeficiencies and its benefits for secondary ailments related to immunodeficiencies has made it an increasingly appealing first or second line treatment.

IVIG is manufactured from pooled human plasma and contains white blood cells called lymphocytes. A lymphocyte is any of three types of immune cells including: (1) natural killer cells (NK cells, which function in cell-mediated, cytotoxic innate immunity), (2) T cells (for cell-mediated, cytotoxic adaptive immunity), and (3) B cells (for humoral, antibody-driven adaptive immunity).

Antibodies are produced by the B-cells and plasma cells after exposure to antigens. They can be either immunoglobin G (IgG), IgA, IgM, IgE, or IgD, but in the case of hyper-immunes, IgG are the antibodies of interest. IgG consists of four polypeptide chains, two pairs of polypeptide chains, two pairs of heavy and light chains in a Y-shaped arrangement. The top ends of the IgG molecule, Fab or antibody binding region, are created from one heavy and one light chain, forming the antigen binding site. This fragment variable (Fv) region contains various amino acid combinations, which makes each antibody unique. Importantly, purified IVIG intravenous hyperimmune products contain human IgG protein, of which at least 96% is IgG containing specific antibodies against the specific antigen. Significantly, since the mid to late 2000s the need for more efficient manufacture of isolated purified IVIG has increased dramatically. Products regulating the percentages of B and T cells to target specific ailments and disease are also desirable.

Echevarria J. M. et al. (Transfusion, American Association of Blood Banks, Bethesda, MD, US, vol 36, no. 8, 1 August 1996, pages 725-730) reports the passive transfer of antibodies to hepatitis C virus (HCV) after infusion of human intravenous immunoglobulin (IVIG) in patients with humoral immunodeficiencies.

Breuer G. S. et al. (Journal of Clinical Gastoenterology, Raven Press LTD., New York, NY, US, vol. 32, no. 2, 1 February 2001, pages 179-180) reports on the treatment of Guillain-Barre syndrome (GBS) after hepatitis A with intravenous immunoglobulin.

WO 2009/117085 concerns the combined use of an immune globulin composition and hyaluronidase for subcutaneous administration, whereby the dosing regimen is the same as for intravenous administration of the same dosage for treatment of an IG-treatable disease or condition.

US 2010/047249 A1 concerns compositions for inhibiting Fcγ-mediated phagocytosis with reduced immunoglobulin preparations, whereby said reduced immunoglobulin inhibitor of Fcy-mediated phagocytosis comprises a reduced IVIG.

### Summary of the Invention:

The invention is defined according to accompanying claims 1 - 18.

The present disclosure relates to isolated purified human immunoglobulin plasma products, methods of their manufacture and their use in the treatment of hepatitis B virus. The purified human immunoglobulin plasma products are useful in treating a variety of chronic and acute, hereditary and acquired diseases by regulating the levels of immune cells and their related proteins in the treated subject.

In embodiments of the invention various purified blood plasma products are for use in the treatment of viral infections such as HBV.

Disclosed herein is the regulation of B and T cell levels in the peripheral blood and organs of a treated individual through prophylactic or therapeutic administration of purified blood plasma products.

Also disclosed herein is the regulation of granulocyte and macrophage levels in the peripheral blood and organs of a treated individual through prophylactic or therapeutic administration of purified blood plasma products.

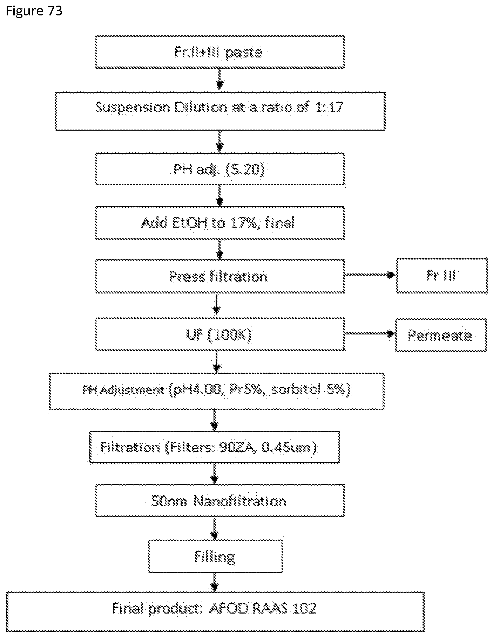

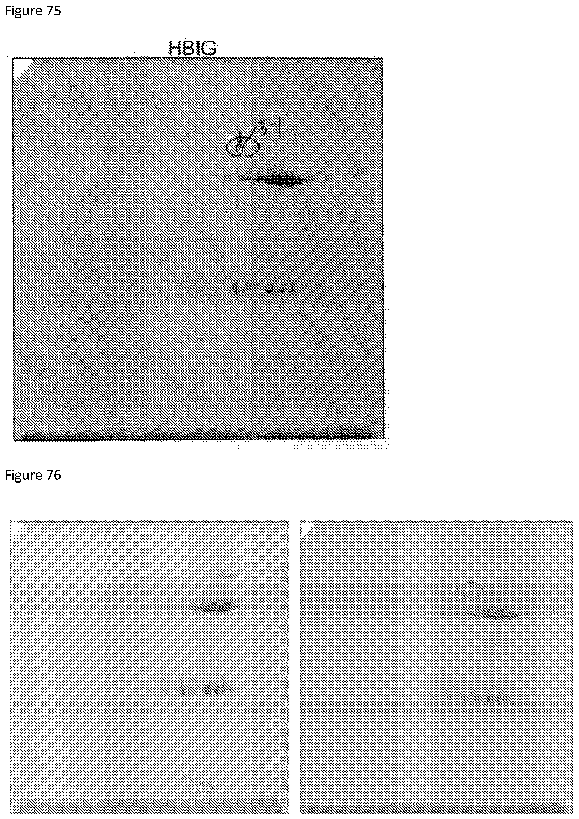

In the present disclosure a purified protein complex is obtained by purifying intravenous immunoglobulin G (IVIG) from human plasma fraction II+III paste. **FIG. 73****.** In addition to the main component of immunoglobulin, analysis of the protein complex has shown the product to contain the following proteins: 120/E19 IGHV4-31, IGHG1 44kDa, 191/H18 IGHV4 31, IGHG1 32kDa, IGHG1 putative uncharacterized protein, DKFZp686G11190, and KH proteins 33-37. **FIG 75**. The combination of KH proteins 33-37 with a concentration of 30% has been found to very effective against viruses such as H1N1, H5N1, foot and mouth disease, and to stop hepatitis B viral DNA replication.

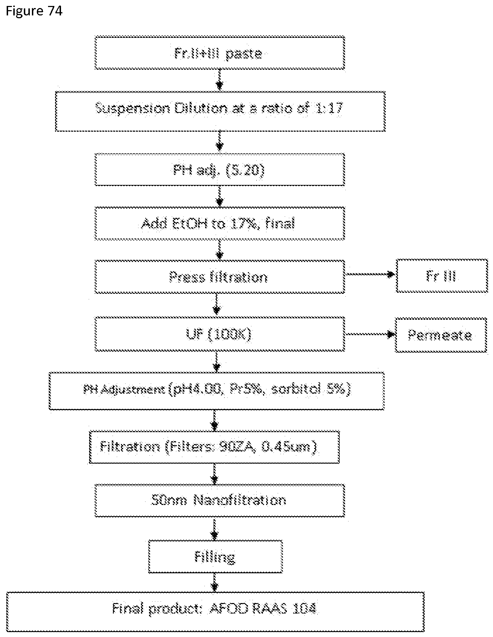

In the present disclosure a purified protein complex is obtained by purifying hepatitis B immune globulin (HBIG) from human plasma fraction II+III of donors having high antibody levels of the hepatitis B surface antigen. **FIG. 74****.** In addition to immunoglobulin proteins, HBIG contains the protein TF serotransferrin (sequence no. 197/H24). This complex contains KH proteins 22-37 and has been found to be effective in stopping hepatitis B viral DNA replication.

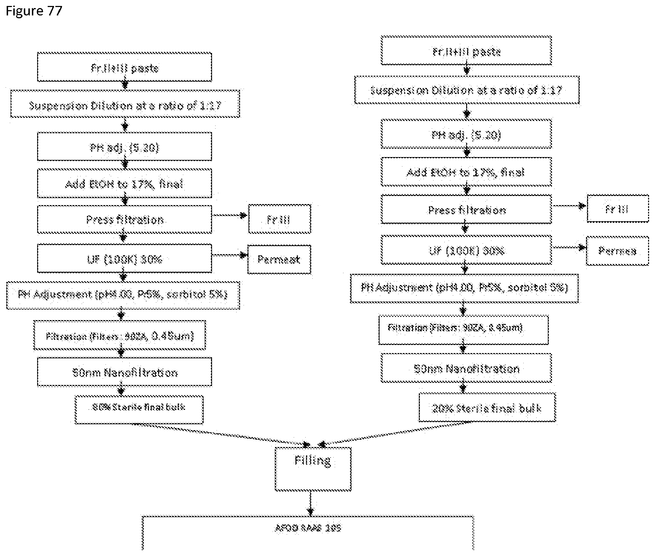

In yet another embodiment a purified protein complex is formulated to combat the scarcity of the hepatitis B antibody. **FIGs 77-78****.** This purified protein complex is a combination of 80% purified normal immunoglobulin and 20% purified hepatitis B immune globulin containing high levels of hepatitis B antibodies. In this embodiment both of the products have a concentration by ultra filtration of at least 30%. The purified protein complex of this embodiment contains proteins designated as KH22-37 and KH51. Additional information regarding KH designated proteins is included herein.

In a related embodiment a method of manufacture for a purified protein complex comprises: following manufacturing protocol to separately manufacture normal immunoglobulin and hepatitis B antibody up to the step of obtaining non-sterile final bulk for both products, taking 80% normal immunoglobulin non-sterile final bulk and mixing with 20% hepatitis B antibody non-sterile final bulk, and performing sterile filtration for filling the final product. **FIG 77****.**

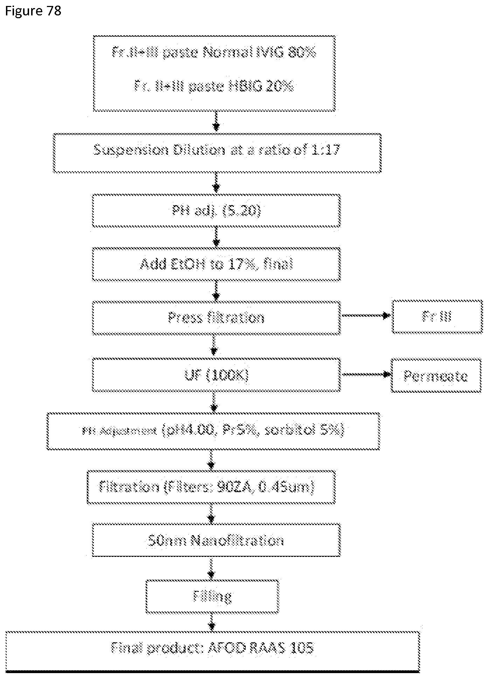

In another embodiment the method of manufacture for a purified protein complex comprises: taking 80% of normal immunoglobulin fraction II+III and 20% hepatitis B antibody fraction II+III, and dissolving the fractions together in a process tank for production of the normal immunoglobulin until the final product is filled. **FIG 78****.**

The present disclosure includes purified protein complexes containing various proteins having unique characteristics useful in treating infection and disease. A "KH" designation has been assigned to certain proteins contained in the purified protein complexes. Those designations, as well as additional information corresponding to those proteins is found in the figures below.

538 functions have been identified for the 55 KH proteins, which provide them with unique characteristics for treating a wide range of disease, infection, and other cellular disturbances as expressed in the present disclosure.

| **Number** | **GI code** | **Fraction - P (process), C (component), F (function)** | **Sequence name** | **Sequence desc.** |
|---|---|---|---|---|
| KH 1 | 21749960 | cryopaste | gi\|2174996 0 | dock4_humandedicator of cytokinesis protein 4 os=homo sapiens gn=dock4 pe=1 sv=3 |
| gi\|21749960 | dock4_humandedicator of cytokinesis protein 4 os=homo sapiens gn=dock4 pe=1 sv=3 | F | GO:00051 02 | receptor binding |
| gi\|21749960 | dock4_humandedicator of cytokinesis protein 4 os=homo sapiens gn=dock4 pe=1 sv=3 | P | GO:00435 47 | positive regulation of GTPase activity |
| gi\|21749960 | dock4_humandedicator of cytokinesis protein 4 os=homo sapiens gn=dock4 pe=1 sv=3 | P | GO:00164 77 | cell migration |
| gi\|21749960 | dock4_humandedicator of cytokinesis protein 4 os=homo sapiens gn=dock4 pe=1 sv=3 | P | GO:00071 65 | signal transduction |
| gi\|21749960 | dock4_humandedicator of cytokinesis protein 4 os=homo sapiens gn=dock4 pe=1 sv=3 | P | GO:00069 35 | chemotaxis |
| gi\|21749960 | dock4_humandedicator of cytokinesis protein 4 os=homo sapiens gn=dock4 pe=1 sv=3 | C | GO:00057 37 | cytoplasm |
| gi\|21749960 | dock4_humandedicator of cytokinesis protein 4 os=homo sapiens gn=dock4 pe=1 sv=3 | F | GO:00050 83 | small GTPase regulator activity |
| gi\|21749960 | dock4_humandedicator of cytokinesis protein 4 os=homo sapiens gn=dock4 pe=1 sv=3 | F | GO:00199 04 | protein domain specific binding |
| gi\|21749960 | dock4_humandedicator of cytokinesis protein 4 os=homo sapiens gn=dock4 pe=1 sv=3 | P | GO:00485 83 | regulation of response to stimulus |
| gi\|21749960 | dock4_humandedicator of cytokinesis protein 4 os=homo sapiens gn=dock4 pe=1 sv=3 | F | GO:00050 96 | GTPase activator activity |
| gi\|21749960 | dock4_humandedicator of cytokinesis protein 4 os=homo sapiens gn=dock4 pe=1 sv=3 | F | GO:00510 20 | GTPase binding |
| gi\|21749960 | dock4_humandedicator of cytokinesis protein 4 os=homo sapiens gn=dock4 pe=1 sv=3 | C | GO:00160 20 | membrane |
| KH 2 | 215415640 | cryopaste | gi\|2154156 40 | apoa1_humanapolipoprotein a-i os=homo sapiens gn=apoa1 pe=1 sv=1 |
| gi\|21541564 0 | apoa1_humanapolipoprotein a-i os=homo sapiens gn=apoa1 pe=1 sv=1 | P | GO:00705 08 | cholesterol import |
| gi\|21541564 0 | apoa1_humanapolipoprotein a-i os=homo sapiens gn=apoa1 pe=1 sv=1 | C | GO:00301 39 | endocytic vesicle |
| gi\|21541564 0 | apoa1_humanapolipoprotein a-i os=homo sapiens gn=apoa1 pe=1 sv=1 | P | GO:00507 28 | negative regulation of inflammatory response |
| gi\|21541564 0 | apoa1_humanapolipoprotein a-i os=homo sapiens gn=apoa1 pe=1 sv=1 | P | GO:00333 44 | cholesterol efflux |
| gi\|21541564 0 | apoa1_humanapolipoprotein a-i os=homo sapiens gn=apoa1 pe=1 sv=1 | P | GO:00341 15 | negative regulation of heterotypic cell-cell adhesion |
| gi\|21541564 0 | apoa1_humanapolipoprotein a-i os=homo sapiens gn=apoa1 pe=1 sv=1 | P | GO:00182 06 | peptidyl- methionine modification |
| gi\|21541564 0 | apoa1_humanapolipoprotein a-i os=homo sapiens gn=apoa1 pe=1 sv=1 | P | GO:00421 57 | lipoprotein metabolic process |
| gi\|21541564 0 | apoa1_humanapolipoprotein a-i os=homo sapiens gn=apoa1 pe=1 sv=1 | P | GO:00436 91 | reverse cholesterol transport |
| gi\|21541564 0 | apoa1_humanapolipoprotein a-i os=homo sapiens gn=apoa1 pe=1 sv=1 | F | GO:00055 43 | phospholipid binding |
| gi\|21541564 0 | apoa1_humanapolipoprotein a-i os=homo sapiens gn=apoa1 pe=1 sv=1 | P | GO:00027 40 | negative regulation of cytokine secretion involved in immune response |
| gi\|21541564 0 | apoa1_humanapolipoprotein a-i os=homo sapiens gn=apoa1 pe=1 sv=1 | P | GO:00082 03 | cholesterol metabolic process |
| gi\|21541564 0 | apoa1_humanapolipoprotein a-i os=homo sapiens gn=apoa1 pe=1 sv=1 | P | GO:00507 13 | negative regulation of interleukin-1 beta secretion |
| gi\|21541564 0 | apoa1_humanapolipoprotein a-i os=homo sapiens gn=apoa1 pe=1 sv=1 | P | GO:00181 58 | protein oxidation |
| gi\|21541564 0 | apoa1_humanapolipoprotein a-i os=homo sapiens gn=apoa1 pe=1 sv=1 | P | GO:00066 56 | phosphatidylcholine biosynthetic process |
| gi\|21541564 0 | apoa1_humanapolipoprotein a-i os=homo sapiens gn=apoa1 pe=1 sv=1 | F | GO:00015 40 | beta-amyloid binding |
| gi\|21541564 0 | apoa1_humanapolipoprotein a-i os=homo sapiens gn=apoa1 pe=1 sv=1 | F | GO:00602 28 | phosphatidylcholine-sterol O-acyltransferase activator activity |
| gi\|21541564 0 | apoa1_humanapolipoprotein a-i os=homo sapiens gn=apoa1 pe=1 sv=1 | P | GO:00426 32 | cholesterol homeostasis |
| gi\|21541564 0 | apoa1_humanapolipoprotein a-i os=homo sapiens gn=apoa1 pe=1 sv=1 | F | GO:00154 85 | cholesterol binding |
| gi\|21541564 0 | apoa1_humanapolipoprotein a-i os=homo sapiens gn=apoa1 pe=1 sv=1 | P | GO:00420 60 | wound healing |
| gi\|21541564 0 | apoa1_humanapolipoprotein a-i os=homo sapiens gn=apoa1 pe=1 sv=1 | F | GO:00341 91 | apolipoprotein A-I receptor binding |
| gi\|21541564 0 | apoa1_humanapolipoprotein a-i os=homo sapiens gn=apoa1 pe=1 sv=1 | F | GO:00428 02 | identical protein binding |
| gi\|21541564 0 | apoa1_humanapolipoprotein a-i os=homo sapiens gn=apoa1 pe=1 sv=1 | P | GO:00109 03 | negative regulation of very-low-density lipoprotein particle remodeling |
| gi\|21541564 0 | apoa1_humanapolipoprotein a-i os=homo sapiens gn=apoa1 pe=1 sv=1 | P | GO:00108 04 | negative regulation of tumor necrosis factor-mediated signaling pathway |
| gi\|21541564 0 | apoa1_humanapolipoprotein a-i os=homo sapiens gn=apoa1 pe=1 sv=1 | P | GO:00343 80 | high-density lipoprotein particle assembly |
| gi\|21541564 0 | apoa1_humanapolipoprotein a-i os=homo sapiens gn=apoa1 pe=1 sv=1 | P | GO:00071 86 | G-protein coupled receptor signaling pathway |
| gi\|21541564 0 | apoa1_humanapolipoprotein a-i os=homo sapiens gn=apoa1 pe=1 sv=1 | P | GO:00508 21 | protein stabilization |
| gi\|21541564 0 | apoa1_humanapolipoprotein a-i os=homo sapiens gn=apoa1 pe=1 sv=1 | C | GO:00343 61 | very-low-density lipoprotein particle |
| gi\|21541564 0 | apoa1_humanapolipoprotein a-i os=homo sapiens gn=apoa1 pe=1 sv=1 | P | GO:00324 88 | Cdc42 protein signal transduction |
| gi\|21541564 0 | apoa1_humanapolipoprotein a-i os=homo sapiens gn=apoa1 pe=1 sv=1 | P | GO:00603 54 | negative regulation of cell adhesion molecule production |
| gi\|21541564 0 | apoa1_humanapolipoprotein a-i os=homo sapiens gn=apoa1 pe=1 sv=1 | P | GO:00550 91 | phospholipid homeostasis |
| gi\|21541564 0 | apoa1_humanapolipoprotein a-i os=homo sapiens gn=apoa1 pe=1 sv=1 | P | GO:00108 73 | positive regulation of cholesterol esterification |
| gi\|21541564 0 | apoa1_humanapolipoprotein a-i os=homo sapiens gn=apoa1 pe=1 sv=1 | F | GO:00171 27 | cholesterol transporter activity |
| gi\|21541564 0 | apoa1_humanapolipoprotein a-i os=homo sapiens gn=apoa1 pe=1 sv=1 | F | GO:00198 99 | enzyme binding |
| gi\|21541564 0 | apoa1_humanapolipoprotein a-i os=homo sapiens gn=apoa1 pe=1 sv=1 | F | GO:00706 53 | high-density lipoprotein particle receptor binding |
| gi\|21541564 0 | apoa1_humanapolipoprotein a-i os=homo sapiens gn=apoa1 pe=1 sv=1 | P | GO:00703 28 | triglyceride homeostasis |
| gi\|21541564 0 | apoa1_humanapolipoprotein a-i os=homo sapiens gn=apoa1 pe=1 sv=1 | C | GO:00343 66 | spherical high-density lipoprotein particle |
| gi\|21541564 0 | apoa1_humanapolipoprotein a-i os=homo sapiens gn=apoa1 pe=1 sv=1 | P | GO:00337 00 | phospholipid efflux |
| gi\|21541564 0 | apoa1_humanapolipoprotein a-i os=homo sapiens gn=apoa1 pe=1 sv=1 | P | GO:00513 45 | positive regulation of hydrolase activity |
| KH 3 | 215415638 | Fr III | gi\|2154156 38 | apoa1_humanapolipoprotein a-i os=homo sapiens gn=apoa1 pe=1 sv=1 |
| gi\|21541563 8 | apoa1_humanapolipoprotein a-i os=homo sapiens gn=apoa1 pe=1 sv=1 | P | GO:00705 08 | cholesterol import |
| gi\|21541563 8 | apoa1_humanapolipoprotein a-i os=homo sapiens gn=apoa1 pe=1 sv=1 | C | GO:00301 39 | endocytic vesicle |
| gi\|21541563 8 | apoa1_humanapolipoprotein a-i os=homo sapiens gn=apoa1 pe=1 sv=1 | P | GO:00507 28 | negative regulation of inflammatory response |
| gi\|21541563 8 | apoa1_humanapolipoprotein a-i os=homo sapiens gn=apoa1 pe=1 sv=1 | P | GO:00333 44 | cholesterol efflux |
| gi\|21541563 8 | apoa1_humanapolipoprotein a-i os=homo sapiens gn=apoa1 pe=1 sv=1 | P | GO:00341 15 | negative regulation of heterotypic cell-cell adhesion |
| gi\|21541563 8 | apoa1_humanapolipoprotein a-i os=homo sapiens gn=apoa1 pe=1 sv=1 | P | GO:00182 06 | peptidyl- methionine modification |
| gi\|21541563 8 | apoa1_humanapolipoprotein a-i os=homo sapiens gn=apoa1 pe=1 sv=1 | P | GO:00421 57 | lipoprotein metabolic process |
| gi\|21541563 8 | apoa1_humanapolipoprotein a-i os=homo sapiens gn=apoa1 pe=1 sv=1 | P | GO:00436 91 | reverse cholesterol transport |
| gi\|21541563 8 | apoa1_humanapolipoprotein a-i os=homo sapiens gn=apoa1 pe=1 sv=1 | F | GO:00055 43 | phospholipid binding |
| gi\|21541563 8 | apoa1_humanapolipoprotein a-i os=homo sapiens gn=apoa1 pe=1 sv=1 | P | GO:00027 40 | negative regulation of cytokine secretion involved in immune response |
| gi\|21541563 8 | apoa1_humanapolipoprotein a-i os=homo sapiens gn=apoa1 pe=1 sv=1 | P | GO:00082 03 | cholesterol metabolic process |
| gi\|21541563 8 | apoa1_humanapolipoprotein a-i os=homo sapiens gn=apoa1 pe=1 sv=1 | P | GO:00507 13 | negative regulation of interleukin-1 beta secretion |
| gi\|21541563 8 | apoa1_humanapolipoprotein a-i os=homo sapiens gn=apoa1 pe=1 sv=1 | P | GO:00181 58 | protein oxidation |
| gi\|21541563 8 | apoa1_humanapolipoprotein a-i os=homo sapiens gn=apoa1 pe=1 sv=1 | P | GO:00066 56 | phosphatidylcholine biosynthetic process |
| gi\|21541563 8 | apoa1_humanapolipoprotein a-i os=homo sapiens gn=apoa1 pe=1 sv=1 | F | GO:00015 40 | beta-amyloid binding |
| gi\|21541563 8 | apoa1_humanapolipoprotein a-i os=homo sapiens gn=apoa1 pe=1 sv=1 | F | GO:00602 28 | phosphatidylcholine-sterol O-acyltransferase activator activity |
| gi\|21541563 8 | apoa1_humanapolipoprotein a-i os=homo sapiens gn=apoa1 pe=1 sv=1 | P | GO:00426 32 | cholesterol homeostasis |
| gi\|21541563 8 | apoa1_humanapolipoprotein a-i os=homo sapiens gn=apoa1 pe=1 sv=1 | F | GO:00154 85 | cholesterol binding |
| gi\|21541563 8 | apoa1_humanapolipoprotein a-i os=homo sapiens gn=apoa1 pe=1 sv=1 | P | GO:00420 60 | wound healing |
| gi\|21541563 8 | apoa1_humanapolipoprotein a-i os=homo sapiens gn=apoa1 pe=1 sv=1 | F | GO:00341 91 | apolipoprotein A-I receptor binding |
| gi\|21541563 8 | apoa1_humanapolipoprotein a-i os=homo sapiens gn=apoa1 pe=1 sv=1 | F | GO:00428 02 | identical protein binding |
| gi\|21541563 8 | apoa1_humanapolipoprotein a-i os=homo sapiens gn=apoa1 pe=1 sv=1 | P | GO:00109 03 | negative regulation of very-low-density lipoprotein particle remodeling |
| gi\|21541563 8 | apoa1_humanapolipoprotein a-i os=homo sapiens gn=apoa1 pe=1 sv=1 | P | GO:00108 04 | negative regulation of tumor necrosis factor-mediated signaling pathway |
| gi\|21541563 8 | apoa1_humanapolipoprotein a-i os=homo sapiens gn=apoa1 pe=1 sv=1 | P | GO:00343 80 | high-density lipoprotein particle assembly |
| gi\|21541563 8 | apoa1_humanapolipoprotein a-i os=homo sapiens gn=apoa1 pe=1 sv=1 | P | GO:00071 86 | G-protein coupled receptor signaling pathway |
| gi\|21541563 8 | apoa1_humanapolipoprotein a-i os=homo sapiens gn=apoa1 pe=1 sv=1 | P | GO:00508 21 | protein stabilization |
| gi\|21541563 8 | apoa1_humanapolipoprotein a-i os=homo sapiens gn=apoa1 pe=1 sv=1 | C | GO:00343 61 | very-low-density lipoprotein particle |
| gi\|21541563 8 | apoa1_humanapolipoprotein a-i os=homo sapiens gn=apoa1 pe=1 sv=1 | P | GO:00324 88 | Cdc42 protein signal transduction |
| gi\|21541563 8 | apoa1_humanapolipoprotein a-i os=homo sapiens gn=apoa1 pe=1 sv=1 | P | GO:00603 54 | negative regulation of cell adhesion molecule production |
| gi\|21541563 8 | apoa1_humanapolipoprotein a-i os=homo sapiens gn=apoa1 pe=1 sv=1 | P | GO:00550 91 | phospholipid homeostasis |
| gi\|21541563 8 | apoa1_humanapolipoprotein a-i os=homo sapiens gn=apoa1 pe=1 sv=1 | P | GO:00108 73 | positive regulation of cholesterol esterification |
| gi\|21541563 8 | apoa1_humanapolipoprotein a-i os=homo sapiens gn=apoa1 pe=1 sv=1 | F | GO:00171 27 | cholesterol transporter activity |
| gi\|21541563 8 | apoa1_humanapolipoprotein a-i os=homo sapiens gn=apoa1 pe=1 sv=1 | F | GO:00198 99 | enzyme binding |
| gi\|21541563 8 | apoa1_humanapolipoprotein a-i os=homo sapiens gn=apoa1 pe=1 sv=1 | F | GO:00706 53 | high-density lipoprotein particle receptor binding |
| gi\|21541563 8 | apoa1_humanapolipoprotein a-i os=homo sapiens gn=apoa1 pe=1 sv=1 | P | GO:00703 28 | triglyceride homeostasis |
| gi\|21541563 8 | apoa1_humanapolipoprotein a-i os=homo sapiens gn=apoa1 pe=1 sv=1 | C | GO:00343 66 | spherical high-density lipoprotein particle |
| gi\|21541563 8 | apoa1_humanapolipoprotein a-i os=homo sapiens gn=apoa1 pe=1 sv=1 | P | GO:00337 00 | phospholipid efflux |
| gi\|21541563 8 | apoa1_humanapolipoprotein a-i os=homo sapiens gn=apoa1 pe=1 sv=1 | P | GO:00513 45 | positive regulation of hydrolase activity |
| KH 4 | 40044478 | Fr III | | |
| KH 5 | 194383496 | Fr III | gi\|1943834 96 | thrb_humanprothrombin os=homo sapiens gn=f2 pe=1 sv=2 |
| gi\|19438349 5 | thrb_humanprothrombin os=homo sapiens gn=f2 pe=1 sv=2 | P | GO:00328 79 | regulation of localization |
| gi\|19438349 6 | thrb_humanprothrombin os=homo sapiens gn=f2 pe=1 sv=2 | P | GO:00484 68 | cell development |
| gi\|19438349 6 | thrb_humanprothrombin os=homo sapiens gn=f2 pe=1 sv=2 | P | GO:20000 26 | regulation of multicellular organismal development |
| gi\|19438349 6 | thrb_humanprothrombin os=homo sapiens gn=f2 pe=1 sv=2 | P | GO:00105 57 | positive regulation of macromolecule biosynthetic process |
| gi\|19438349 6 | thrb_humanprothrombin os=homo sapiens gn=f2 pe=1 sv=2 | P | GO:00301 94 | positive regulation of blood coagulation |
| gi\|19438349 6 | thrb_humanprothrombin os=homo sapiens gn=f2 pe=1 sv=2 | F | GO:00051 02 | receptor binding |
| gi\|19438349 6 | thrb_humanprothrombin os=homo sapiens gn=f2 pe=1 sv=2 | P | GO:00099 67 | positive regulation of signal transduction |
| gi\|19438349 5 | thrb_humanprothrombin os=homo sapiens gn=f2 pe=1 sv=2 | C | GO:00056 15 | extracellular space |
| gi\|19438349 5 | thrb_humanprothrombin os=homo sapiens gn=f2 pe=1 sv=2 | P | GO:00301 68 | platelet activation |
| gi\|19438349 5 | thrb_humanprothrombin os=homo sapiens gn=f2 pe=1 sv=2 | F | GO:00082 36 | serine-type peptidase activity |
| gi\|19438349 5 | thrb_humanprothrombin os=homo sapiens gn=f2 pe=1 sv=2 | P | GO:00164 77 | cell migration |
| gi\|19438349 5 | thrb_humanprothrombin os=homo sapiens gn=f2 pe=1 sv=2 | P | GO:00065 08 | proteolysis |
| gi\|19438349 5 | thrb_humanprothrombin os=homo sapiens gn=f2 pe=1 sv=2 | P | GO:00019 34 | positive regulation of protein phosphorylation |
| gi\|19438349 5 | thrb_humanprothrombin os=homo sapiens gn=f2 pe=1 sv=2 | P | GO:00071 66 | cell surface receptor signaling pathway |
| gi\|19438349 6 | thrb_humanprothrombin os=homo sapiens gn=f2 pe=1 sv=2 | P | GO:00485 23 | negative regulation of cellular process |
| gi\|19438349 6 | thrb_humanprothrombin os=homo sapiens gn=f2 pe=1 sv=2 | P | GO:00068 10 | transport |
| gi\|19438349 5 | thrb_humanprothrombin os=homo sapiens gn=f2 pe=1 sv=2 | P | GO:00427 30 | fibrinolysis |
| gi\|19438349 5 | thrb_humanprothrombin os=homo sapiens gn=f2 pe=1 sv=2 | C | GO:00056 22 | intracellular |
| gi\|19438349 5 | thrb_humanprothrombin os=homo sapiens gn=f2 pe=1 sv=2 | P | GO:00487 31 | system development |
| gi\|19438349 6 | thrb_humanprothrombin os=homo sapiens gn=f2 pe=1 sv=2 | C | GO:00160 20 | membrane |
| gi\|19438349 6 | thrb_humanprothrombin os=homo sapiens gn=f2 pe=1 sv=2 | P | GO:00514 80 | cytosolic calcium ion homeostasis |
| KH 6 | 28071026 | Fr III | gi\|2807102 6 | ighm_humanig mu chain c region os=homo sapiens gn=ighm pe=1 sv=3 |
| gi\|28071026 | ighm_humanig mu chain c region os=homo sapiens gn=ighm pe=1 sv=3 | F | GO:00054 88 | binding |
| gi\|28071026 | ighm_humanig mu chain c region os=homo sapiens gn=ighm pe=1 sv=3 | C | GO:00444 64 | cell part |
| gi\|28071026 | ighm_humanig mu chain c region os=homo sapiens gn=ighm pe=1 sv=3 | C | GO:00160 20 | membrane |
| gi\|28071026 | ighm_humanig mu chain c region os=homo sapiens gn=ighm pe=1 sv=3 | P | GO:00069 55 | immune response |
| KH 7 | 300621695 | Fr III | gi\|3006216 95 | ighm_humanig mu chain c region os=homo sapiens gn=ighm pe=1 sv=3 |
| sil30062169 5 | ighm_humanig mu chain c region os=homo sapiens gn=ighm pe=1 sv=3 | P | GO:00069 55 | immune response |
| KH 8 | 1335098 | Fr III | gi\|1335098 | hemo_humanhemopexin os=homo sapiens gn=hpx pe=1 sv=2 |
| gi\|1335098 | hemo_humanhemopexin os=homo sapiens gn=hpx pe=1 sv=2 | P | GO:00081 52 | metabolic process |
| gi\|1335098 | hemo_humanhemopexin os=homo sapiens gn=hpx pe=1 sv=2 | P | GO:00511 79 | localization |
| gi\|1335098 | hemo_humanhemopexin os=homo sapiens gn=hpx pe=1 sv=2 | C | GO:00056 15 | extracellular space |
| gi\|1335098 | hemo_humanhemopexin os=homo sapiens gn=hpx pe=1 sv=2 | F | GO:00055 15 | protein binding |
| gi\|1335098 | hemo_humanhemopexin os=homo sapiens gn=hpx pe=1 sv=2 | P | GO:00485 22 | positive regulation of cellular process |
| gi\|1335098 | hemo_humanhemopexin os=homo sapiens gn=hpx pe=1 sv=2 | P | GO:00508 96 | response to stimulus |
| KH 9 | 10434804 | Fr III | gi\|1043480 4 | mthsd_humanmethenyltetrahydr ofolate synthase domain-containing protein os=homo sapiens gn=mthfsd pe=1 sv=2 |
| gi\|10434804 | mthsd_humanmethenyltetrahydr ofolate synthase domain-containing protein os=homo sapiens gn=mthfsd pe=1 sv=2 | F | GO:00055 24 | ATP binding |
| gi\|10434804 | mthsd_humanmethenyltetrahydr ofolate synthase domain-containing protein os=homo sapiens gn=mthfsd pe=1 sv=2 | P | GO:00093 96 | folic acid-containing compound biosynthetic process |
| gi\|10434804 | mthsd_humanmethenyltetrahydr ofolate synthase domain-containing protein os=homo sapiens gn=mthfsd pe=1 sv=2 | F | GO:00302 72 | 5-formyltetrahydrofolate cyclo-ligase activity |
| KH 10 | 221044726 | Fr III | gi\|2210447 26 | hemo_humanhemopexin os=homo sapiens gn=hpx pe=1 sv=2 |
| gi\|22104472 5 | hemo_humanhemopexin os=homo sapiens gn=hpx pe=1 sv=2 | F | GO:00055 15 | protein binding |
| gi\|22104472 5 | hemo_humanhemopexin os=homo sapiens gn=hpx pe=1 sv=2 | C | GO:00056 15 | extracellular space |
| gi\|22104472 5 | hemo_humanhemopexin os=homo sapiens gn=hpx pe=1 sv=2 | P | GO:00099 87 | cellular process |
| gi\|22104472 6 | hemo_humanhemopexin os=homo sapiens gn=hpx pe=1 sv=2 | P | GO:00650 07 | biological regulation |
| KH 11 | 215415638 | PCC | same as KH 3 | |
| KH 12 | 189066554 | PCC | gi\|1890665 54 | thrb_humanprothrombin os=homo sapiens gn=f2 pe=1 sv=2 |
| gi\|18906655 4 | thrb_humanprothrombin os=homo sapiens gn=f2 pe=1 sv=2 | C | GO:00444 46 | intracellular organelle part |
| gi\|18906655 4 | thrb_humanprothrombin os=homo sapiens gn=f2 pe=1 sv=2 | P | GO:00487 12 | negative regulation of astrocyte differentiation |
| gi\|18906655 4 | thrb_humanprothrombin os=homo sapiens gn=f2 pe=1 sv=2 | C | GO:00432 33 | organelle lumen |
| gi\|18906655 4 | thrb_humanprothrombin os=homo sapiens gn=f2 pe=1 sv=2 | P | GO:00301 94 | positive regulation of blood coagulation |
| gi\|18906655 4 | thrb _humanprothrombin os=homo sapiens gn=f2 pe=1 sv=2 | F | GO:00051 02 | receptor binding |
| gi\|18906655 4 | thrb_humanprothrombin os=homo sapiens gn=f2 pe=1 sv=2 | P | GO:20003 79 | positive regulation of reactive oxygen species metabolic process |
| gi\|18906655 4 | thrb_humanprothrombin os=homo sapiens gn=f2 pe=1 sv=2 | P | GO:00458 61 | negative regulation of proteolysis |
| gi\|18906655 4 | thrb_humanprothrombin os=homo sapiens gn=f2 pe=1 sv=2 | C | GO:00056 15 | extracellular space |
| gi\|18906655 4 | thrb_humanprothrombin os=homo sapiens gn=f2 pe=1 sv=2 | P | GO:00301 68 | platelet activation |
| gi\|18906655 4 | thrb_humanprothrombin os=homo sapiens gn=f2 pe=1 sv=2 | P | GO:19007 38 | positive regulation of phospholipase C-activating G-protein coupled receptor signaling pathway |
| gi\|18906655 4 | thrb_humanprothrombin os=homo sapiens gn=f2 pe=1 sv=2 | P | GO:00164 77 | cell migration |
| gi\|18906655 4 | thrb_humanprothrombin os=homo sapiens gn=f2 pe=1 sv=2 | C | GO:00432 31 | intracellular membrane-bounded organelle |
| gi\|18906655 4 | thrb_humanprothrombin os=homo sapiens gn=f2 pe=1 sv=2 | P | GO:00019 34 | positive regulation of protein phosphorylation |
| gi\|18906655 4 | thrb_humanprothrombin os=homo sapiens gn=f2 pe=1 sv=2 | C | GO:00058 86 | plasma membrane |
| gi\|18906655 4 | thrb_humanprothrombin os=homo sapiens gn=f2 pe=1 sv=2 | F | GO:00700 53 | thrombospondin receptor activity |
| gi\|18906655 4 | thrb_humanprothrombin os=homo sapiens gn=f2 pe=1 sv=2 | P | GO:00512 81 | positive regulation of release of sequestered calcium ion into cytosol |
| gi\|18906655 4 | thrb_humanprothrombin os=homo sapiens gn=f2 pe=1 sv=2 | F | GO:00042 52 | serine-type endopeptidase activity |
| gi\|18906655 4 | thrb_humanprothrombin os=homo sapiens gn=f2 pe=1 sv=2 | P | GO:00427 30 | fibrinolysis |
| gi\|18906655 4 | thrb_humanprothrombin os=homo sapiens gn=f2 pe=1 sv=2 | C | GO:00444 44 | cytoplasmic part |
| gi\|18906655 4 | thrb_humanprothrombin os=homo sapiens gn=f2 pe=1 sv=2 | P | GO:00329 67 | positive regulation of collagen biosynthetic process |
| KH 13 | 194391084 | PCC | gi\|1943910 84 | kng1_humankininogen-1 os=homo sapiens gn=kngl pe=1 sv=2 |
| gi\|19439108 4 | kng1_humankininogen-1 os=homo sapiens gn=kng1 pe=1 sv=2 | F | GO:00055 15 | protein binding |
| gi\|19439108 4 | kng1_humankininogen-1 os=homo sapiens gn=kng1 pe=1 sv=2 | P | GO:00550 65 | metal ion homeostasis |
| gi\|19439108 4 | kng1_humankininogen-1 os=homo sapiens gn=kng1 pe=1 sv=2 | P | GO:00512 41 | negative regulation of multicellular organismal process |
| gi\|19439108 4 | kng1_humankininogen-1 os=homo sapiens gn=kng1 pe=1 sv=2 | P | GO:00075 96 | blood coagulation |
| gi\|19439108 4 | kng1_humankininogen-1 os=homo sapiens gn=kng1 pe=1 sv=2 | C | GO:00432 29 | intracellular organelle |
| gi\|19439108 4 | kng1_humankininogen-1 os=homo sapiens gn=kng1 pe=1 sv=2 | P | GO:00485 23 | negative regulation of cellular process |
| gi\|19439108 4 | kng1_humankininogen-1 os=homo sapiens gn=kng1 pe=1 sv=2 | P | GO:00081 52 | metabolic process |
| gi\|19439108 4 | kng1_humankininogen-1 os=homo sapiens gn=kng1 pe=1 sv=2 | P | GO:00030 08 | system process |
| KH 14 | 158255114 | PCC | gi\|1582551 14 | kng1_humankininogen-1 os=homo sapiens gn=kng1 pe=1 sv=2 |
| gi\|15825511 4 | kng1_humankininogen-1 os=homo sapiens gn=kng1 pe=1 sv=2 | F | GO:00055 15 | protein binding |
| gi\|15825511 4 | kng1_humankininogen-1 os=homo sapiens gn=kng1 pe=1 sv=2 | P | GO:00550 65 | metal ion homeostasis |
| gi\|15825511 4 | kng1_humankininogen-1 os=homo sapiens gn=kng1 pe=1 sv=2 | P | GO:00512 41 | negative regulation of multicellular organismal process |
| gi\|15825511 4 | kng1_humankininogen-1 os=homo sapiens gn=kng1 pe=1 sv=2 | P | GO:00075 96 | blood coagulation |
| gi\|15825511 4 | kng1_humankininogen-1 os=homo sapiens gn=kng1 pe=1 sv=2 | C | GO:00432 29 | intracellular organelle |
| gi\|15825511 4 | kng1_humankininogen-1 os=homo sapiens gn=kng1 pe=1 sv=2 | P | GO:00485 23 | negative regulation of cellular process |
| gi\|15825511 4 | kng1_humankininogen-1 os=homo sapiens gn=kng1 pe=1 sv=2 | P | GO:00081 52 | metabolic process |
| gi\|15825511 4 | kng1_humankininogen-1 os=homo sapiens gn=kng1 pe=1 sv=2 | P | GO:00030 08 | system process |
| KH 15 | 213506121 | PCC | gi\|2135061 21 | kng1_humankininogen-1 os=homo sapiens gn=kngl pe=1 sv=2 |
| gi\|21350612 1 | kng1_humankininogen-1 os=homo sapiens gn=kng1 pe=1 sv=2 | F | GO:00055 15 | protein binding |
| gi\|21350612 1 | kng1_humankininogen-1 os=homo sapiens gn=kng1 pe=1 sv=2 | P | GO:00550 65 | metal ion homeostasis |
| gi\|21350612 1 | kng1_humankininogen-1 os=homo sapiens gn=kng1 pe=1 sv=2 | P | GO:00512 41 | negative regulation of multicellular organismal process |
| gi\|21350612 1 | kng1_humankininogen-1 os=homo sapiens gn=kng1 pe=1 sv=2 | P | GO:00075 96 | blood coagulation |
| gi\|21350612 1 | kng1_humankininogen-1 os=homo sapiens gn=kng1 pe=1 sv=2 | C | GO:00432 29 | intracellular organelle |
| gi\|21350612 1 | kng1_humankininogen-1 os=homo sapiens gn=kng1 pe=1 sv=2 | P | GO:00485 23 | negative regulation of cellular process |
| gi\|21350612 1 | kng1_humankininogen-1 os=homo sapiens gn=kng1 pe=1 sv=2 | P | GO:00081 52 | metabolic process |
| gi\|21350612 1 | kng1_humankininogen-1 os=homo sapiens gn=kng1 pe=1 sv=2 | P | GO:00030 08 | system process |
| KH 16 | 213506103 | PCC | gi\|213 5061 03 | kng1_humankininogen-1 os=homo sapiens gn=kng1 pe=1 sv=2 |
| gi\|21350610 3 | kng1_humankininogen-1 os=homo sapiens gn=kng1 pe=1 sv=2 | F | GO:00055 15 | protein binding |
| gi\|21350610 3 | kng1_humankininogen-1 os=homo sapiens gn=kng1 pe=1 sv=2 | P | GO:00550 65 | metal ion homeostasis |
| gi\|21350610 3 | kng1_humankininogen-1 os=homo sapiens gn=kng1 pe=1 sv=2 | P | GO:00512 41 | negative regulation of multicellular organismal process |
| gi\|21350610 3 | kng1_humankininogen-1 os=homo sapiens gn=kng1 pe=1 sv=2 | P | GO:00075 96 | blood coagulation |
| gi\|21350610 3 | kng1_humankininogen-1 os=homo sapiens gn=kng1 pe=1 sv=2 | C | GO:00432 29 | intracellular organelle |
| gi\|21350610 3 | kng1_humankininogen-1 os=homo sapiens gn=kng1 pe=1 sv=2 | P | GO:00485 23 | negative regulation of cellular process |
| gi\|21350610 3 | kng1_humankininogen-1 os=homo sapiens gn=kng1 pe=1 sv=2 | P | GO:00081 52 | metabolic process |
| gi\|21350610 3 | kng1_humankininogen-1 os=homo sapiens gn=kng1 pe=1 sv=2 | P | GO:00030 08 | system process |
| KH 17 | 194376310 | PCC | gi\|1943763 10 | cytoplasmic 1 os=homo sapiens gn=actb pe=1 sv=1 |
| gi\|19437631 0 | cytoplasmic 1 os=homo sapiens gn=actb pe=1 sv=1 | P | GO:00098 88 | tissue development |
| gi\|19437631 0 | cytoplasmic 1 os=homo sapiens gn=actb pe=1 sv=1 | P | GO:00300 48 | actin filament-based movement |
| gi\|19437631 0 | cytoplasmic 1 os=homo sapiens gn=actb pe=1 sv=1 | P | GO:00030 12 | muscle system process |
| gi\|19437631 0 | cytoplasmic 1 os=homo sapiens gn=actb pe=1 sv=1 | C | GO:00300 17 | sarcomere |
| gi\|19437631 0 | cytoplasmic 1 os=homo sapiens gn=actb pe=1 sv=1 | P | GO:00302 39 | myofibril assembly |
| gi\|19437631 0 | cytoplasmic 1 os=homo sapiens gn=actb pe=1 sv=1 | P | GO:00442 38 | primary metabolic process |
| gi\|19437631 0 | cytoplasmic 1 os=homo sapiens gn=actb pe=1 sv=1 | C | GO:00058 84 | actin filament |
| gi\|19437631 0 | cytoplasmic 1 os=homo sapiens gn=actb pe=1 sv=1 | P | GO:00723 58 | cardiovascular system development |
| gi\|19437631 0 | cytoplasmic 1 os=homo sapiens gn=actb pe=1 sv=1 | P | GO:00442 37 | cellular metabolic process |
| gi\|19437631 0 | cytoplasmic 1 os=homo sapiens gn=actb pe=1 sv=1 | P | GO:00485 13 | organ development |
| gi\|19437631 0 | cytoplasmic 1 os=homo sapiens gn=actb pe=1 sv=1 | F | GO:00055 15 | protein binding |
| gi\|19437631 0 | cytoplasmic 1 os=homo sapiens gn=actb pe=1 sv=1 | P | GO:00422 21 | response to chemical stimulus |
| gi\|19437631 0 | cytoplasmic 1 os=homo sapiens gn=actb pe=1 sv=1 | P | GO:00080 15 | blood circulation |
| KH 18 | 194388064 | PCC | gi\|1943880 64 | cytoplasmic 2 os=homo sapiens gn=actg1 pe=1 sv=1 |
| gi\|19438806 4 | cytoplasmic 2 os=homo sapiens gn=actg1 pe=1 sv=1 | P | GO:00098 88 | tissue development |
| gi\|19438806 4 | cytoplasmic 2 os=homo sapiens gn=actg1 pe=1 sv=1 | P | GO:00300 48 | actin filament-based movement |
| gi\|19438806 4 | cytoplasmic 2 os=homo sapiens gn=actg1 pe=1 sv=1 | P | GO:00030 12 | muscle system process |
| gi\|19438806 4 | cytoplasmic 2 os=homo sapiens gn=actg1 pe=1 sv=1 | C | GO:00300 17 | sarcomere |
| gi\|19438806 | cytoplasmic 2 os=homo sapiens gn=actg1 pe=1 sv=1 | P | GO:00302 39 | myofibril assembly |
| gi\|194388064 | cytoplasmic 2 os=homo sapiens gn=actg1 pe=1 sv=1 | P | GO:00442 38 | primary metabolic process |
| gi\|194388064 | cytoplasmic 2 os=homo sapiens gn=actg1 pe=1 sv=1 | C | GO:00058 84 | actin filament |
| gi\|194388064 | cytoplasmic 2 os=homo sapiens gn=actg1 pe=1 sv=1 | P | GO:00723 58 | cardiovascular system development |
| gi\|194388064 | cytoplasmic 2 os=homo sapiens gn=actg1 pe=1 sv=1 | P | GO:00442 37 | cellular metabolic process |
| gi\|194388064 | cytoplasmic 2 os=homo sapiens gn=actg1 pe=1 sv=1 | P | GO:00485 13 | organ development |
| gi\|194388064 | cytoplasmic 2 os=homo sapiens gn=actg1 pe=1 sv=1 | P | GO:00422 21 | response to chemical stimulus |
| gi\|194388064 | cytoplasmic 2 os=homo sapiens gn=actg1 pe=1 sv=1 | F | GO:00080 92 | cytoskeletal protein binding |
| gi\|194388064 | cytoplasmic 2 os=homo sapiens gn=actg1 pe=1 sv=1 | P | GO:00650 08 | regulation of biological quality |
| gi\|194388064 | cytoplasmic 2 os=homo sapiens gn=actg1 pe=1 sv=1 | C | GO:00444 51 | nucleoplasm part |
| gi\|194388064 | cytoplasmic 2 os=homo sapiens gn=actg1 pe=1 sv=1 | P | GO:00080 15 | blood circulation |
| gi\|194388064 | cytoplasmic 2 os=homo sapiens gn=actg1 pe=1 sv=1 | F | GO:00198 99 | enzyme binding |
| gi\|19439108 4 | kng1_humankininogen-1 os=homo sapiens gn=kng1 pe=1 sv=2 | F | GO:00055 15 | protein binding |
| gi\|19439108 4 | kng1_humankininogen-1 os=homo sapiens gn=kng1 pe=1 sv=2 | P | GO:00550 65 | metal ion homeostasis |
| gi\|19439108 4 | kng1_humankininogen-1 os=homo sapiens gn=kng1 pe=1 sv=2 | P | GO:00512 41 | negative regulation of multicellular organismal process |
| gi\|19439108 4 | kng1_humankininogen-1 os=homo sapiens gn=kng1 pe=1 sv=2 | P | GO:00075 96 | blood coagulation |
| gi\|19439108 4 | kng1_humankininogen-1 os=homo sapiens gn=kng1 pe=1 sv=2 | C | GO:00432 29 | intracellular organelle |
| gi\|19439108 4 | kng1_humankininogen-1 os=homo sapiens gn=kng1 pe=1 sv=2 | P | GO:00485 23 | negative regulation of cellular process |
| gi\|19439108 4 | kng1_humankininogen-1 os=homo sapiens gn=kng1 pe=1 sv=2 | P | GO:00081 52 | metabolic process |
| gi\|19439108 4 | kng1_humankininogen-1 os=homo sapiens gn=kng1 pe=1 sv=2 | P | GO:00030 08 | system process |
| KH 19 | IPI00964149 | PCC | IPI009641 49 | pacrl_humanpacrg-like protein os=homo sapiens gn=pacrgl pe=1 sv=2 |
| KH 20 | IPI00966721 | PCC | IPI009667 21 | ce028 humantransmembrane protein c5orf28 os=homo sapiens gn=c5orf28 pe=2 sv=1 |
| IPI00966721 | ce028_humantransmembrane protein c5orf28 os=homo sapiens gn=c50rf28 pe=2 sv=1 | C | GO:00160 21 | integral to membrane |
| KH 21 | IPI00966826 | FrIV | IPI009668 26 | pds5a_humansister chromatid cohesion protein pds5 homolog a os=homo sapiens gn=pds5a pe=1 sv=1 |
| IPI00966826 | pds5a_humansister chromatid cohesion protein pds5 homolog a os=homo sapiens gn=pds5a pe=1 sv=1 | P | GO:00081 56 | negative regulation of DNA replication |
| IPI00966826 | pds5a_humansister chromatid cohesion protein pds5 homolog a os=homo sapiens gn=pds5a pe=1 sv=1 | C | GO:00057 30 | nucleolus |
| IPI00966826 | pds5a_humansister chromatid cohesion protein pds5 homolog a os=homo sapiens gn=pds5a pe=1 sv=1 | C | GO:00007 85 | chromatin |
| IPI00966826 | pds5a_humansister chromatid cohesion protein pds5 homolog a | F | GO:00055 15 | protein binding |
| | os=homo sapiens gn=pds5a pe=1 sv=1 | | | |
| IPI00966826 | pds5a_humansister chromatid cohesion protein pds5 homolog a os=homo sapiens gn=pds5a pe=1 sv=1 | P | GO:00082 83 | cell proliferation |
| IPI00966826 | pds5a_humansister chromatid cohesion protein pds5 homolog a os=homo sapiens gn=pds5a pe=1 sv=1 | C | GO:00058 86 | plasma membrane |
| KH 22 | IPI00760788 | FrIV | IPI007607 88 | klh22_humankelch-like protein 22 os=homo sapiens gn=klhl22 pe=1 sv=2 |
| IPI00760788 | klh22_humankelch-like protein 22 os=homo sapiens gn=klhl22 pe=1 sv=2 | P | GO:00513 01 | cell division |
| IPI00760788 | klh22_humankelch-like protein 22 os=homo sapiens gn=klhl22 pe=1 sv=2 | C | GO:00314 63 | Cul3-RING ubiquitin ligase complex |
| KH 23 | IPI00917278 | FrIV | | |
| KH 24 | IPI00966721 | Apolipoprotei n complex | same as KH 20 | |
| KH 25 | IPI01012037 | Apolipoprotei n complex | IPI010120 37 | mcm8_humandna helicase mcm8 os=homo sapiens gn=mcm8 pe=1 sv=2 |
| IPI01012037 | mcm8_humandna helicase mcm8 os=homo sapiens gn=mcm8 pe=1 sv=2 | P | GO:00513 29 | interphase of mitotic cell cycle |
| IPI01012037 | mcm8_humandna helicase mcm8 os=homo sapiens gn=mcm8 pe=1 sv=2 | P | GO:00346 45 | cellular macromolecule biosynthetic process |
| IPI01012037 | mcm8_humandna helicase mcm8 os=homo sapiens gn=mcm8 pe=1 sv=2 | P | GO:00903 04 | nucleic acid metabolic process |
| KH 26 | IPI00940730 | Apolipoprotei n complex | IPI009407 30 | enoph _humanenolase-phosphatase el os=homo sapiens gn=enoph1 pe=l sv=1 |
| IPI00940730 | enoph humanenolase-phosphatase el os=homo sapiens gn=enoph1 pe=1 sv=1 | P | GO:00195 09 | L-methionine salvage from methylthioadenosine |
| IPI00940730 | enoph_humanenolase-phosphatase el os=homo sapiens gn=enoph1 pe=1 sv=1 | F | GO:00438 74 | acireductone synthase activity |
| KH 27 | IPI00977191 | Apolipoprotei n complex | | |
| KH 28 | IPI00022434 | HemoRAAS | IPI000224 34 | albu_humanserum albumin os=homo sapiens gn=alb pe=1 sv=2 |
| IPI00022434 | albu_humanserum albumin os=homo sapiens gn=alb pe=1 sv=2 | P | GO:00082 02 | steroid metabolic process |
| IPI00022434 | albu_humanserum albumin os=homo sapiens gn=alb pe=1 sv=2 | P | GO:00517 04 | multi-organism process |
| IPI00022434 | albu_humanserum albumin os=homo sapiens gn=alb pe=1 sv=2 | C | GO:00444 46 | intracellular organelle part |
| IPI00022434 | albu_humanserum albumin os=homo sapiens gn=alb pe=1 sv=2 | P | GO:00516 41 | cellular localization |
| IPI00022434 | albu_humanserum albumin os=homo sapiens gn=alb pe=1 sv=2 | P | GO:00517 16 | cellular response to stimulus |
| IPI00022434 | albu_humanserum albumin os=homo sapiens gn=alb pe=1 sv=2 | F | GO:00082 89 | lipid binding |
| IPI00022434 | albu_humanserum albumin os=homo sapiens gn=alb pe=1 sv=2 | P | GO:00430 69 | negative regulation of programmed cell death |
| IPI00022434 | albu_humanserum albumin os=homo sapiens gn=alb pe=1 sv=2 | P | GO:00442 60 | cellular macromolecule metabolic process |
| IPI00022434 | albu_humanserum albumin os=homo sapiens gn=alb pe=1 sv=2 | P | GO:00316 67 | response to nutrient levels |
| IPI00022434 | albu_humanserum albumin os=homo sapiens gn=alb pe=1 sv=2 | C | GO:00432 31 | intracellular membrane-bounded organelle |
| IPI00022434 | albu_humanserum albumin os=homo sapiens gn=alb pe=1 sv=2 | P | GO:00442 81 | small molecule metabolic process |
| IPI00022434 | albu_humanserum albumin os=homo sapiens gn=alb pe=1 sv=2 | F | GO:00055 15 | protein binding |
| IPI00022434 | albu_humanserum albumin os=homo sapiens gn=alb pe=1 sv=2 | P | GO:00068 10 | transport |
| IPI00022434 | albu_humanserum albumin os=homo sapiens gn=alb pe=1 sv=2 | P | GO:00650 08 | regulation of biological quality |
| IPI00022434 | albu_humanserum albumin os=homo sapiens gn=alb pe=1 sv=2 | P | GO:00071 54 | cell communication |
| IPI00022434 | albu_humanserum albumin os=homo sapiens gn=alb pe=1 sv=2 | F | GO:00198 42 | vitamin binding |
| IPI00022434 | albu_humanserum albumin os=homo sapiens gn=alb pe=1 sv=2 | P | GO:00069 50 | response to stress |
| IPI00022434 | albu_humanserum albumin os=homo sapiens gn=alb pe=1 sv=2 | C | GO:00444 44 | cytoplasmic part |
| IPI00022434 | albu_humanserum albumin os=homo sapiens gn=alb pe=1 sv=2 | P | GO:00325 01 | multicellular organismal process |
| IPI00022434 | albu_humanserum albumin os=homo sapiens gn=alb pe=1 sv=2 | C | GO:00444 21 | extracellular region part |
| KH 29 | IPI00022434 | HemoRAAS | same as KH 28 | |
| KH 30 | IPI00219713 | FibroRAAS | IPI002197 13 | fibg_humanfibrinogen gamma chain os=homo sapiens gn=fgg pe=1 sv=3 |
| IPI00219713 | fibg_humanfibrinogen gamma chain os=homo sapiens gn=fgg pe=1 sv=3 | P | GO:00099 87 | cellular process |
| IPI00219713 | fibg_humanfibrinogen gamma chain os=homo sapiens gn=fgg pe=1 sv=3 | C | GO:00098 97 | external side of plasma membrane |
| IPI00219713 | fibg_humanfibrinogen gamma chain os=homo sapiens gn=fgg pe=1 sv=3 | F | GO:00434 99 | eukaryotic cell surface binding |
| IPI00219713 | fibg_humanfibrinogen gamma chain os=homo sapiens gn=fgg pe=1 sv=3 | C | GO:00056 15 | extracellular space |
| IPI00219713 | fibg_humanfibrinogen gamma chain os=homo sapiens gn=fgg pe=1 sv=3 | C | GO:00310 91 | platelet alpha granule |
| IPI00219713 | fibg_humanfibrinogen gamma chain os=homo sapiens gn=fgg pe=1 sv=3 | P | GO:00325 01 | multicellular organismal process |
| IPI00219713 | fibg_humanfibrinogen gamma chain os=homo sapiens gn=fgg pe=1 sv=3 | P | GO:00650 07 | biological regulation |
| IPI00219713 | fibg_humanfibrinogen gamma chain os=homo sapiens gn=fgg pe=1 sv=3 | P | GO:00515 92 | response to calcium ion |
| KH 31 | IPI00219713 | FibroRAAS | same as KH 30 | |
| KH 32 | IPI00220327 | FibroRAAS | IPI002203 27 | type ii cytoskeletal 1 os=homo sapiens gn=krt1 pe=1 sv=6 |
| IPI00220327 | type ii cytoskeletal 1 os=homo sapiens gn=krt1 pe=1 sv=6 | P | GO:00099 87 | cellular process |
| IPI00220327 | type ii cytoskeletal 1 os=homo sapiens gn=krt1 pe=1 sv=6 | P | GO:00487 31 | system development |
| IPI00220327 | type ii cytoskeletal 1 os=homo sapiens gn=krt1 pe=1 sv=6 | P | GO:00098 88 | tissue development |
| IPI00220327 | type ii cytoskeletal 1 os=homo sapiens gn=krt1 pe=1 sv=6 | C | GO:00058 56 | cytoskeleton |
| IPI00220327 | type ii cytoskeletal 1 os=homo sapiens gn=krt1 pe=1 sv=6 | F | GO:00055 15 | protein binding |
| IPI00220327 | type ii cytoskeletal 1 os=homo sapiens gn=krt1 pe=1 sv=6 | P | GO:00018 67 | complement activation, lectin pathway |
| IPI00220327 | type ii cytoskeletal 1 os=homo sapiens gn=krt1 pe=1 sv=6 | F | GO:00302 46 | carbohydrate binding |
| IPI00220327 | type ii cytoskeletal 1 os=homo sapiens gn=krt1 pe=1 sv=6 | C | GO:00160 20 | membrane |
| KH 33 | IPI00029739 | GammaRAAS | IPI000297 39 | cfah - humancomplement factor h os=homo sapiens gn=cfh pe=1 sv=4 |
| [PI00029739 | cfah_humancomplement factor h os=homo sapiens gn=cfh pe=1 sv=4 | P | GO:00304 49 | regulation of complement activation |
| [PI00029739 | cfah_humancomplement factor h os=homo sapiens gn=cfh pe=1 sv=4 | P | GO:00450 87 | innate immune response |
| KH 34 | IPI00384853 | GammaRAAS | | |
| KH 35 | IPI00479708 | GammaRAAS | IPI004797 08 | ighm_humanig mu chain c region os=homo sapiens gn=ighm pe=1 sv=3 |
| IPI00479708 | ighm_humanig mu chain c region os=homo sapiens gn=ighm pe=1 sv=3 | F | GO:00054 88 | binding |
| IPI00479708 | ighm_humanig mu chain c region os=homo sapiens gn=ighm pe=1 sv=3 | C | GO:00444 64 | cell part |
| IPI00479708 | ighm_humanig mu chain c region os=homo sapiens gn=ighm pe=1 sv=3 | C | GO:00160 20 | membrane |
| IPI00479708 | ighm_humanig mu chain c region os=homo sapiens gn=ighm pe=1 sv=3 | P | GO:00069 55 | immune response |
| KH 36 | IPI00298497 | GammaRAAS | IPI002984 97 | fibb_humanfibrinogen beta chain os=homo sapiens gn=fgb pe=1 sv=2 |
| IPI00298497 | fibb_humanfibrinogen beta chain os=homo sapiens gn=fgb pe=1 sv=2 | F | GO:00510 87 | chaperone binding |
| IPI00298497 | fibb_humanfibrinogen beta chain os=homo sapiens gn=fgb pe=1 sv=2 | P | GO:00515 92 | response to calcium ion |
| IPI00298497 | fibb_humanfibrinogen beta chain os=homo sapiens gn=fgb pe=1 sv=2 | C | GO:00056 15 | extracellular space |
| IPI00298497 | fibb_humanfibrinogen beta chain os=homo sapiens gn=fgb pe=1 sv=2 | P | GO:00511 79 | localization |
| IPI00298497 | fibb_humanfibrinogen beta chain os=homo sapiens gn=fgb pe=1 sv=2 | C | GO:00310 91 | platelet alpha granule |
| IPI00298497 | fibb_humanfibrinogen beta chain os=homo sapiens gn=fgb pe=1 sv=2 | C | GO:00098 97 | external side of plasma membrane |
| IPI00298497 | fibb_humanfibrinogen beta chain os=homo sapiens gn=fgb pe=1 sv=2 | P | GO:00507 94 | regulation of cellular process |
| IPI00298497 | fibb_humanfibrinogen beta chain os=homo sapiens gn=fgb pe=1 sv=2 | P | GO:00069 50 | response to stress |
| IPI00298497 | fibb_humanfibrinogen beta chain os=homo sapiens gn=fgb pe=1 sv=2 | F | GO:00434 99 | eukaryotic cell surface binding |
| IPI00298497 | fibb_humanfibrinogen beta chain os=homo sapiens gn=fgb pe=1 sv=2 | P | GO:00325 01 | multicellular organismal process |
| KH 37 | IPI00021841 | GammaRAAS | IPI000218 41 | apoa1_humanapolipoprotein a-i os=homo sapiens gn=apoa1 pe=1 sv=1 |
| IPI00021841 | apoa1_humanapolipoprotein a-i os=homo sapiens gn=apoa1 pe=1 sv=1 | P | GO:00705 08 | cholesterol import |
| IPI00021841 | apoa1_humanapolipoprotein a-i os=homo sapiens gn=apoa1 pe=1 sv=1 | C | GO:00301 39 | endocytic vesicle |
| IPI00021841 | apoa1_humanapolipoprotein a-i os=homo sapiens gn=apoa1 pe=1 sv=1 | P | GO:00507 28 | negative regulation of inflammatory response |
| IPI00021841 | apoa1_humanapolipoprotein a-i os=homo sapiens gn=apoa1 pe=1 sv=1 | P | GO:00333 44 | cholesterol efflux |
| IPI00021841 | apoa1_humanapolipoprotein a-i os=homo sapiens gn=apoa1 pe=1 sv=1 | P | GO:00341 15 | negative regulation of heterotypic cell-cell adhesion |
| IPI00021841 | apoa1_humanapolipoprotein a-i os=homo sapiens gn=apoa1 pe=1 sv=1 | P | GO:00182 06 | peptidyl- methionine modification |
| IPI00021841 | apoa1_humanapolipoprotein a-i os=homo sapiens gn=apoa1 pe=1 sv=1 | P | GO:00421 57 | lipoprotein metabolic process |
| IPI00021841 | apoa1_humanapolipoprotein a-i os=homo sapiens gn=apoa1 pe=1 sv=1 | P | GO:00436 91 | reverse cholesterol transport |
| IPI00021841 | apoa1_humanapolipoprotein a-i os=homo sapiens gn=apoa1 pe=1 sv=1 | F | GO:00055 43 | phospholipid binding |
| IPI00021841 | apoa1_humanapolipoprotein a-i os=homo sapiens gn=apoa1 pe=1 sv=1 | P | GO:00027 40 | negative regulation of cytokine secretion involved in immune response |
| IPI00021841 | apoa1_humanapolipoprotein a-i os=homo sapiens gn=apoa1 pe=1 sv=1 | P | GO:00082 03 | cholesterol metabolic process |
| IPI00021841 | apoa1_humanapolipoprotein a-i os=homo sapiens gn=apoa1 pe=1 sv=1 | P | GO:00507 13 | negative regulation of interleukin-1 beta secretion |
| IPI00021841 | apoa1_humanapolipoprotein a-i os=homo sapiens gn=apoa1 pe=1 sv=1 | P | GO:00181 58 | protein oxidation |
| IPI00021841 | apoa1_humanapolipoprotein a-i os=homo sapiens gn=apoa1 pe=1 sv=1 | P | GO:00066 56 | phosphatidylcholine biosynthetic process |
| IPI00021841 | apoa1_humanapolipoprotein a-i os=homo sapiens gn=apoa1 pe=1 sv=1 | F | GO:00015 40 | beta-amyloid binding |
| IPI00021841 | apoa1_humanapolipoprotein a-i os=homo sapiens gn=apoa1 pe=1 sv=1 | F | GO:00602 28 | phosphatidylcholine-sterol O-acyltransferase activator activity |
| IPI00021841 | apoa1_humanapolipoprotein a-i os=homo sapiens gn=apoa1 pe=1 sv=1 | P | GO:00426 32 | cholesterol homeostasis |
| IPI00021841 | apoa1_humanapolipoprotein a-i os=homo sapiens gn=apoa1 pe=1 sv=1 | F | GO:00154 85 | cholesterol binding |
| IPI00021841 | apoa1_humanapolipoprotein a-i os=homo sapiens gn=apoa1 pe=1 sv=1 | P | GO:00420 60 | wound healing |
| IPI00021841 | apoa1_humanapolipoprotein a-i os=homo sapiens gn=apoa1 pe=1 sv=1 | F | GO:00341 91 | apolipoprotein A-I receptor binding |
| IPI00021841 | apoa1_humanapolipoprotein a-i os=homo sapiens gn=apoa1 pe=1 sv=1 | F | GO:00428 02 | identical protein binding |
| IPI00021841 | apoa1_humanapolipoprotein a-i os=homo sapiens gn=apoa1 pe=1 sv=1 | P | GO:00109 03 | negative regulation of very-low-density lipoprotein particle remodeling |
| IPI00021841 | apoa1_humanapolipoprotein a-i os=homo sapiens gn=apoa1 pe=1 sv=1 | P | GO:00108 04 | negative regulation of tumor necrosis factor-mediated signaling pathway |
| IPI00021841 | apoa1_humanapolipoprotein a-i os=homo sapiens gn=apoa1 pe=1 sv=1 | P | GO:00343 80 | high-density lipoprotein particle assembly |
| IPI00021841 | apoa1_humanapolipoprotein a-i os=homo sapiens gn=apoa1 pe=1 sv=1 | P | GO:00071 86 | G-protein coupled receptor signaling pathway |
| IPI00021841 | apoa1_humanapolipoprotein a-i os=homo sapiens gn=apoa1 pe=1 sv=1 | P | GO:00508 21 | protein stabilization |
| IPI00021841 | apoa1_humanapolipoprotein a-i os=homo sapiens gn=apoa1 pe=1 sv=1 | C | GO:00343 61 | very-low-density lipoprotein particle |
| IPI00021841 | apoa1_humanapolipoprotein a-i os=homo sapiens gn=apoa1 pe=1 sv=1 | P | GO:00324 88 | Cdc42 protein signal transduction |
| IPI00021841 | apoa1_humanapolipoprotein a-i os=homo sapiens gn=apoa1 pe=1 sv=1 | P | GO:00603 54 | negative regulation of cell adhesion molecule production |
| IPI00021841 | apoa1_humanapolipoprotein a-i os=homo sapiens gn=apoa1 pe=1 sv=1 | P | GO:00550 91 | phospholipid homeostasis |
| IPI00021841 | apoa1_humanapolipoprotein a-i os=homo sapiens gn=apoa1 pe=1 sv=1 | P | GO:00108 73 | positive regulation of cholesterol esterification |
| IPI00021841 | apoa1_humanapolipoprotein a-i os=homo sapiens gn=apoa1 pe=1 sv=1 | F | GO:00171 27 | cholesterol transporter activity |
| IPI00021841 | apoa1_humanapolipoprotein a-i os=homo sapiens gn=apoa1 pe=1 sv=1 | F | GO:00198 99 | enzyme binding |
| IPI00021841 | apoa1_humanapolipoprotein a-i os=homo sapiens gn=apoa1 pe=1 sv=1 | F | GO:00706 53 | high-density lipoprotein particle receptor binding |
| IPI00021841 | apoa1_humanapolipoprotein a-i os=homo sapiens gn=apoa1 pe=1 sv=1 | P | GO:00703 28 | triglyceride homeostasis |
| IPI00021841 | apoa1_humanapolipoprotein a-i os=homo sapiens gn=apoa1 pe=1 sv=1 | C | GO:00343 66 | spherical high-density lipoprotein particle |
| IPI00021841 | apoa1_humanapolipoprotein a-i os=homo sapiens gn=apoa1 pe=1 sv=1 | P | GO:00337 00 | phospholipid efflux |
| IPI00021841 | apoa1_humanapolipoprotein a-i os=homo sapiens gn=apoa1 pe=1 sv=1 | P | GO:00513 45 | positive regulation of hydrolase activity |
| KH 38 | IPI00783987 | AFCC | IPI007839 87 | co3_humancomplement c3 os=homo sapiens gn=c3 pe=1 sv=2 |
| IPI00783987 | co3_humancomplement c3 os=homo sapiens gn=c3 pe=1 sv=2 | C | GO:00444 64 | cell part |
| IPI00783987 | co3_humancomplement c3 os=homo sapiens gn=c3 pe=1 sv=2 | P | GO:00105 75 | positive regulation vascular endothelial growth factor production |
| IPI00783987 | co3_ humancomplement c3 os=homo sapiens gn=c3 pe=1 sv=2 | P | GO:00304 49 | regulation of complement activation |
| IPI00783987 | co3_humancomplement c3 os=homo sapiens gn=c3 pe=1 sv=2 | P | GO:00071 65 | signal transduction |
| IPI00783987 | co3_humancomplement c3 os=homo sapiens gn=c3 pe=1 sv=2 | P | GO:00450 87 | innate immune response |
| IPI00783987 | co3_humancomplement c3 os=homo sapiens gn=c3 pe=1 sv=2 | F | GO:00055 15 | protein binding |
| IPI00783987 | co3_humancomplement c3 os=homo sapiens gn=c3 pe=1 sv=2 | C | GO:00160 20 | membrane |
| KH 39 | IPI00878282 | AFCC | IPI008782 82 | albu_humanserum albumin os=homo sapiens gn=alb pe=1 sv=2 |
| IPI00878282 | albu_humanserum albumin os=homo sapiens gn=alb pe=1 sv=2 | P | GO:00082 02 | steroid metabolic process |
| IPI00878282 | albu_humanserum albumin os=homo sapiens gn=alb pe=1 sv=2 | F | GO:00510 87 | chaperone binding |
| IPI00878282 | albu_humanserum albumin os=homo sapiens gn=alb pe=1 sv=2 | C | GO:00444 46 | intracellular organelle part |
| IPI00878282 | albu_humanserum albumin os=homo sapiens gn=alb pe=1 sv=2 | F | GO:00156 43 | toxin binding |
| IPI00878282 | albu_humanserum albumin os=homo sapiens gn=alb pe=1 sv=2 | P | GO:00442 60 | cellular macromolecule metabolic process |
| IPI00878282 | albu_humanserum albumin os=homo sapiens gn=alb pe=1 sv=2 | C | GO:00056 15 | extracellular space |
| IPI00878282 | albu_humanserum albumin os=homo sapiens gn=alb pe=1 sv=2 | P | GO:00516 59 | maintenance of mitochondrion location |
| IPI00878282 | albu_humanserum albumin os=homo sapiens gn=alb pe=1 sv=2 | F | GO:00081 44 | drug binding |
| IPI00878282 | albu_humanserum albumin os=homo sapiens gn=alb pe=1 sv=2 | C | GO:00432 31 | intracellular membrane-bounded organelle |
| IPI00878282 | albu_humanserum albumin os=homo sapiens gn=alb pe=1 sv=2 | P | GO:00442 81 | small molecule metabolic process |
| IPI00878282 | albu_humanserum albumin os=homo sapiens gn=alb pe=1 sv=2 | F | GO:00055 04 | fatty acid binding |
| IPI00878282 | albu_humanserum albumin os=homo sapiens gn=alb pe=1 sv=2 | P | GO:00422 21 | response to chemical stimulus |
| IPI00878282 | albu_humanserum albumin os=homo sapiens gn=alb pe=1 sv=2 | F | GO:00036 77 | DNA binding |
| IPI00878282 | albu_humanserum albumin os=homo sapiens gn=alb pe=1 sv=2 | P | GO:00092 67 | cellular response to starvation |
| IPI00878282 | albu_humanserum albumin os=homo sapiens gn=alb pe=1 sv=2 | F | GO:00301 70 | pyridoxal phosphate binding |
| IPI00878282 | albu_humanserum albumin os=homo sapiens gn=alb pe=1 sv=2 | P | GO:00068 10 | transport |
| IPI00878282 | albu_humanserum albumin os=homo sapiens gn=alb pe=1 sv=2 | F | GO:00198 25 | oxygen binding |
| IPI00878282 | albu_humanserum albumin os=homo sapiens gn=alb pe=1 sv=2 | P | GO:00508 78 | regulation of body fluid levels |
| IPI00878282 | albu_humanserum albumin os=homo sapiens gn=alb pe=1 sv=2 | P | GO:00430 66 | negative regulation of apoptotic process |
| IPI00878282 | albu_humanserum albumin os=homo sapiens gn=alb pe=1 sv=2 | C | GO:00444 44 | cytoplasmic part |
| IPI00878282 | albu_humanserum albumin os=homo sapiens gn=alb pe=1 sv=2 | P | GO:00096 11 | response to wounding |
| IPI00878282 | albu_humanserum albumin os=homo sapiens gn=alb pe=1 sv=2 | P | GO:00198 36 | hemolysis by symbiont of host erythrocytes |
| IPI00878282 | albu_humanserum albumin os=homo sapiens gn=alb pe=1 sv=2 | P | GO:00069 55 | immune response |
| IPI00878282 | albu_humanserum albumin os=homo sapiens gn=alb pe=1 sv=2 | C | GO:00198 14 | immunoglobulin complex |
| IPI00784842 | ighg1_humanig gamma-1 chain c region os=homo sapiens gn=ighg1 pe=1 sv=1 | P | GO:00507 76 | regulation of immune response |
| IPI00784842 | ighg1_humanig gamma-1 chain c region os=homo sapiens gn=ighg1 pe=1 sv=1 | F | GO:00055 15 | protein binding |
| KH 40 | IPI00784842 | AFCC | IPI007848 42 | ighg1_humanig gamma-1 chain c region os=homo sapiens gn=ighg1 pe=1 sv=1 |
| IPI00784842 | ighgl_humanig gamma-1 chain c region os=homo sapiens gn=ighg1 pe=1 sv=1 | P | GO:00507 76 | regulation of immune response |
| IPI00784842 | ighg1_humanig gamma-1 chain c region os=homo sapiens gn=ighg1 pe=1 sv=1 | F | GO:00055 15 | protein binding |
| KH 41 | IPI00022434 | Fraction III-II | same as KH 28 | |
| KH 42 | IPI00298497 | Fraction III | same as KH 36 | |
| KH 43 | IPI00965713 | Fraction III | IPI009657 13 | fibb_humanfibrinogen beta chain os=homo sapiens gn=fgb pe=1 sv=2 |
| IPI00965713 | fibb_humanfibrinogen beta chain os=homo sapiens gn=fgb pe=1 sv=2 | P | GO:00422 21 | response to chemical stimulus |
| IPI00965713 | fibb_humanfibrinogen beta chain os=homo sapiens gn=fgb pe=1 sv=2 | F | GO:00055 15 | protein binding |
| IPI00965713 | fibb_humanfibrinogen beta chain os=homo sapiens gn=fgb pe=1 sv=2 | C | GO:00056 15 | extracellular space |
| IPI00965713 | fibb_humanfibrinogen beta chain os=homo sapiens gn=fgb pe=1 sv=2 | P | GO:00511 79 | localization |
| IPI00965713 | fibb_humanfibrinogen beta chain os=homo sapiens gn=fgb pe=1 sv=2 | C | GO:00310 91 | platelet alpha granule |
| IPI00965713 | fibb_humanfibrinogen beta chain os=homo sapiens gn=fgb pe=1 sv=2 | C | GO:00444 25 | membrane part |
| IPI00965713 | fibb_humanfibrinogen beta chain os=homo sapiens gn=fgb pe=1 sv=2 | P | GO:00507 94 | regulation of cellular process |
| IPI00965713 | fibb_humanfibrinogen beta chain os=homo sapiens gn=fgb pe=1 sv=2 | P | GO:00069 50 | response to stress |
| IPI00965713 | fibb_humanfibrinogen beta chain os=homo sapiens gn=fgb pe=1 sv=2 | C | GO:00058 86 | plasma membrane |
| IPI00965713 | fibb_humanfibrinogen beta chain os=homo sapiens gn=fgb pe=1 sv=2 | P | GO:00325 01 | multicellular organismal process |
| KH 44 | IPI00645363 | FibringluRAA S® Human Thrombin | IPI006453 63 | ighg1_humanig gamma-1 chain c region os=homo sapiens gn=ighg1 pe=1 sv=1 |
| IPI00645363 | ighg1_humanig gamma-1 chain c region os=homo sapiens gn=ighg1 pe=1 sv=1 | P | GO:00507 76 | regulation of immune response |
| IPI00645363 | ighg1_humanig gamma-1 chain c region os=homo sapiens gn=ighg1 pe=1 sv=1 | F | GO:00055 15 | protein binding |
| KH 45 | IPI00219713 | FibringluRAA S® Human Thrombin | same as KH 30 | |
| IPI00219713 | fibg_humanfibrinogen gamma chain os=homo sapiens gn=fgg pe=1 sv=3 | P | GO:00099 87 | cellular process |
| IPI00219713 | fibg_humanfibrinogen gamma chain os=homo sapiens gn=fgg pe=1 sv=3 | C | GO:00098 97 | external side of plasma membrane |
| IPI00219713 | fibg_humanfibrinogen gamma chain os=homo sapiens gn=fgg pe=1 sv=3 | F | GO:00434 99 | eukaryotic cell surface binding |
| IPI00219713 | fibg_humanfibrinogen gamma chain os=homo sapiens gn=fgg pe=1 sv=3 | C | GO:00056 15 | extracellular space |
| IPI00219713 | fibg_humanfibrinogen gamma chain os=homo sapiens gn=fgg pe=1 sv=3 | C | GO:00310 91 | platelet alpha granule |
| IPI00219713 | fibg_humanfibrinogen gamma chain os=homo sapiens gn=fgg pe=1 sv=3 | P | GO:00325 01 | multicellular organismal process |
| IPI00219713 | fibg_humanfibrinogen gamma chain os=homo sapiens gn=fgg pe=1 sv=3 | P | GO:00650 07 | biological regulation |
| IPI00219713 | fibg_humanfibrinogen gamma chain os=homo sapiens gn=fgg pe=1 sv=3 | P | GO:00515 92 | response to calcium ion |
| KH 46 | IPI00022371 | FibringluRAA S® Human Thrombin | IPI000223 71 | hrg_humanhistidine-rich glycoprotein os=homo sapiens gn=hrg pe=1 sv=1 |
| IPI00022371 | hrg_humanhistidine-rich glycoprotein os=homo sapiens gn=hrg pe=1 sv=1 | P | GO:00430 65 | positive regulation of apoptotic process |
| IPI00022371 | hrg_humanhistidine-rich glycoprotein os=homo sapiens gn=hrg pe=1 sv=1 | P | GO:00104 68 | regulation of gene expression |
| IPI00022371 | hrg_humanhistidine-rich glycoprotein os=homo sapiens gn=hrg pe=1 sv=1 | P | GO:00329 56 | regulation of actin cytoskeleton organization |
| IPI00022371 | hrg_humanhistidine-rich glycoprotein os=homo sapiens gn=hrg pe=1 sv=1 | P | GO:00165 25 | negative regulation of angiogenesis |
| IPI00022371 | hrg_humanhistidine-rich glycoprotein os=homo sapiens gn=hrg pe=1 sv=1 | P | GO:20005 04 | positive regulation of blood vessel remodeling |
| IPI00022371 | hrg_humanhistidine-rich glycoprotein os=homo sapiens gn=hrg pe=1 sv=1 | P | GO:00432 54 | regulation of protein complex assembly |
| IPI00022371 | hrg_humanhistidine-rich glycoprotein os=homo sapiens gn=hrg pe=1 sv=1 | P | GO:00028 39 | positive regulation of immune response to tumor cell |
| IPI00022371 | hrg_humanhistidine-rich glycoprotein os=homo sapiens gn=hrg pe=1 sv=1 | F | GO:00082 01 | heparin binding |
| IPI00022371 | hrg_humanhistidine-rich glycoprotein os=homo sapiens gn=hrg pe=1 sv=1 | P | GO:00105 93 | negative regulation of lamellipodium assembly |
| IPI00022371 | hrg_humanhistidine-rich glycoprotein os=homo sapiens gn=hrg pe=1 sv=1 | P | GO:00508 32 | defense response to fungus |
| IPI00022371 | hrg_humanhistidine-rich glycoprotein os=homo sapiens gn=hrg pe=1 sv=1 | F | GO:00200 37 | heme binding |
| IPI00022371 | hrg_humanhistidine-rich glycoprotein os=homo sapiens gn=hrg pe=1 sv=1 | F | GO:00198 65 | immunoglobulin binding |
| IPI00022371 | hrg_humanhistidine-rich glycoprotein os=homo sapiens gn=hrg pe=1 sv=1 | P | GO:00301 68 | platelet activation |
| IPI00022371 | hrg_humanhistidine-rich glycoprotein os=homo sapiens gn=hrg pe=1 sv=1 | F | GO:00433 95 | heparan sulfate proteoglycan binding |
| IPI00022371 | hrg_humanhistidine-rich glycoprotein os=homo sapiens gn=hrg pe=1 sv=1 | P | GO: 19007 47 | negative regulation of vascular endothelial growth factor signaling pathway |
| IPI00022371 | hrg_humanhistidine-rich glycoprotein os=homo sapiens gn=hrg pe=1 sv=1 | F | GO:00082 70 | zinc ion binding |
| IPI00022371 | hrg_humanhistidine-rich glycoprotein os=homo sapiens gn=hrg pe=1 sv=1 | F | GO:00434 98 | cell surface binding |
| IPI00022371 | hrg_humanhistidine-rich glycoprotein os=homo sapiens gn=hrg pe=1 sv=1 | P | GO:20010 27 | negative regulation of endothelial cell chemotaxis |
| IPI00022371 | hrg_humanhistidine-rich glycoprotein os=homo sapiens gn=hrg pe=1 sv=1 | P | GO:00082 85 | negative regulation of cell proliferation |
| IPI00022371 | hrg_humanhistidine-rich glycoprotein os=homo sapiens gn=hrg pe=1 sv=1 | P | GO:00518 94 | positive regulation of focal adhesion assembly |
| IPI00022371 | hrg_humanhistidine-rich glycoprotein os=homo sapiens gn=hrg pe=1 sv=1 | P | GO:00301 93 | regulation of blood coagulation |
| IPI00022371 | hrg_humanhistidine-rich glycoprotein os=homo sapiens gn=hrg pe=1 sv=1 | P | GO:00507 30 | regulation of peptidyl-tyrosine phosphorylation |
| IPI00022371 | hrg_humanhistidine-rich glycoprotein os=homo sapiens gn=hrg pe=1 sv=1 | P | GO:00336 29 | negative regulation of cell adhesion mediated by integrin |
| IPI00022371 | hrg_humanhistidine-rich glycoprotein os=homo sapiens gn=hrg pe=1 sv=1 | P | GO:00303 08 | negative regulation of cell growth |
| IPI00022371 | hrg_humanhistidine-rich glycoprotein os=homo sapiens gn=hrg pe=1 sv=1 | C | GO:00055 76 | extracellular region |
| IPI00022371 | hrg_humanhistidine-rich glycoprotein os=homo sapiens gn=hrg pe=1 sv=1 | C | GO:00198 14 | immunoglobulin complex |
| KH 47 | IPI00022371 | FibringluRAA S® Human Thrombin | same as KH 46 | |
| KH 48 | IPI00022463 | AFOD | IPI000224 63 | trfe_humanserotransferrin os=homo sapiens gn=tf pe=1 sv=3 |
| IPI00022463 | trfe_humanserotransferrin os=homo sapiens gn=tf pe=1 sv=3 | P | GO:00099 87 | cellular process |
| IPI00022463 | trfe_humanserotransferrin os=homo sapiens gn=tf pe=1 sv=3 | P | GO:00650 08 | regulation of biological quality |
| IPI00022463 | trfe_humanserotransferrin os=homo sapiens gn=tf pe=1 sv=3 | P | GO:00068 10 | transport |
| IPI00022463 | trfe_humanserotransferrin os=homo sapiens gn=tf pe=1 sv=3 | C | GO:00099 25 | basal plasma membrane |
| IPI00022463 | trfe_humanserotransferrin os=homo sapiens gn=tf pe=1 sv=3 | C | GO:00057 39 | mitochondrion |
| IPI00022463 | trfe_humanserotransferrin os=homo sapiens gn=tf pe=1 sv=3 | C | GO:00301 39 | endocytic vesicle |
| IPI00022463 | trfe_humanserotransferrin os=homo sapiens gn=tf pe=1 sv=3 | C | GO:00059 05 | coated pit |
| IPI00022463 | trfe_humanserotransferrin os=homo sapiens gn=tf pe=1 sv=3 | C | GO:00057 70 | late endosome |
| IPI00022463 | trfe_humanserotransferrin os=homo sapiens gn=tf pe=1 sv=3 | C | GO:00057 69 | early endosome |
| IPI00022463 | trfe_humanserotransferrin os=homo sapiens gn=tf pe=1 sv=3 | C | GO:00550 37 | recycling endosome |
| IPI00022463 | trfe_humanserotransferrin os=homo sapiens gn=tf pe=1 sv=3 | F | GO:00055 15 | protein binding |
| IPI00022463 | trfe_humanserotransferrin os=homo sapiens gn=tf pe=1 sv=3 | C | GO:00484 71 | perinuclear region of cytoplasm |
| IPI00022463 | trfe_humanserotransferrin os=homo sapiens gn=tf pe=1 sv=3 | C | GO:00163 24 | apical plasma membrane |
| IPI00022463 | trfe_humanserotransferrin os=homo sapiens gn=tf pe=1 sv=3 | P | GO:00069 50 | response to stress |
| KH 49 | IPI00023006 | AFOD | IPI000230 06 | alpha cardiac muscle 1 os=homo sapiens gn=actc1 pe=1 sv=1 |
| IPI00023006 | alpha cardiac muscle 1 os=homo sapiens gn=actc1 pe=1 sv=1 | C | GO:00058 65 | striated muscle thin filament |
| IPI00023006 | alpha cardiac muscle 1 os=homo sapiens gn=actc1 pe=1 sv=1 | F | GO:00170 22 | myosin binding |
| IPI00023006 | alpha cardiac muscle 1 os=homo sapiens gn=actc1 pe=1 sv=1 | P | GO:00302 40 | skeletal muscle thin filament assembly |
| IPI00023006 | alpha cardiac muscle 1 os=homo sapiens gn=actc1 pe=1 sv=1 | P | GO:00062 00 | ATP catabolic process |
| IPI00023006 | alpha cardiac muscle 1 os=homo sapiens gn=actc1 pe=1 sv=1 | P | GO:00721 44 | glomerular mesangial cell development |
| IPI00023006 | alpha cardiac muscle 1 os=homo sapiens gn=actc1 pe=1 sv=1 | P | GO:00069 36 | muscle contraction |
| IPI00023006 | alpha cardiac muscle 1 os=homo sapiens gn=actc1 pe=1 sv=1 | P | GO:00332 75 | actin-myosin filament sliding |
| IPI00023006 | alpha cardiac muscle 1 os=homo sapiens gn=actc1 pe=1 sv=1 | C | GO:00426 43 | actomyosin, actin part |
| IPI00023006 | alpha cardiac muscle 1 os=homo sapiens gn=actc1 pe=1 sv=1 | P | GO:00422 21 | response to chemical stimulus |
| IPI00023006 | alpha cardiac muscle 1 os=homo sapiens gn=actc1 pe=1 sv=1 | F | GO:00055 24 | ATP binding |
| IPI00023006 | alpha cardiac muscle 1 os=homo sapiens gn=actc1 pe=1 sv=1 | C | GO:00017 25 | stress fiber |
| IPI00023006 | alpha cardiac muscle 1 os=homo sapiens gn=actc1 pe=1 sv=1 | F | GO:00168 87 | ATPase activity |
| IPI00023006 | alpha cardiac muscle 1 os=homo sapiens gn=actc1 pe=1 sv=1 | P | GO:00650 08 | regulation of biological quality |
| IPI00023006 | alpha cardiac muscle 1 os=homo sapiens gn=actc1 pe=1 sv=1 | C | GO:00444 51 | nucleoplasm part |
| IPI00023006 | alpha cardiac muscle 1 os=homo sapiens gn=actc1 pe=1 sv=1 | P | GO:00096 15 | response to virus |
| IPI00023006 | alpha cardiac muscle 1 os=homo sapiens gn=actc1 pe=1 sv=1 | P | GO:00600 47 | heart contraction |
| IPI00023006 | alpha cardiac muscle 1 os=homo sapiens gn=actc1 pe=1 sv=1 | F | GO:00198 99 | enzyme binding |
| IPI00023006 | alpha cardiac muscle 1 os=homo sapiens gn=actc1 pe=1 sv=1 | C | GO:00164 59 | myosin complex |
| KH 50 | IPI00021841 | AFOD | same as KH 37 | |
| KH 51 | IPI00023006 | AlbuRAAS | same as KH 49 | |
| KH 52 | IPI00930226 | FibringluRAA S® High Concentrate Human Fibrinogen | IPI009302 26 | cytoplasmic 2 os=homo sapiens gn=actg1 pe=1 sv=1 |
| IPI00930226 | cytoplasmic 2 os=homo sapiens gn=actg1 pe=1 sv=1 | P | GO:00098 88 | tissue development |
| IPI00930226 | cytoplasmic 2 os=homo sapiens gn=actg1 pe=1 sv=1 | P | GO:00300 48 | actin filament-based movement |
| IPI00930226 | cytoplasmic 2 os=homo sapiens gn=actg1 pe=1 sv=1 | P | GO:00030 12 | muscle system process |
| IPI00930226 | cytoplasmic 2 os=homo sapiens gn=actg1 pe=1 sv=1 | C | GO:00300 17 | sarcomere |
| IPI00930226 | cytoplasmic 2 os=homo sapiens gn=actg1 pe=1 sv=1 | P | GO:00302 39 | myofibril assembly |
| IPI00930226 | cytoplasmic 2 os=homo sapiens gn=actg1 pe=1 sv=1 | P | GO:00442 38 | primary metabolic process |
| IPI00930226 | cytoplasmic 2 os=homo sapiens gn=actg1 pe=1 sv=1 | C | GO:00058 84 | actin filament |
| IPI00930226 | cytoplasmic 2 os=homo sapiens gn=actg1 pe=1 sv=1 | P | GO:00723 58 | cardiovascular system development |
| IPI00930226 | cytoplasmic 2 os=homo sapiens gn=actg1 pe=1 sv=1 | P | GO:00442 37 | cellular metabolic process |
| IPI00930226 | cytoplasmic 2 os=homo sapiens gn=actg1 pe=1 sv=1 | P | GO:00485 13 | organ development |
| IPI00930226 | cytoplasmic 2 os=homo sapiens gn=actg1 pe=1 sv=1 | P | GO:00422 21 | response to chemical stimulus |
| IPI00930226 | cytoplasmic 2 os=homo sapiens gn=actg1 pe=1 sv=1 | F | GO:00080 92 | cytoskeletal protein binding |
| IPI00930226 | cytoplasmic 2 os=homo sapiens gn=actg1 pe=1 sv=1 | P | GO:00650 08 | regulation of biological quality |
| IPI00930226 | cytoplasmic 2 os=homo sapiens gn=actg1 pe=1 sv=1 | C | GO:00444 51 | nucleoplasm part |
| IPI00930226 | cytoplasmic 2 os=homo sapiens gn=actg1 pe=1 sv=1 | P | GO:00080 15 | blood circulation |
| IPI00930226 | cytoplasmic 2 os=homo sapiens gn=actg1 pe=1 sv=1 | F | GO:00198 99 | enzyme binding |
| KH 53 | 194373497 | AFCC (Fraction IV) | gi\|1943734 97 | thrb_humanprothrombin os=homo sapiens gn=f2 pe=1 sv=2 |
| gi\|19437349 7 | thrb_humanprothrombin os=homo sapiens gn=f2 pe=1 sv=2 | C | GO:00444 46 | intracellular organelle part |
| gi\|19437349 7 | thrb_humanprothrombin os=homo sapiens gn=f2 pe=1 sv=2 | P | GO:00487 12 | negative regulation of astrocyte differentiation |
| gi\|19437349 7 | thrb_humanprothrombin os=homo sapiens gn=f2 pe=1 sv=2 | C | GO:00432 33 | organelle lumen |
| gi\|19437349 7 | thrb_humanprothrombin os=homo sapiens gn=f2 pe=1 sv=2 | P | GO:00301 94 | positive regulation of blood coagulation |
| gi\|19437349 7 | thrb_humanprothrombin os=homo sapiens gn=f2 pe=1 sv=2 | F | GO:00051 02 | receptor binding |
| gi\|19437349 7 | thrb_humanprothrombin os=homo sapiens gn=f2 pe=1 sv=2 | P | GO:20003 79 | positive regulation of reactive oxygen species metabolic process |
| gi\|19437349 7 | thrb_humanprothrombin os=homo sapiens gn=f2 pe=1 sv=2 | P | GO:00458 61 | negative regulation of proteolysis |
| gi\|19437349 7 | thrb_humanprothrombin os=homo sapiens gn=f2 pe=1 sv=2 | C | GO:00056 15 | extracellular space |
| gi\|19437349 7 | thrb_humanprothrombin os=homo sapiens gn=f2 pe=1 sv=2 | P | GO:00301 68 | platelet activation |
| gi\|19437349 7 | thrb_humanprothrombin os=homo sapiens gn=f2 pe=1 sv=2 | P | GO: 19007 38 | positive regulation of phospholipase C-activating G-protein coupled receptor signaling pathway |
| gi\|19437349 7 | thrb_humanprothrombin os=homo sapiens gn=f2 pe=1 sv=2 | P | GO:00164 77 | cell migration |
| gi\|19437349 7 | thrb_humanprothrombin os=homo sapiens gn=f2 pe=1 sv=2 | C | GO:00432 31 | intracellular membrane-bounded organelle |
| gi\|19437349 7 | thrb_humanprothrombin os=homo sapiens gn=f2 pe=1 sv=2 | P | GO:00019 34 | positive regulation of protein phosphorylation |
| gi\|19437349 7 | thrb_humanprothrombin os=homo sapiens gn=f2 pe=1 sv=2 | C | GO:00058 86 | plasma membrane |
| gi\|19437349 7 | thrb_humanprothrombin os=homo sapiens gn=f2 pe=1 sv=2 | F | GO:00700 53 | thrombospondin receptor activity |
| gi\|19437349 7 | thrb_humanprothrombin os=homo sapiens gn=f2 pe=1 sv=2 | P | GO:00512 81 | positive regulation of release of sequestered calcium ion into cytosol |
| gi\|19437349 7 | thrb_humanprothrombin os=homo sapiens gn=f2 pe=1 sv=2 | F | GO:00042 52 | serine-type endopeptidase activity |
| gi\|19437349 7 | thrb_humanprothrombin os=homo sapiens gn=f2 pe=1 sv=2 | P | GO:00427 30 | fibrinolysis |
| gi\|19437349 7 | thrb_humanprothrombin os=homo sapiens gn=f2 pe=1 sv=2 | C | GO:00444 44 | cytoplasmic part |
| gi\|19437349 7 | thrb_humanprothrombin os=homo sapiens gn=f2 pe=1 sv=2 | P | GO:00329 67 | positive regulation of collagen biosynthetic process |
| KH 54 | 194380034 | Transferrin | gi\|1943800 34 | trfe_humanserotransferrin os=homo sapiens gn=tf pe=1 sv=3 |
| gi\|19438003 4 | trfe_humanserotransferrin os=homo sapiens gn=tf pe=1 sv=3 | P | GO:00099 87 | cellular process |
| gi\|19438003 4 | trfe_humanserotransferrin os=homo sapiens gn=tf pe=1 sv=3 | P | GO:00650 08 | regulation of biological quality |
| gi\|19438003 4 | trfe_humanserotransferrin os=homo sapiens gn=tf pe=1 sv=3 | P | GO:00068 10 | transport |
| gi\|19438003 4 | trfe_humanserotransferrin os=homo sapiens gn=tf pe=1 sv=3 | C | GO:00099 25 | basal plasma membrane |
| gi\|19438003 4 | trfe_humanserotransferrin os=homo sapiens gn=tf pe=1 sv=3 | C | GO:00057 39 | mitochondrion |
| gi\|19438003 4 | trfe_humanserotransferrin os=homo sapiens gn=tf pe=1 sv=3 | C | GO:00301 39 | endocytic vesicle |
| gi\|19438003 4 | trfe_humanserotransferrin os=homo sapiens gn=tf pe=1 sv=3 | C | GO:00059 05 | coated pit |
| gi\|19438003 4 | trfe_humanserotransferrin os=homo sapiens gn=tf pe=1 sv=3 | C | GO:00057 70 | late endosome |
| gi\|19438003 4 | trfe_humanserotransferrin os=homo sapiens gn=tf pe=1 sv=3 | C | GO:00057 69 | early endosome |
| gi\|19438003 4 | trfe_humanserotransferrin os=homo sapiens gn=tf pe=1 sv=3 | C | GO:00550 37 | recycling endosome |
| gi\|19438003 4 | trfe_humanserotransferrin os=homo sapiens gn=tf pe=1 sv=3 | F | GO:00055 15 | protein binding |
| gi\|19438003 4 | trfe_humanserotransferrin os=homo sapiens gn=tf pe=1 sv=3 | C | GO:00484 71 | perinuclear region of cytoplasm |
| gi\|19438003 4 | trfe_humanserotransferrin os=homo sapiens gn=tf pe=1 sv=3 | C | GO:00163 24 | apical plasma membrane |
| gi\|19438003 4 | trfe_humanserotransferrin os=homo sapiens gn=tf pe=1 sv=3 | P | GO:00069 50 | response to stress |
| KH 55 | 194380034 | Transferrin | same as KH 54 | |

Additional protein sequence information as well as sequence identifiers and accession numbers for KH proteins 1-55 are found in the table below.

| **KH Protein/SEQ ID NO** | **Sequence Identifier(s)** | **Protein Sequence** |
|---|---|---|
| 1 | gi:21749960 BAC03696.1 | |
| 2 | gi:215415640 CAT02162.1 | |
| 3 | gi:215415638 CAT02161.1 | |
| 4 | gi: 40044478 CAF01015.1 | |
| 5 | gi:194383496 BAG64719.1 | |
| 6 | gi:28071026 CAD61894.1 | |
| 7 | gi:300621695 CBU30464.1 | |
| 8 | gi:1335098 CAA26382.1 | |
| 9 | gi:10434804 BAB14383.1 | |
| 10 | gi:221044726 BAH14040.1 | |
| 11 | gi:215415638 CAT02161.1 | Same as KH3 |
| 12 | gi:189066554 BAG35804.1 | |
| 13 | gi:194391084 BAG60660.1 | |
| 14 | gi:158255114 BAF83528.1 | |
| 15 | gi:213506121 CAS91511.1 | |
| 16 | gi:213506103 CAS91502.1 | |
| 17 | gi:194376310 BAG62914.1 | |
| 18 | gi:194388064 BAG65416.1 | |
| 19 | IPI00964149 gi:126215685 Q8N7B6.2 | |
| 20 | IPI00966721 gi: 121940485 Q0VDI3.1 | |
| 21 | IPI00966826 gi:121947590 Q29RF7.1 | |
| 22 | IPI00760788 gi:109892504 Q53GT1.2 | |
| 23 | IPI00917278 gi: | |
| 24 | IPI00966721 gi: 121940485 Q0VDI3.1 | Same as KH 20 |
| 25 | IPI01012037 gi:74735024 Q9UHY7.1 | |
| 26 | IPI00940730 gi: | |
| 27 | IPI00977191 gi:23503077 P49321.2 | |
| 28 | IPI00022434 gi: 113576 P02768.2 | |
| 29 | IPI00022434 gi:113576 P02768.2 | Same as KH 28 |
| 30 | IPI00219713 gi:20178280 P02679 | |
| 31 | IPI00219713 gi:20178280 P02679 | Same as KH 30 |
| 32 | IPI00220327 gi:238054406 P04264.6 | |
| 33 | IPI00029739 gi:158517847 P08603.4 | |
| 34 | IPI00384853 gi: | QAHGRCSAGAQFVFCRRSAGAACTQQALSR (Sequence 59-88) |
| | | CLVGAQCVLSR (Sequence 100-110) |
| | | CTVCTQQALSR (Sequence 125-135) |
| 35 | IPI00479708 gi:193806374 P01871.3 | |
| 36 | IPI00298497 gi:399492 P02675.2 | |
| 37 | IPI00021841 gi:113992 P02647.1 | |
| 38 | IPI00783987 gi: 119370332 P01024.2 | |
| 39 | IPI00878282 gi:113576 P02768.2 | |
| 40 | IPI00784842 gi: | GRFTISGDISTNTLYLQMHSLR (Sequence 85-106) |
| | | TPEVTCVVVDVSHEDPEVKFNWYVDGVEVHNAK (Sequence 284-316) |
| | | ALPAPIEK (Sequence 355-362) |
| | | GQPREPQVYTLPPSRDELTKGFYPSDIAVEWESNGQPENNYK (Sequence 369-420) |
| 41 | IPI00022434 gi: 113576 P02768.2 | Same as KH28 |
| 42 | IPI00298497 gi:399492 P02675.2 | Same as KH36 |
| 43 | IPI00965713 gi:399492 P02675.2 | |
| 44 | IPI00645363 gi : | NSLYLQMNSLRAEDTALYYCAK (Sequence 96-117) |
| | | GPSVFPLAPSSK (Sequence 147-158) |
| | | TPEVTCVVVDVSHEDPEVK (Sequence 281-299) |
| | | FNWYVDGVEVHNAK (Sequence 300-313) |
| | | ALPAPIEK (Sequence 352-359) |
| | | GQPREPQVYTLPPSRDELTKNQVSLTCLVKGFYPSDIAVEWESNGQPENNYK (Sequence 366-417) |
| 45 | IPI00219713 gi:20178280 P02679 | Same as KH30 |
| 46 | IPI00022371 gi:123523 P04196.1 | |
| 47 | IPI00022371 gi:123523 P04196.1 | Same as KH46 |
| 48 | IPI00022463 gi:313104271 P02787.3 | |
| 49 | IPI00023006 gi:54036697 P68032.1 | |
| 50 | IPI00021841 gi:113992 P02647.1 | Same as KH 37 |
| 51 | IPI00023006 gi:54036697 P68032.1 | Same as KH49 |
| 52 | IPI00930226 gi:54036678 P63261.1 | |
| **KH Protein/SEQ ID NO** | **Sequence Identifier(s)** | **Protein Sequence** |
| 53 | gi:194373497 BAG56844.1 | |
| 54 | gi:194380034 BAG58369.1 | |
| 55 | gi:194380034 BAG58369.1 | Same as 54 |

The details of one or more embodiments of the present invention are set forth in the accompanying figures and the description below. Further areas of applicability will become apparent from the description provided herein.

### Brief Description of the Drawings:

Figure 1 is a graph depicting percentages of T and B lymphocytes in peripheral blood, with and without therapeutic RAAS 105 treatment.
Figure 2 is a graph depicting percentages of T and B lymphocytes in peripheral blood, with further analysis done on CD4 and CD8 T cell lineages, with and without therapeutic RAAS 105 treatment.
Figure 3 is a graph depicting percentages of CD4 and CD8 T cells in peripheral blood, with and without therapeutic RAAS 105 treatment.
Figure 4 is a graph depicting percentages of CD4 and CD8 T cells in peripheral blood, with further analysis done on the percentages of CD1 1c⁺ dendritic cells (DC) and Gr-1⁺ granulocytes.
Figure 5 is graphs depicting percentages of dendritic cells and granulocytes in peripheral blood, with and without therapeutic RAAS 105 treatment.
Figure 6 is graph showing another representation of Gr-1 vs. CD 11 c cells, with and without therapeutic RAAS 105 treatment.
Figure 7 is a graph depicting the percentage of monocytes in peripheral blood, with and without therapeutic RAAS 105 treatment.
Figure 8 is a graph showing another representation of monocytes in peripheral blood, with and without therapeutic RAAS 105 treatment.
Figure 9 is graphs depicting percentages of T and B lymphocytes in the spleen, with and without therapeutic RAAS 105 treatment.
Figure 10 is a graph showing another representation of T and B lymphocytes in the spleen, with and without therapeutic RAAS 105 treatment.
Figure 11 is graphs depicting percentages of CD4 and CD8 T cells in the spleen, with and without therapeutic RAAS 105 treatment.
Figure 12 is a graph showing another representation of CD4 and CD8 T cells in the spleen, with CD3 T cells being gated, with and without therapeutic RAAS 105 treatment.
Figure 13 is graphs depicting T cell subset percentages in the spleen, with and without therapeutic RAAS 105 treatment.
Figure 14 is a graph of CD4 T cell subset percentages in the spleen, with and without therapeutic RAAS 105 treatment.
Figure 15 is graphs depicting T cell subset percentages in the spleen, with and without therapeutic RAAS 105 treatment.
Figure 16 is a graph of CD8 T cell subset percentages in the spleen, with and without therapeutic RAAS 105 treatment.
Figure 17 is a graph depicting percentages of regulatory T cells in the spleen, with and without therapeutic RAAS 105 treatment.
Figure 18 is another graphical representation of percentages of regulatory T cells in the spleen, with and without therapeutic RAAS 105 treatment.
Figure 19 is graphs depicting percentages of mDc and pDcs in the spleen, with and without therapeutic RAAS 105 treatment.
Figure 20 is another graphical representation of mDC and pDcs in the spleen, with and without therapeutic RAAS 105 treatment.
Figure 21 is graphs depicting percentages of macrophages and granulocytes in the spleen, with and without therapeutic RAAS 105 treatment.
Figure 22 is another graphical representation of percentages of macrophages and granulocytes in the spleen, with and without therapeutic RAAS 105 treatment.
Figure 23 is a graph depicting percentages of T cells in the lymph nodes, with and without therapeutic RAAS 105 treatment.
Figure 24 is graphs showing percentages of CD3 T cells in the lymph nodes, with and without therapeutic RAAS 105 treatment.
Figure 25 is graphs depicting percentages of CD4 and CD8 T cells in the lymph nodes, with and without therapeutic RAAS 105 treatment.
Figure 26 is another graphical representation of CD4 and CD8 T cells in the lymph nodes, with and without therapeutic RAAS 105 treatment.
Figure 27 is graphs depicting CD4 T cell subset percentages in the lymph nodes, with and without therapeutic RAAS 105 treatment.
Figure 28 is another graphical representation of CD4 T cell subset percentages in the lymph nodes, with and without therapeutic RAAS 105 treatment.
Figure 29 is graphs depicting CD8 T cell subset percentages in the lymph nodes, with and without therapeutic RAAS 105 treatment.
Figure 30 is another graphical representation of CD8 T cell subset percentages in the lymph nodes, with and without therapeutic RAAS 105 treatment.
Figure 31 is a graph depicting percentages of Foxp3 regulatory T cells in the lymph nodes, with and without therapeutic RAAS 105 treatment.
Figure 32 is another graphical representation of Foxp3 regulatory T cells in the lymph nodes, with and without therapeutic RAAS 105 treatment.
Figure 33 is a graph depicting percentages of DCs in the lymph nodes, with and without therapeutic RAAS 105 treatment.
Figure 34 is another graphical representation of percentages of DCs in the lymph nodes, with and without therapeutic RAAS 105 treatment.
Figure 35 is graphs depicting percentages of macrophages and granulocytes in the lymph nodes, with and without therapeutic RAAS 105 treatment.
Figure 36 is another graphical representation of percentages of macrophages and granulocytes in the lymph nodes, with and without therapeutic RAAS 105 treatment.
Figure 37 is graphs depicting T and B lymphocytes in peripheral blood, with and without prophylactic RAAS 105 treatment.
Figure 38 is another graphical representation of T and B cells in peripheral blood, with and without prophylactic RAAS 105 treatment.
Figure 39 is graphs depicting percentages of CD4 and CD 8 T cells in peripheral blood, with and without prophylactic RAAS 105 treatment.
Figure 40 is another graphical representation of CD4 and CD 8 T cells in peripheral blood, with and without prophylactic RAAS 105 treatment.
Figure 41 is graphs depicting percentages of dendritic cells and granulocytes in peripheral blood, with and without prophylactic RAAS 105 treatment.
Figure 42 is another graphical representation of dendritic cells and granulocytes in peripheral blood, with and without prophylactic RAAS 105 treatment.
Figure 43 is a graph depicting percentages of monocytes in peripheral blood, with and without prophylactic RAAS 105 treatment.
Figure 44 is another graphical representation of percentages of monocytes in peripheral blood, with and without prophylactic RAAS 105 treatment.
Figure 45 is graphs depicting percentages of T and B lymphocytes in the spleen, with and without prophylactic RAAS 105 treatment.
Figure 46 is another graphical representation of percentages of T and B lymphocytes in the spleen, with and without prophylactic RAAS 105 treatment.
Figure 47 is graphs depicting percentages of CD4 and CD8 T cells in the spleen, with and without prophylactic RAAS 105 treatment.
Figure 48 is another graphical representation of percentages of CD4 and CD8 T cells in the spleen, with and without prophylactic RAAS 105 treatment.
Figure 49 is graphs depicting subset percentages of T cells in the spleen, with and without prophylactic RAAS 105 treatment.
Figure 50 is another graphical representation of subset percentages of T cells in the spleen, with and without prophylactic RAAS 105 treatment.
Figure 51 is graphs depicting subset percentages of T cells in the spleen, with and without prophylactic RAAS 105 treatment.
Figure 52 is another graphical representation of subset percentages of T cells in the spleen, with and without prophylactic RAAS 105 treatment.
Figure 53 is a graph depicting Foxp3 regulator T cells in the spleen, with and without prophylactic RAAS 105 treatment.
Figure 54 is another graphical representation of Foxp3 regulator T cells in the spleen, with and without prophylactic RAAS 105 treatment.
Figure 55 is graphs depicting percentages of pDCs and mDCs in the spleen, with and without prophylactic RAAS 105 treatment.
Figure 56 is another graphical representation of percentages of pDCs and mDCs in the spleen, with and without prophylactic RAAS 105 treatment.
Figure 57 is graphs depicting percentages of macrophages and granulocytes in the spleen, with and without prophylactic RAAS 105 treatment.
Figure 58 is another graphical representation of percentages of macrophages and granulocytes in the spleen, with and without prophylactic RAAS 105 treatment.
Figure 59 is a graph depicting percentages of T cells in the lymph nodes, with and without prophylactic RAAS 105 treatment.
Figure 60 is another graphical representation of percentages of CD3 T cells in the lymph nodes, with and without prophylactic RAAS 105 treatment..
Figure 61 is graphs depicting percentages of CD4 and CD8 T cells in the lymph nodes, with and without prophylactic RAAS 105 treatment.
Figure 62 is another graphical representation of percentages of CD4 and CD8 T cells in the lymph nodes, with and without prophylactic RAAS 105 treatment.
Figure 63 is graphs depicting T cell subset percentages in the lymph nodes, with and without prophylactic RAAS 105 treatment.
Figure 64 is another graphical representation of T cell subset percentages in the lymph nodes, with and without prophylactic RAAS 105 treatment.
Figure 65 is graphs depicting T cell subset percentages in the lymph nodes, with and without prophylactic RAAS 105 treatment.
Figure 66 is another graphical representation of T cell subset percentages in the lymph nodes, with and without prophylactic RAAS 105 treatment.
Figure 67 is a graph depicting percentages of Foxp3 regulatory T cells in the lymph nodes, with and without prophylactic RAAS 105 treatment.
Figure 68 is another graphical representation of Foxp3 regulatory T cells in the lymph nodes, with and without prophylactic RAAS 105 treatment.
Figure 69 is a graph depicting percentages of DCs in the lymph nodes, with and without prophylactic RAAS 105 treatment.
Figure 70 is another graphical representation of percentages of DCs in the lymph nodes, with and without prophylactic RAAS 105 treatment.
Figure 71 is graphs depicting percentages of macrophages and granulocytes in the lymph nodes, with and without prophylactic RAAS 105 treatment.
Figure 72 is another graphical representation of percentages of macrophages and granulocytes in the lymph nodes, with and without prophylactic RAAS 105 treatment.
Figure 73 is a process flowchart of the manufacturing of AFOD RAAS 102 from fraction II+III paste.
Figure 74 is a process flowchart of the manufacturing of AFOD RAAS 104 HBIG purification process from Fraction II+III paste.
Figure 75 is a protein analysis of HBIG beside the immunoglobulin proteins containing the protein TF serotransferrin.
Figure 76 is a protein analysis comparison of immunoglobulin from fraction II+III paste, immunoglobulin produced from fraction III paste, and hepatitis B immunoglobulin produced from fraction II+III paste, including a depiction of the different proteins in each of the products alongside the main immunoglobulin protein analysis.
Figure 77 is a process flowchart for AFOD RAAS 105.
Figure 78 is a process flowchart for AFOD RAAS 105.

### Detailed Description of the Invention:

### Characterization of lymphoid tissues and peripheral blood in HBV infected BALB/c mice treated with RAAS 105

### Executive Summary

Investigation was made into the effects of RAAS 105 on multiple cell lineages in lymphoid tissues and peripheral blood in HBV infected BALB/c mice. HBV infection and RAAS 105 treatment were performed by ID unit at Wuxi. At the termination, blood samples and lymphoid tissues were provided to us for analysis of various cell lineages by FACS.

Two independent experiments were performed. One experiment was to test therapeutic effects of RAAS 105 and the other experiment was to test prophylactic effects of RAAS 105.

Compared with the vehicle group, the differences observed in the animals treated with RAAS 105 therapeutically include: 1) percentages of T cells and B cells in peripheral blood, spleen and lymph nodes were decreased significantly; 2) CD62L was greatly downregulated on both CD4⁺ and CD8⁺T cells in the spleen and lymph nodes;3) granulocytes and monocytes/macrophages in peripheral blood and lymph nodes increased significantly; 4) the percentages of regulatory T cells (CD4⁺CD25⁺Foxp3⁺) in the spleen and lymph nodes were increased significantly.

However, prophylactic treatment with RAAS 105 led to somewhat different results. In the group treated with RAAS 105, T- and B-lymphocytes were also decreased. The percentages of monocytes and macrophages were increased albeit to a less degree.

These results suggested that administration of RAAS 105 had significant effects on the frequencies of immune cell lineages.

### List of Abbreviations

| | |
|---|---|
| FACS | Flow Cytometry |
| mDC | Myeloid dendritic cell |
| pDC | Plasmacytoid dendritic cell |

### Materials and Methods

### Materials

### Reagents

FITC, Rat Anti-Mouse CD4, BD, Cat: 557307
PerCP-Cy5.5, Rat Anti-Mouse CD4, BD, Cat: 550954
FITC, Rat Anti-MouseCD3 molecular complex, BD, Cat: 561798
PerCP-Cy5.5, Rat Anti-Mouse CD3, BD, Cat:560527
PerCP-Cy5.5, Rat Anti-Mouse CD8a, BD, Cat: 551162
PE, Rat Anti-MouseCD8a, BD, Cat:553032
PE, Rat Anti-Mouse B220/CD45R, BD, Cat: 553089
APC, Rat Anti-Mouse CD11b, BD, Cat: 553312
APC, Ar Ham Anti-Mouse CD11c, BD, Cat: 550261
PE, Rat Anti-Mouse CD62L, BD, Cat: 553151
APC, Rat Anti-Mouse CD44, BD, Cat: 559250
PE,Rat Anti-Mouse Gr-1(Ly-6G and Ly-6C), BD, Cat: 553128
Alexa Fluor® 647, Rat Anti-Mouse Foxp3, BD, Cat: 560401
PerCP-Cy5.5, Rat Anti-Mouse CD19, BD, Cat: 551001
PE, Rat Anti-Mouse CD25, BD, Cat: 553075
ACK Lysing buffer, Invitrogen, Cat: A10492-01
RPMI 1640 medium, Invitrogen Gibco, Cat: 22400105
Dulbecco's Phosphate Buffered Saline, Thermo. Cat: SH30028.01B.
Fetal bovine serum, Invitrogen Gibco, Cat: 10099141

### Equipment

Vi-CELL Cell Viability Analyzer, Beckman Coulter, Cat: 731050
FACS Caliburflow cytometer, BD, Cat: 342975
Cell strainer (70µm), BD, Cat: 352350
BD Falcon tubes (12x75 mm, 5 ml), BD, Cat: 352054

### Methods

Peripheral blood was collected through cardiac puncture. After removing red blood cells with lysis buffer followed by two rounds of washing using 1 ×PBS, mononuclear cells (monocytes, macrophages, dendritic cells, and lymphocytes) and granulocytes were obtained. Spleen and lymph nodes cell suspension were obtained after filtering through 70µm cell strainer. Cell viability and number were analyzed by Vi-CELL Cell Viability Analyzer followed by cell surface staining. Cells were centrifuged and resuspended in staining buffer (0.08% NaN₃/PBS+ 1% FBS) containing appropriate fluorescent-conjugated antibodies. After 30 min incubation at 4 °C in the dark, cells were washed twice with 0.08% NaN₃/PBS (200 µl per sample), and resuspended with 400 µl 0.08% NaN₃/PBS in BD Falcon tubes (12x75 mm, 5 ml) followed by FACS analysis.

### Data analysis

FACS data were analyzed by flowjo software.

### Study Summary

### Study initiation date and completion date

To investigate the therapeutic and prophylactic effect of RAAS 105 on the immune system in mice infected with HBV, the study had divided into two parts.

### Study purpose

The purpose of this study was to investigate the effect of RAAS 105 on cellular composition in lymphoid tissues and peripheral blood of HBV infected mice treated with RAAS 105.

### Study results

### Effect of therapeutic treatment with RAAS 105

### Mice information

Total 10 female BALB/c mice including 2 naive mice at the same age were transferred from Infectious Disease (ID) Group of WuxiApptec. The group and the regimen information were shown by Table 1.

**Table 1. The experimental group and dosing regimen of the 1^{st} part of the study Cell**

| **Groups** | **N** | **Group ID** | **Dose** | **1^{st} or last dosing** | **Analysis** |
|---|---|---|---|---|---|
| 1 | 4 | Therapeutic vehicle | -- | 1^{St}, 4 hrs post-HDI | Day 5 |
| 3 | 4 | Therapeutic RAAS 105 | 0.4 ml/mouse | 1^{st,} 4 hrs post-HDI | Day 5 |
| 11 | 2 | Naive | - | - | - |

### populations in peripheral blood

After removing red blood cells, T cell lineages, B cells, DCs, granulocytes, and monocytes/macrophages in peripheral blood were analyzed by FACS analysis.

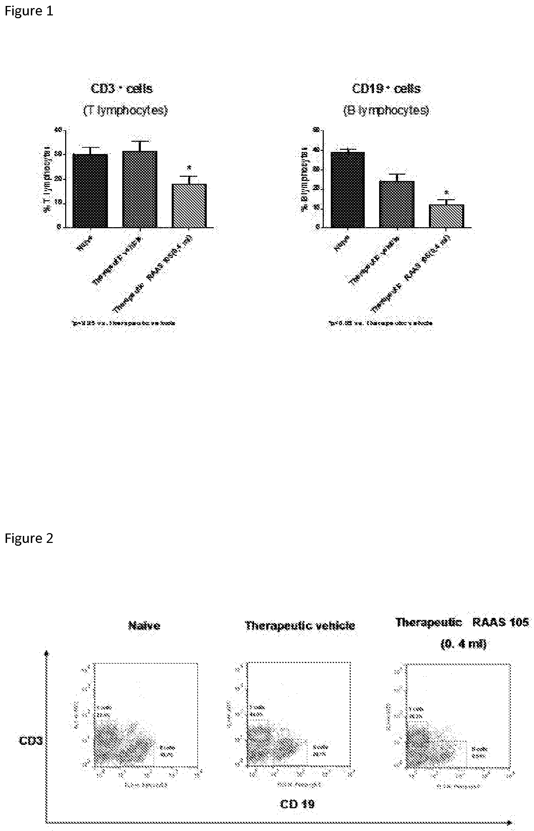

Total T cells and B cells were characterized by CD3 and CD19, respectively. HBV infection did not change the percentages of CD3⁺ T cells compared with naive mice. Therapeutic treatment of RAAS 105 reduced the percentages of both CD3⁺ T cells and CD19⁺B cells significantly (Figure 1). The representative FACS profiles from each group were illustrated in Figure 2.

**Figure 1**. Percentages of T and B lymphocytes in peripheral blood. Total lymphocytes were gated. After therapeutic treated by RAAS 105, percentages of T/B cells significantly decreased in peripheral blood. (by test)

**Figure 2**. Percent of T cells and B cells in peripheral blood. Total lymphocytes were gated.

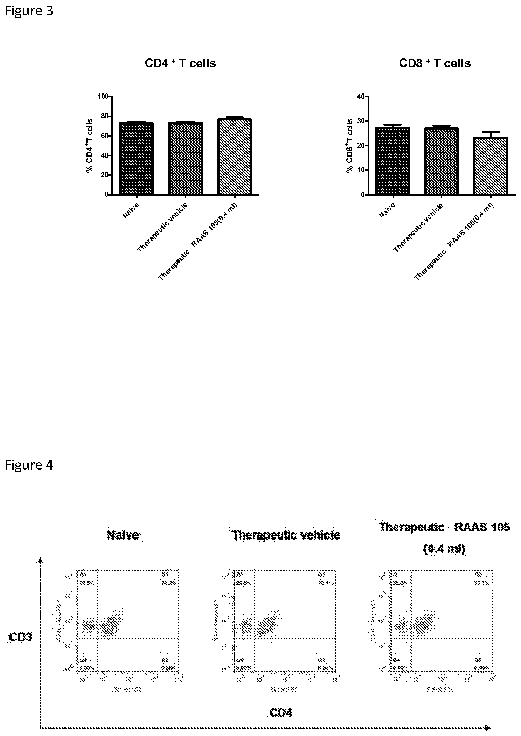

Further analysis of the percentages of CD4⁺ and CD8⁻ (non-CD4⁺) T cell lineages were performed gating on total CD3⁺ T cells. The results showed there were no differences in the percentages of CD4⁺ and CD8⁺T cells among all the groups (Figures 3).The representative FACS profiles from each group were illustrated in Figure 4.

**Figure 3****.** Percentages of CD4 and CD8 T cells in peripheral blood. Total CD3 T cells were gated and further analyzed for CD4/CD8 percentages.

**Figure 4****.** Percentages of CD4 and CD8 T cells in peripheral blood. Total CD3 T cells were gated.

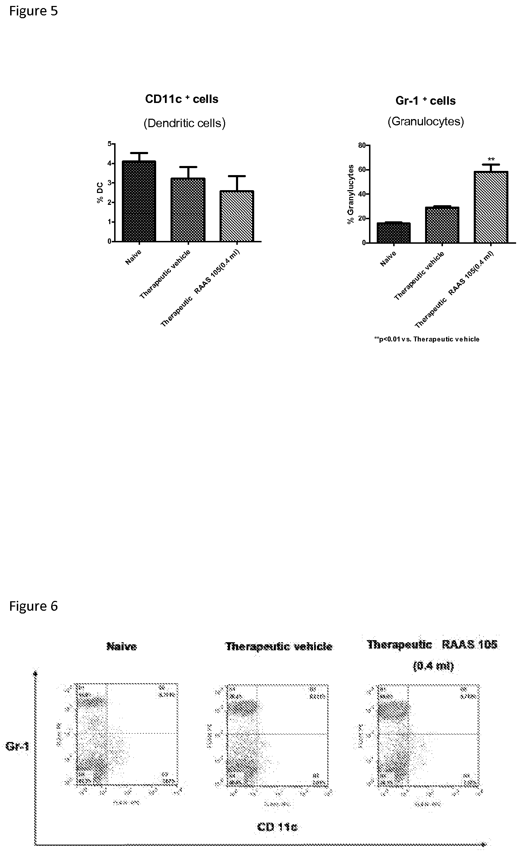

Percentages of total CD11c⁺ dendritic cells (DC)and Gr-1⁺ granulocytes in peripheral blood were investigated. HBV infection reduced the percentages of CD11c⁺DCs, a phenomenon which also be observed in human patients, whereas the percentages of Gr-1⁺ granulocytes were not affected. Therapeutic treatment of RAAS 105 did not show any effect on CD11c⁺DCs, but increased the percentages of Gr-1⁺ granulocytes significantly (Figure 5). The representative FACS profiles from each group were illustrated in Figure 6.

**Figure 5****.** Percents of Dendritic cells and Granulocytes in peripheral blood. Total live cells were gated. After therapeutic treatment, percents of granulocytes increased in peripheral blood (by T test)

**Figure 6****.** Percents of Granulocytes / Dendritic cells in peripheral blood. Total live cells were gated.

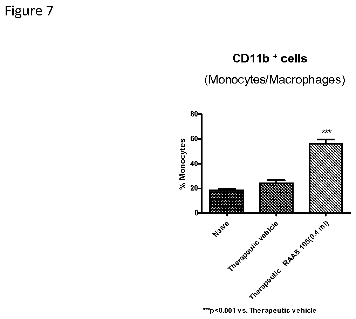

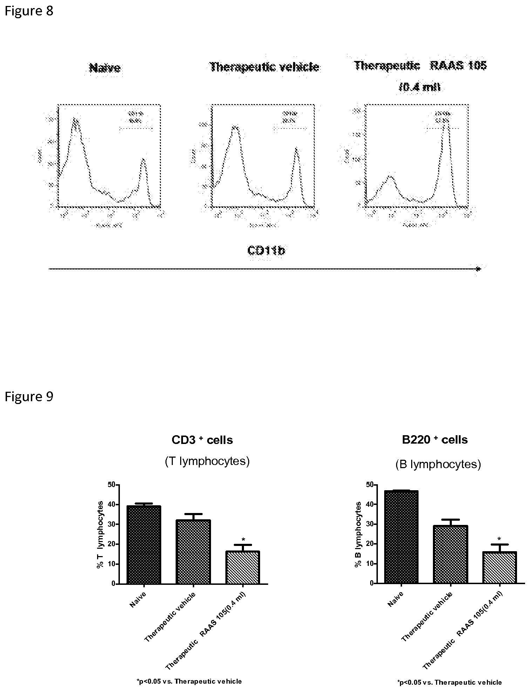

Percentages of Monocytes were examined using surface marker CD11b. It increased significantly as same as Gr1+ granulocytes compared with the vehicle group (Figure 7). The representative FACS profiles from each group were illustrated in Figure 8.

**Figure 7****.** Percentages of Monocytes in peripheral blood. Total live cells were gated. After treatment, percentages of monocytes in peripheral blood significantly increased (t test)

**Figure 8****.** Percentages of monocytes in peripheral blood. Total live cells were gated.

### Cell populations in spleen

Cell lineages in spleen including T cell lineages (CD4⁺/CD8⁺ T cells, naive T cells, memory T cells and regulatory T cells), B cells, mDCs, pDCs, granulocytes and macrophages were characterized by cell surface and intracellular markers.

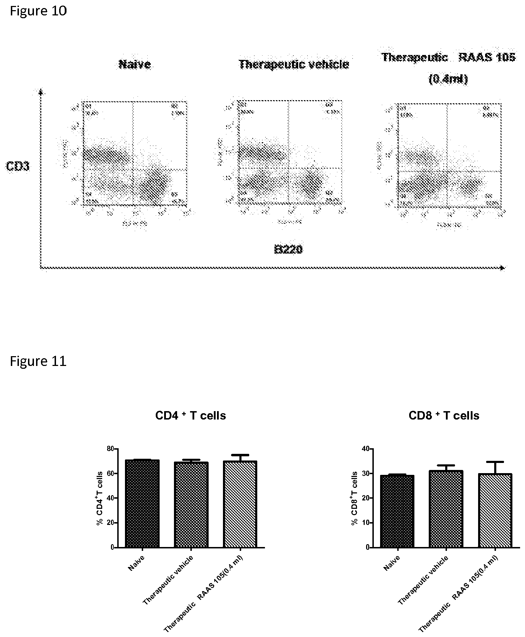

Percentages of total T cells and B cells in spleen were investigated. Therapeutic treatment of RAAS 105 reduced the percentages of both CD3⁺ T cells and CD19⁺B cells significantly (Figure 9). The representative FACS profiles from each group were illustrated in Figure 10.

**Figure 9****.** Percentages of T and B lymphocytes in spleen. Total lymphocytes were gated. After therapeutic treatment by RAAS 105, percents of T cells and B cells significantly decreased in spleen.

**Figure 10****.** Percents of T cells and B cells in spleen. Total lymphocytes were gated.

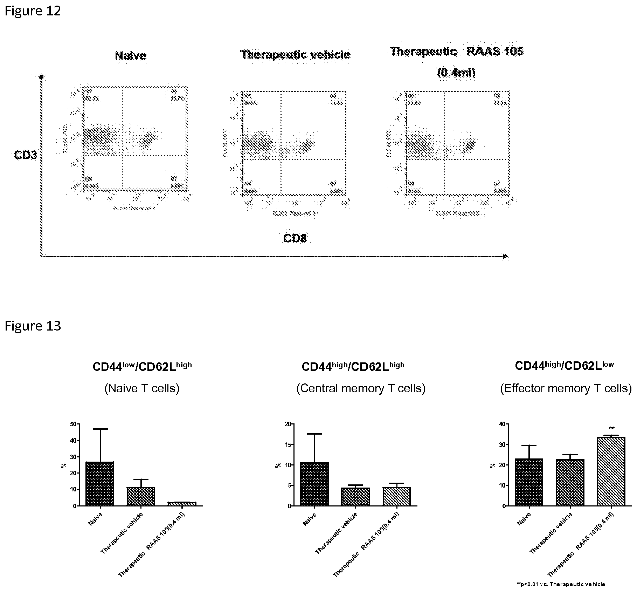

Further analysis of the percentages of CD4⁺(non-CD8⁺) and CD8⁺T cell lineages were performed gating on total CD3⁺ T cells. There were no differences in the percentages of CD4⁺ and CD8⁺T cells among all the groups (Figure 11). The representative FACS profiles from each group were illustrated in Figure 12.

**Figure 11****.** Percentages of CD4 and CD8 T cells in spleen. Total CD3 T cells were gated and further analyzed for CD4 /CD8 percentages.

**Figure 12****.** Percentages of CD4 and CD8 T cells in spleen. Total CD3 T cells were gated.

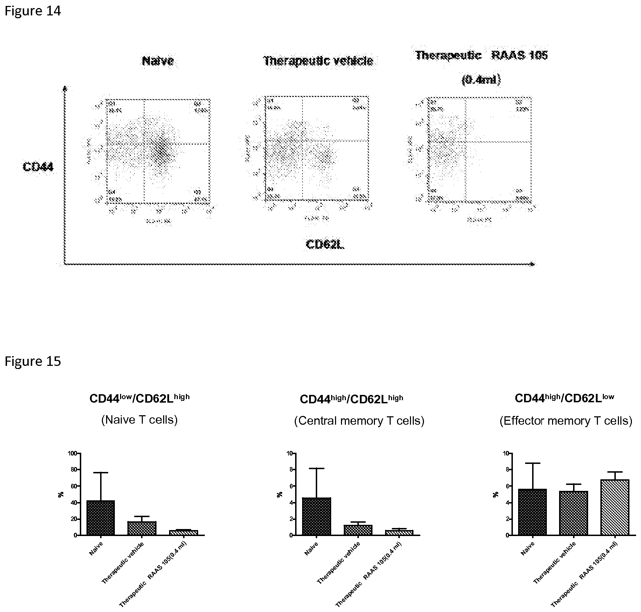

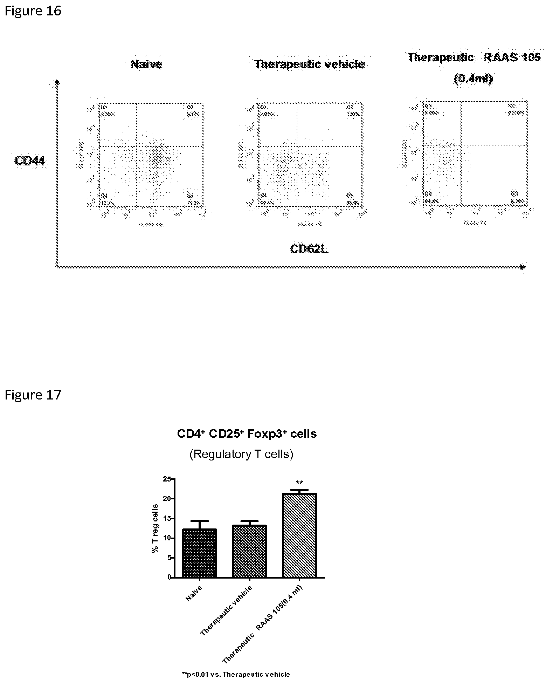

Three T cell lineages, naive T cells (CD44^{low}D62L^{high}), central memory T cells (T_{CM}S, CD44^{high}CD62L^{high}) and Effector memory T cells (T_{EM}S, CD44^{high}CD62L^{low}), were characterized by surface markers CD44 and CD62L. Percentages of these T cell lineages in CD4⁺ or CD8⁺ T cells were analyzed respectively. Both in CD4⁺ and CD8⁺ T cells, percentages of naive T cells and T_{CM}Sdecreased and T_{EM}S increased after the therapeutic treatment of RAAS 105, suggesting the compound may have effect to promote the transformation of T cells from naive T cells to memory T cells in spleen (Figure 13 and 15). The representative FACS profiles from each group were illustrated in Figure 14 and 16.

**Figure 13****.** T cell subsets percentages in spleen. Total CD4 T cells were gated and T cell subsets were determined.

**Figure 14****.** CD4 T cell subsets percentages in spleen. Total CD4 T cells were gated and T cell subsets were determined.

**Figure 15****.** T cell subsets percentages in spleen. Total CD8 T cells were gated and T cell subsets were determined.

**Figure 16****.** CD8 T cell subsets percentages in spleen. Total CD8 T cells were gated and T cell subsets were determined.

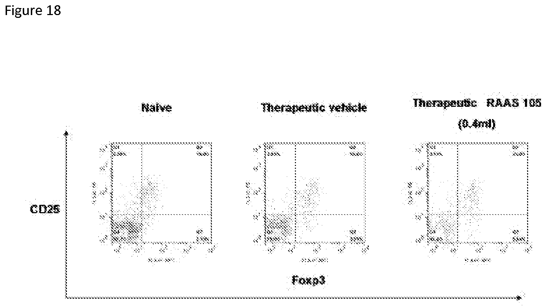

Regulatory T cells (Tregs) were analyzed by cell surface staining of anti-CD4 and anti-CD25 antibodies followed by intracellular staining of anti-Foxp3 antibody. Percents of Tregs in spleen increased compared with the vehicle group (Figure 17). The representative FACS profiles from each group were illustrated in Figure 18.

**Figure 17****.** Percentages of Foxp3 regulatory T cells in spleen. Foxp3 regulatory T cells were analyzed by intracellular staining. After treatment, the percentage of T regulate cells is increased.

**Figure 18****.** Percentages of regulatory T cells in spleen. Total CD4 T cells were gated.

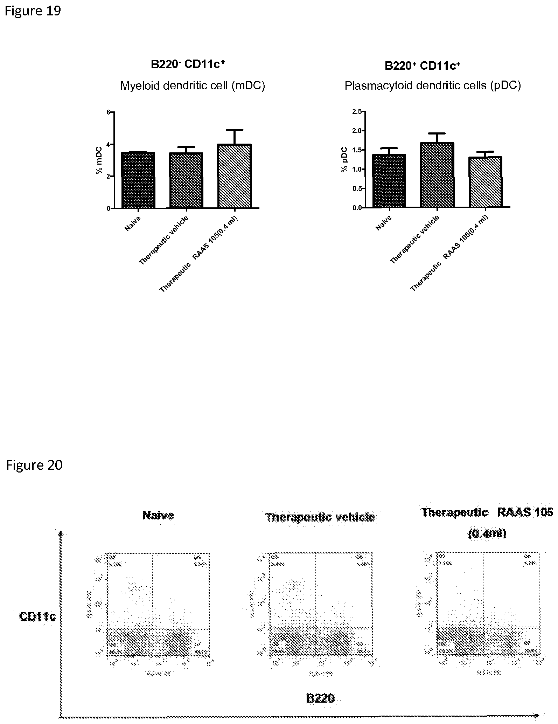

Dendritic cells, including myeloid dendritic cells (mDC, B220⁻CD11c⁺) and plasmacytoid dendritic cells (pDC, B220⁺CD11c⁺) in spleen were analyzed. No significant differences of mDCs and pDCs were observed among all groups (Figure 19). The representative FACS profiles from each group were illustrated in Figure 20.

**Figure 19****.** Percentages of pDcs and mDcs in spleen. Total live cells were gated. There were no significant differences after compound treatment, (by t test)

**Figure 20****.** Percentages of mDc and pDcs in spleen. Total live cells were gated.

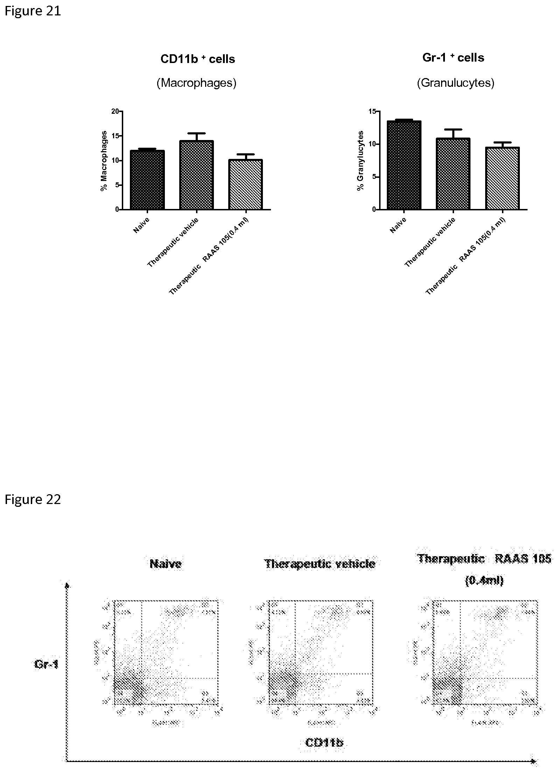

CD11b⁺ macrophages and Gr-1⁺ granulocytes in spleen were analyzed. There were no significant alterations among all groups in the percentages of these cell lineages in spleen, as shown in Figure 21. The representative FACS profiles from each group were illustrated in Figure 22.

**Figure 21****.** Percentages of Macrophages and Granulocytes in Spleen. Total live cells were gated. There were no significant differences after compound treatment, (by t test)

**Figure 22****.** Percentages of macrophages/Granulocytes in spleen. Total live cells were gated.

### Cell populations in draining lymph nodes

Cell lineages in draining lymph nodes including T cell lineages (CD4⁺/CD8⁺ T cells, naive T cells, memory T cells and regulatory T cells), DCs, granulocytes and macrophages were characterized by cell surface and intracellular markers.

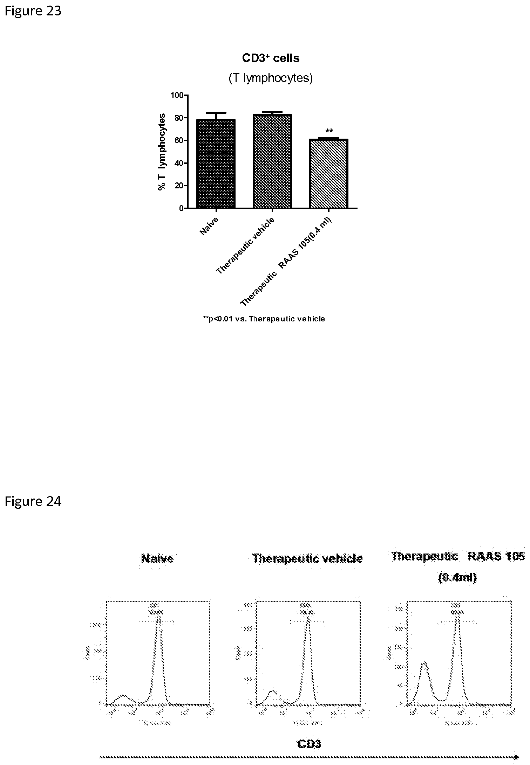

Percentages of total T cells in lymph nodes were analyzed. HBV infection did not affect the percentages of CD3⁺ T cells but therapeutic treatment of RAAS 105 reduced it significantly compared with vehicle group (Figure 23). The representative FACS profiles from each group were illustrated in Figure 24.

**Figure 23****.** Percentages of T cells in lymph nodes. Total lymphocytes were gated. After the treatment, the percentage of T cells in the lymph nodes were significantly decreased (t test)

**Figure 24****.** Percentages of CD3 T cells in lymph nodes. Total lymphocytes were gated.

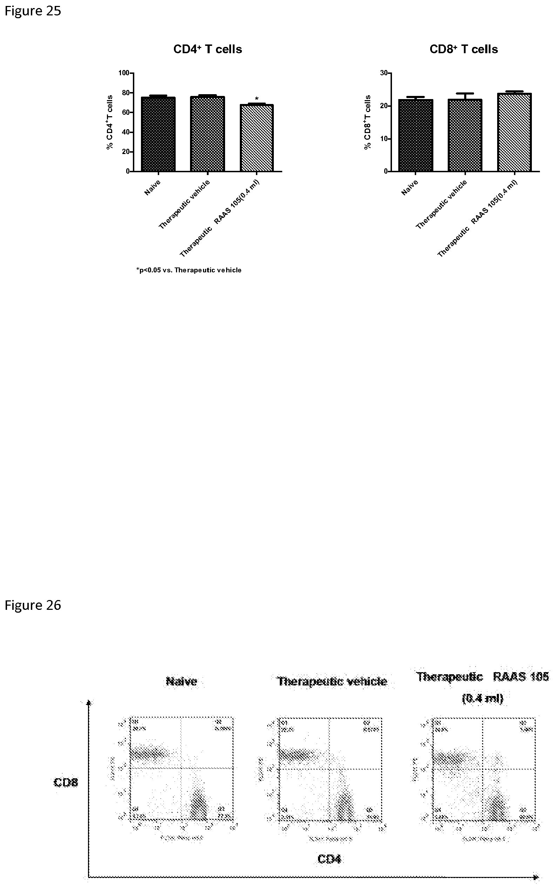

Further analysis of the percentages of CD4⁺ and CD8⁻T cell lineages were performed gating on total CD3⁺ T cells. Percentages of CD4⁺ T cells tended to decrease while CD8⁺ T cells tended to increase, suggesting that therapeutic treatment of RAAS 105 may have effect on the ratio of CD4⁺/CD8⁺ T cells in lymph nodes (Figure 25).The representative FACS profiles from each group were illustrated in Figure 26.

**Figure 25****.** Percentages of CD4 and CD8 T cells in lymph nodes. Total CD3 T cells were gated and further analyzed for CD4/CD8 percentages. After therapeutic treatment, the percentage of CD4 T cells decreased, (by t test)

**Figure 26****.** Percentages of CD4 and CD8 T cells in lymph nodes. Total CD3 T cells were gated and further analyzed for CD4/CD8 percentages.

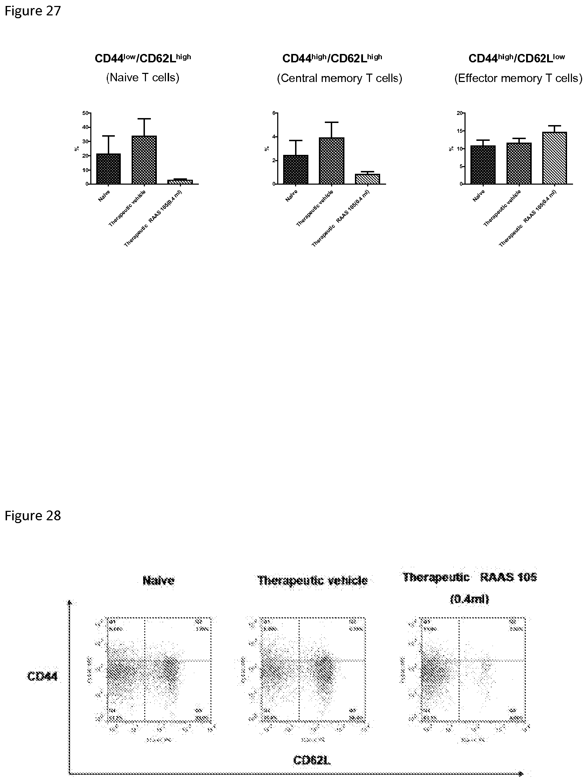

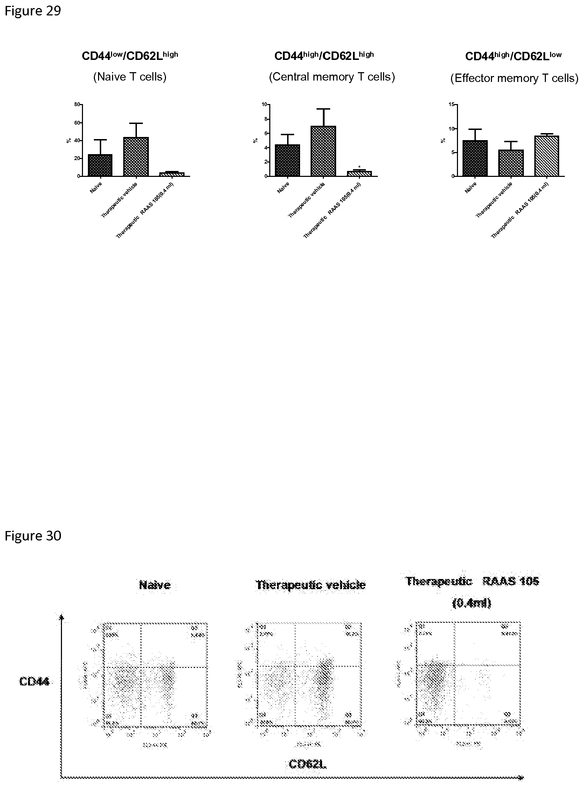

Three T cell lineages, naive T cells, T_{CM}S and T_{EM}S were characterized by surface markers CD44 and CD62L. Percentages of these T cell lineages in CD4⁺ or CD8⁺ T cells were analyzed respectively. The results in lymph nodes were comparable to those in spleen. Both in CD4⁺ and CD8⁺ T cells, percentages of naive T cells and T_{CM}S decreased and T_{EM}S increased after the therapeutic treatment of RAAS 105, suggesting the compound also have effect to promote the transformation of T cells from naive T cells to memory T cells in lymph nodes (Figure 27 and 29). The representative FACS profiles from each group were illustrated in Figure 28 and 30.

**Figure 27****.** CD4 T cell subsets percentages in lymph nodes. Total CD4 T cells were gated and T cell subsets were determined. No significant differences were found in all the groups compared to vehicle group.

**Figure 28****.** CD4 T cell subset percentages in lymph nodes. Total CD4 T cells were gated and T cell subsets were determined.

**Figure 29****.** CD8 T cell subsets percentages in lymph nodes. Total CD8 T cells were gated and T cell subsets were determined.

**Figure 30****.** CD8 T cell subsets percentages in lymph nodes. Total CD8 T cells were gated and T cell subsets were determined.

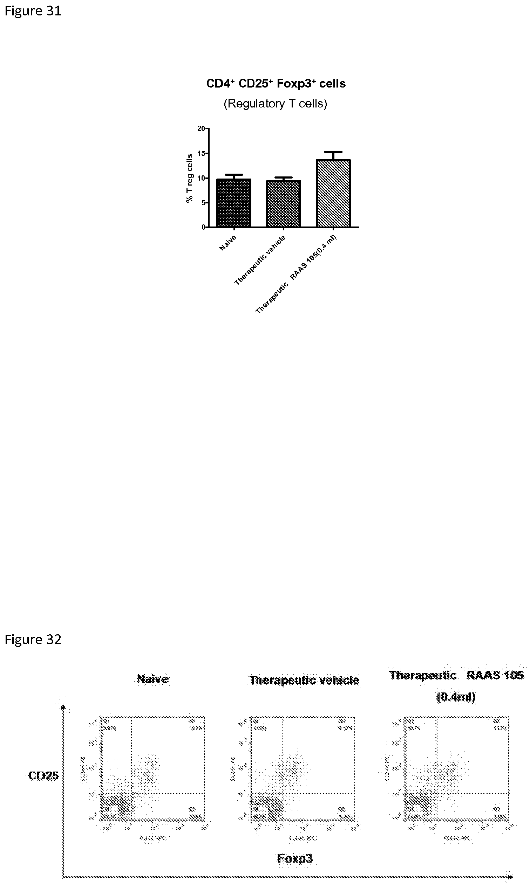

Regulatory T cells (Tregs) were analyzed. Percentages of Tregs in lymph node slightly increased without significant differences (Figure 31). The representative FACS profiles from each group were illustrated in Figure 32.

**Figure 31****.** Percentages of Foxp3 regulatory T cells in lymph nodes. There were no significant alterations after compound treatment

**Figure 32****.** Percentages of regulatory T cells in lymph nodes. Total CD4 T cells were gated. One representative profile from each group is shown.

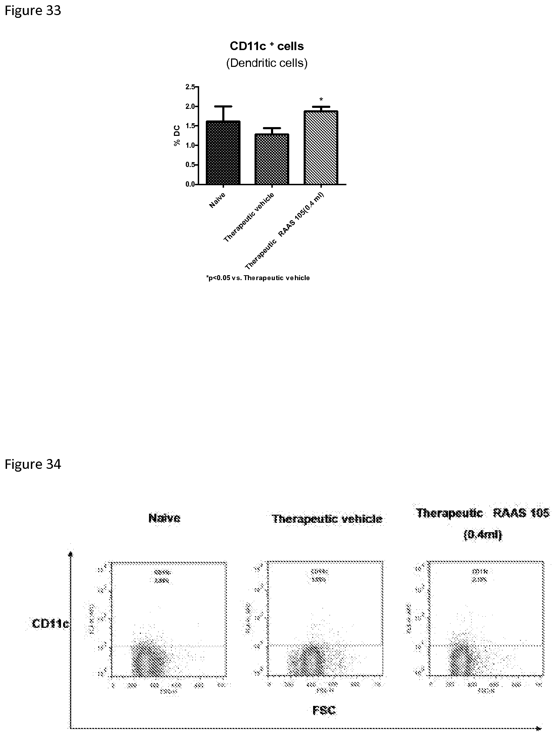

Total dendritic cells in lymph nodes were analyzed. Therapeutic treatment of RAAS 105 may reverse the reduction of DCs induced by HBV infection (Figure 33). The representative FACS profiles from each group were illustrated in Figure 34.

**Figure 33****.** Percentages of DCs in lymph nodes. Total live cells were gated. After treatment, percents of DCs increased significantly (by t test)

**Figure 34****.** Percentages of DCs in lymph nodes. Total live cells were gated.

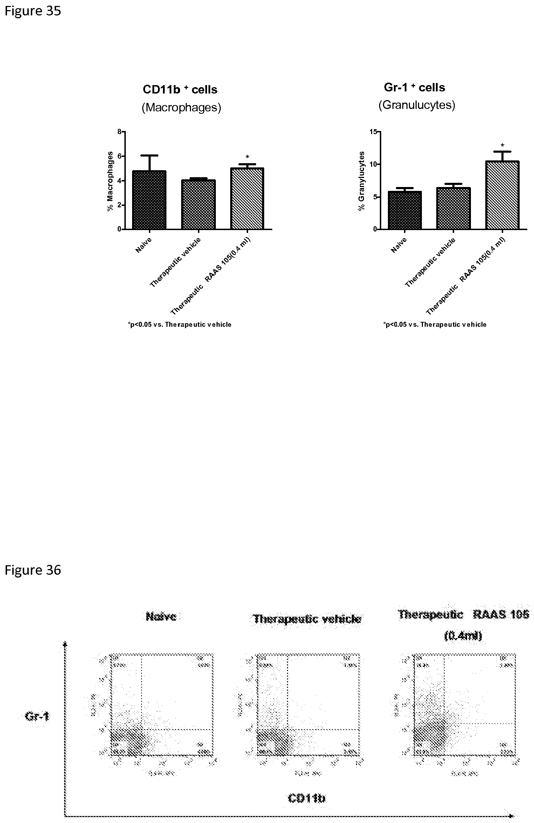

CD11b⁺ macrophages and Gr-1⁺ granulocytes in lymph nodes were analyzed. Both percentages of CD11b⁺ macrophages and Gr-1⁺ granulocytes increased significantly (Figure 35). The representative FACS profiles from each group were illustrated in Figure 36.

**Figure 35****.** Percentages of Macrophages and Granulocytes in lymph nodes. Total live cells were gated. Percentages of macrophages and granulocytes significantly increased in lymph node. (by t test)

**Figure 36****.** Percentages of Macrophages/Granulocytes in lymph nides. Total live cells were gated.

### Effect of prophylactic treatment with RAAS 105

### Mice information

Total 14 female BALB/c mice including 2 naive mice at the same age were transferred from Infectious Disease (ID) Group of Wuxi Apptec. The group and the regimen information were shown by Table 2.

**Table 2. The experimental group and dosing regimen of the 2^{nd} part of the study**

| **Groups** | **N** | **Group ID** | **Dose** | **1^{st} or last dosing** | **Analysis** |
|---|---|---|---|---|---|
| 5 | 4 | Prophylactic vehicle^{#} | -- | last, 4 hrs pre-HDI | Day 5 |
| 7 | 4 | Prophylactic RAAS 105 | 0.4 ml/mouse | last, 4 hrs pre-HDI | Day 5 |
| 10 | 4 | ETV | 0.1 mg/kg | 1^{st}, 4 hrs pre-HDI | Day 5 |
| 11 | 2 | Naive | - | - | - |

### Cell populations in peripheral blood

After removing red blood cells, T cell lineages, B cells, DCs, granulocytes, and monocytes/macrophages in peripheral blood were analyzed by FACS analysis.

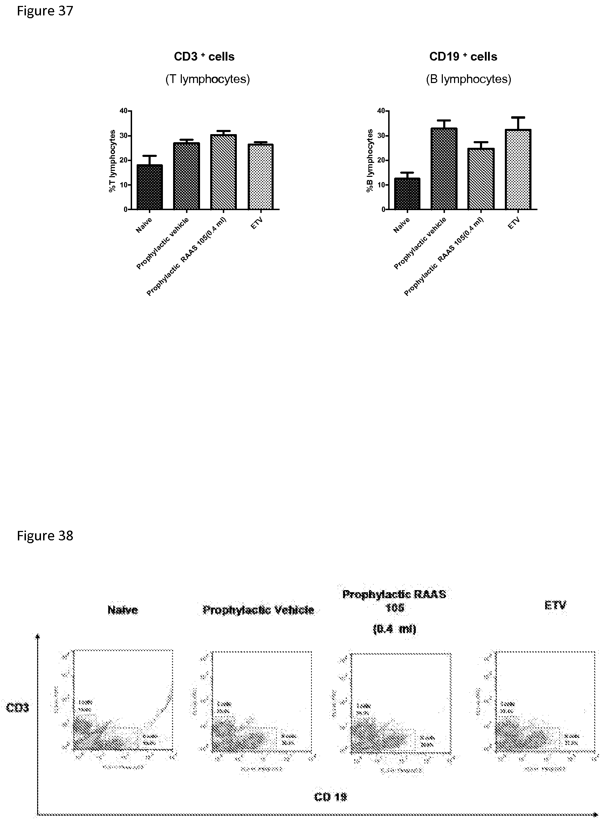

Total T cells and B cells were characterized. Unlike therapeutic treatment, prophylactic treatment of RAAS 105 had no effect on percentages of CD3⁺ T cells but reduced the percentages of CD19⁺B cells although the statistical significance was not found (Figure 37). The representative FACS profiles from each group were illustrated in Figure 38.

**Figure 37****.** Percents of T and B lymphocytes in peripheral blood. Total lymphocytes were gated.

**Figure 38****.** Percents of T cells and B cells in peripheral blood. Total lymphocytes were gated.

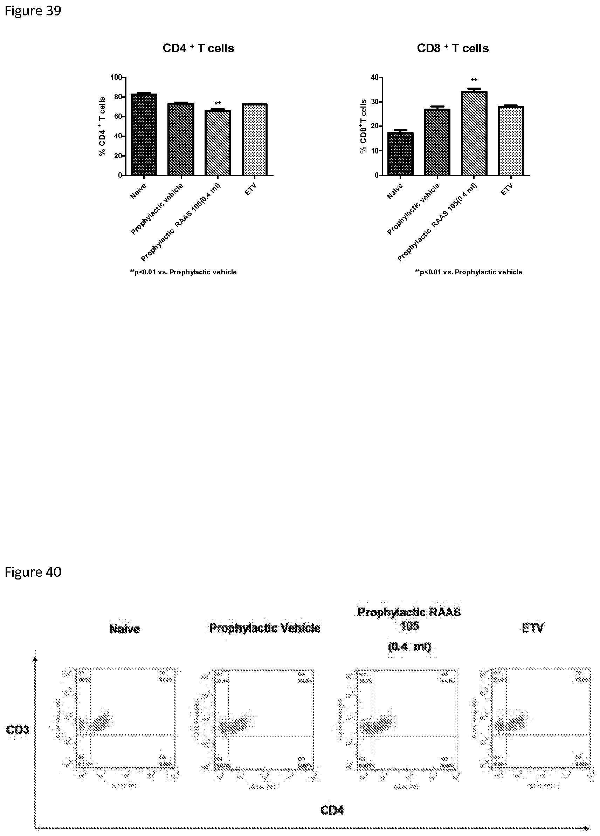

Further analysis of the percentages of CD4⁺ and CD8⁻(non-CD4⁺) T cell lineages were performed gating on total CD3⁺ T cells. Unlike therapeutic treatment, prophylactic treatment reduced percentages of CD4⁺ T cells and increased percentage of CD8⁺ T cells, suggesting the potential effect of RAAS 105 to reduce the ratio of CD4⁺/CD8⁺ T cells in peripheral blood (Figure 39). The representative FACS profiles from each group were illustrated in Figure 40.

**Figure 39****.** Percentages of CD4 and CD8 T cells in peripheral blood. Total CD3 T cells were gated and further analyzed for CD4/CD8 percentages. After prophylactic treated by RAAS 105, percentages of CD4 T cells decreased while CD8 T cells increased (by t test)

**Figure 40****.** Percentages of CD4 and CD8 T cells in peripheral blood. Total CD3 T cells were gated.

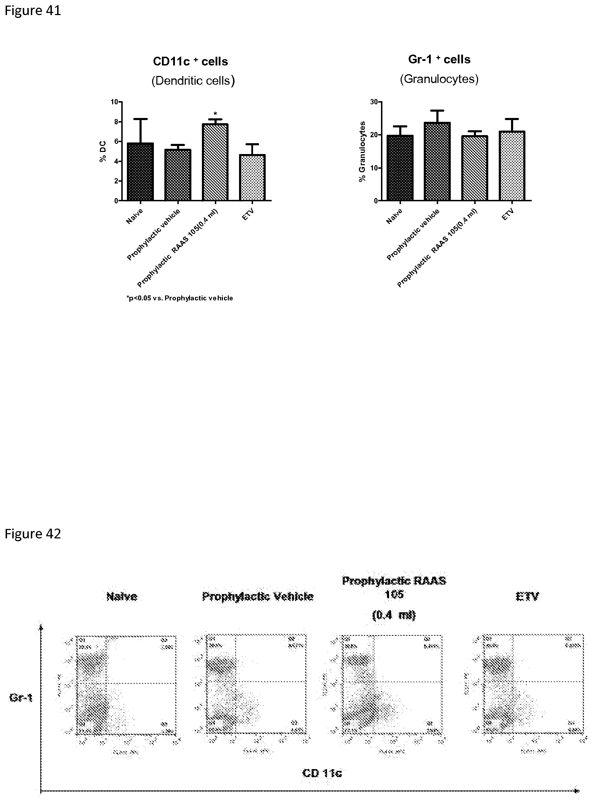

Results of total CD11c⁺ dendritic cells (DC)and Gr-1⁺ granulocytes in peripheral blood were also different from those in therapeutic treatment. Prophylactic treatment of RAAS 105 reversed the reduction of DCs induced by HBV infection, but had no significant effect on granulocytes in peripheral blood (Figure 41). The representative FACS profiles from each group were illustrated in Figure 42.

**Figure 41****.** Percentages of Dendritic cells and Granulocytes in peripheral blood. Total live cells were gated. After prophylactic treated, percents of dendritic cells increased in [eripheral blood.

**Figure 42****.** Percentages of Granulocytes/Dendritic cells in peripheral blood. Total live cells were gated.

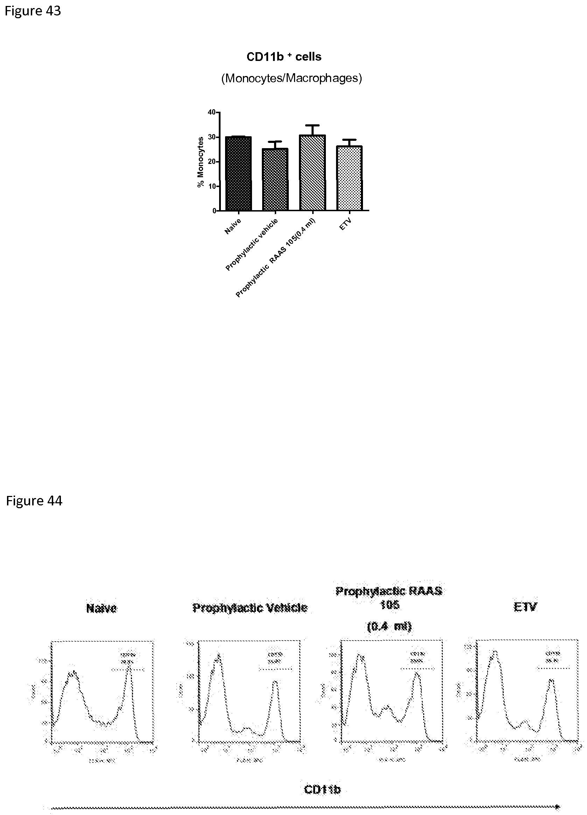

Percentages of Monocytes were examined. There were no significant differences among all groups (Figure 43). The representative FACS profiles from each group were illustrated in Figure 44.

**Figure 43****.** Percentages of Monocytes in peripheral blood. Total live cells were gated.

**Figure 44****.** Percentages of monocytes in peripheral blood. Total live cells were gated.

### Cell populations in spleen

Cell lineages in spleenincluding T cell lineages (CD4⁺/CD8⁺ T cells,naive T cells, memory T cells and regulatory T cells), B cells, mDCs, pDCs, granulocytes and macrophages were characterized by cell surface and intracellular markers.

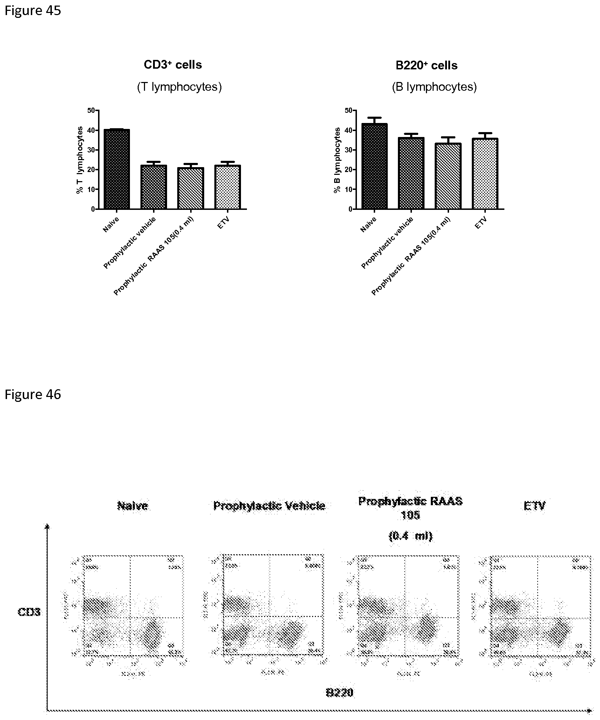

Percentages of total T cells and B cells in spleen were investigated. Unlike therapeutic treatment, prophylactic treatment did not show effects on percentages of CD3⁺ T cells and CD19⁺B cells (Figure 45). The representative FACS profiles from each group were illustrated in Figure 46.

**Figure 45****.** Percentages of T and B 1tmphocytes in spleen. Total lymphocytes were gated.

**Figure 46****.** Percentages of T and B cells in spleen. Total lymphocytes were gated.

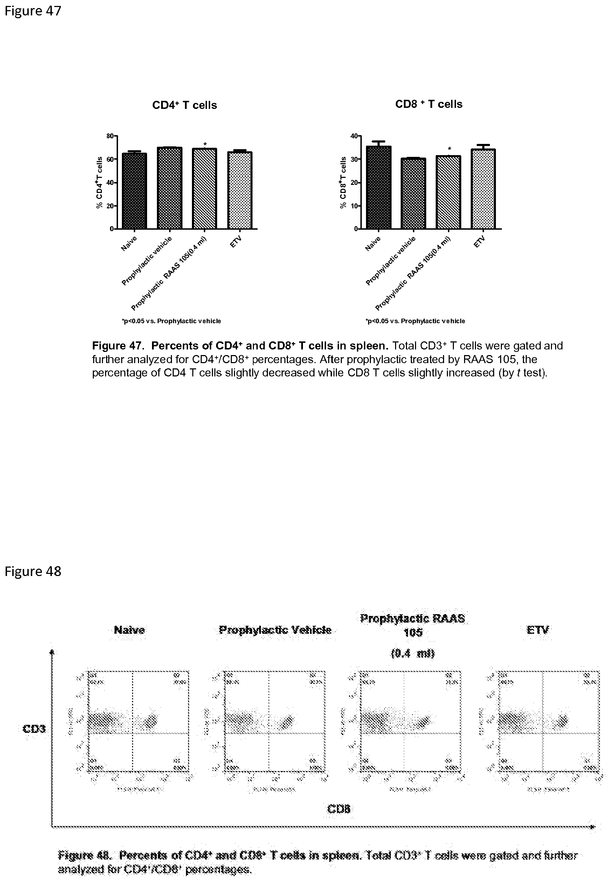

Further analysis of the percentages of CD4⁺(non-CD8⁺) and CD8⁺T cell lineages were performed gating on total CD3⁺ T cells. Percentages of CD4⁺ T cells slightly decreased and CD8⁺T cells slightly increased in spleen (Figure 47).The representative FACS profiles from each group were illustrated in Figure 48.

**Figure 47****.** Percentages of CD4 and CD8 T cells in spleen. Total CD3 T cells were gated and further analyzed for CD4/CD8 percentages. After prophylactic treated by RAAS 105, the percentage of CD4 T cells slightly decreased while CD8 T cells slightly increased (by t test)

**Figure 48****.** Percentages of CD4 and CD8 T cells in spleen. Total CD3 T cells were gated and further analyzed for CD4/CD8 percentages.

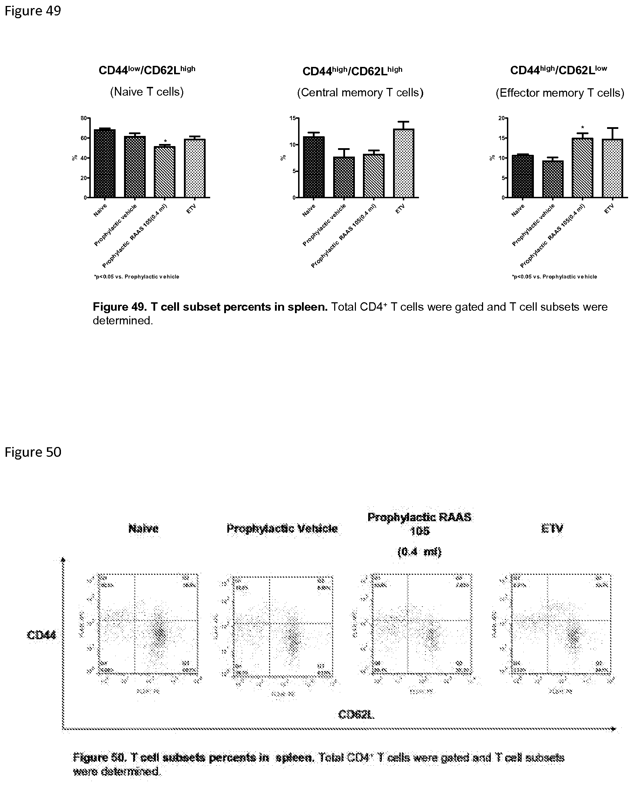

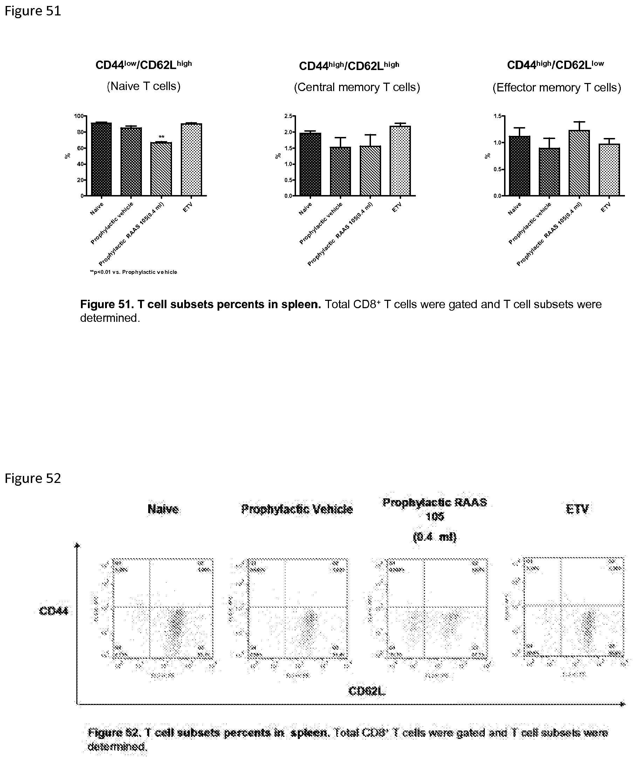

Naive T cells, central memory T cells and Effector memory T cells were investigated. Percentages of these T cell lineages in CD4⁺ or CD8⁺ T cells in spleen were analyzed respectively. Both in CD4⁺ and CD8⁺ T cells, percentages of naive T cells decreased and T_{EM}S increased significantly after the prophylactic treatment of RAAS 105 (Figure 49 and 51). The representative FACS profiles from each group were illustrated in Figure 50 and 52.

**Figure 49****.** T cell subset percentages in spleen. Total CD4 T cells were gated and T cell subsets were determined.

**Figure 50****.** T Cell subsets percentages in spleen. Total CD4 T cells were gated and T cell subsets were determined.

**Figure 51****.** T cell subsets percentages in spleen. Total CD8 T cells were gated and T cell subsets were determined.

**Figure 52****.** T cell subsets percentages in spleen. Total CD8 T cells were gated and T cell subsets were determined.

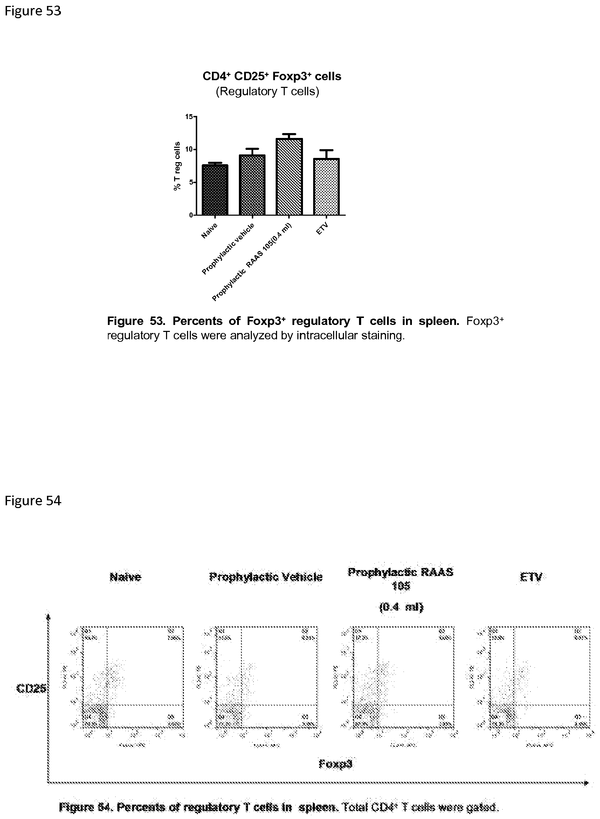

Results of regulatory T cells (Tregs) were comparable with those in therapeutic treatment. Percentages of Tregs in spleen increased compared with the vehicle group by prophylactic treatment of RAAS 105 (Figure 53). The representative FACS profiles from each group were illustrated in Figure 54.

**Figure 53****.** Percentages of Foxp3 regulatory T cells in spleen. Foxp3 regulatory T cells were analyzed by intracellular staining.

**Figure 54****.** Percentages of regulatory T cells in spleen. Total CD4 T cells were gated.

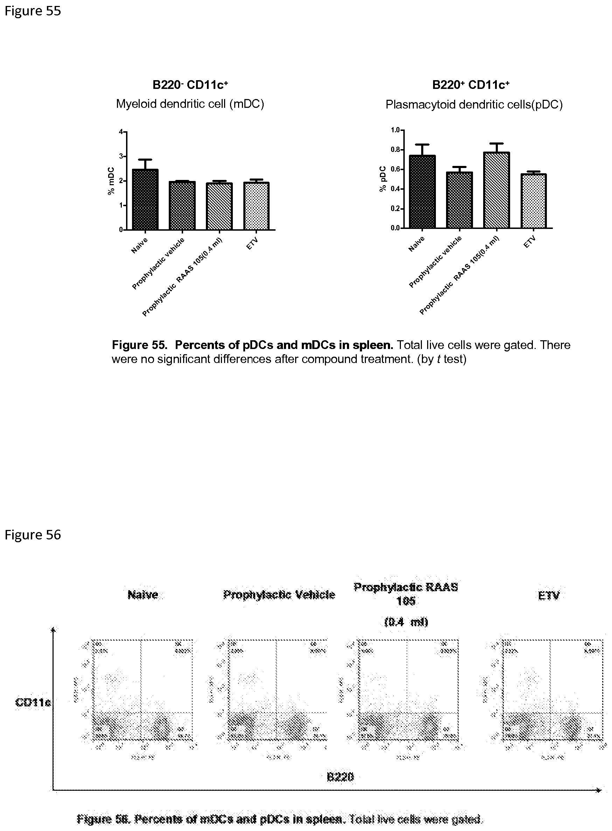

Dendritic cells, including mDCsand pDCs in spleen were analyzed. No significant differences of mDCs and pDCs were observed among all groups after prophylactic treatment (Figure 55). The representative FACS profiles from each group were illustrated in Figure 56.

**Figure 55****.** Percentages of pDCs and mDC in spleen. Total live cells were gated. There were no significant differences after compound tratment (by t test)

**Figure 56****.** Percentages of mDCs and pDCs in spleen. Total live cells were gated.

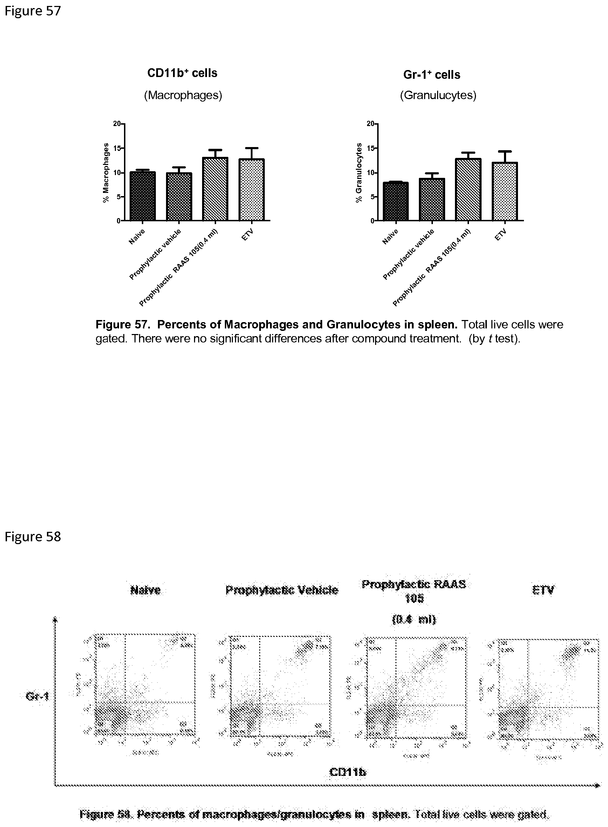

CD11b⁺ macrophages and Gr-1⁺ granulocytes in spleen were analyzed. Percentages of macrophages and granulocytes increased, but no statistical differences were observed, as shown in Figure 57. The representative FACS profiles from each group were illustrated in Figure 58.

**Figure 57****.** Percentages of Macrophages and Granulocytes in spleen. Total live cells were gated. There were no significant differences after compound treatment, (by t test)

**Figure 58****.** Percentages of macrophages/granulocytes in spleen. Total live cells were gated.

### Cell populations in draining lymph nodes

Cell lineages in draining lymph nodesincluding T cell lineages (CD4⁺/CD8⁺ T cells, naive T cells, memory T cells and regulatory T cells), DCs, granulocytes and macrophages were characterized by cell surface and intracellular markers.

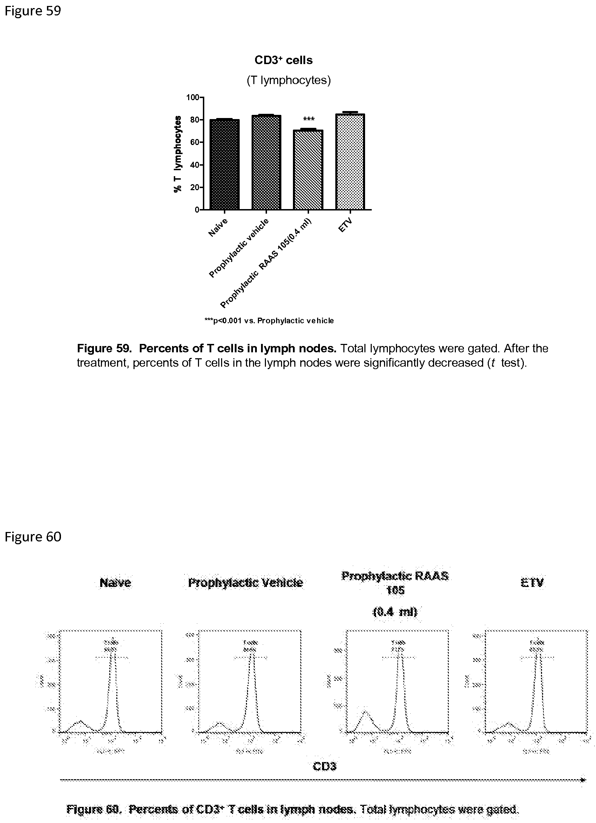

Percentages of total T cells in lymph nodes were analyzed. Similar with therapeutic treatment, HBV infection did not affect the percentages of CD3⁺ T cells but prophylactic treatment of RAAS 105 reduced it significantly compared with vehicle group (Figure 59). The representative FACS profiles from each group were illustrated in Figure 60.

**Figure 59****.** Percentages of T cells in lymph nodes. Total lymphocytes were gated. After the treatment, percents of T cells in the lymph nodes were signicantly decreased. (t test)

**Figure 60****.** Percentages of CD3 T cells in lymph nodes. Total lymphocytes were gated.

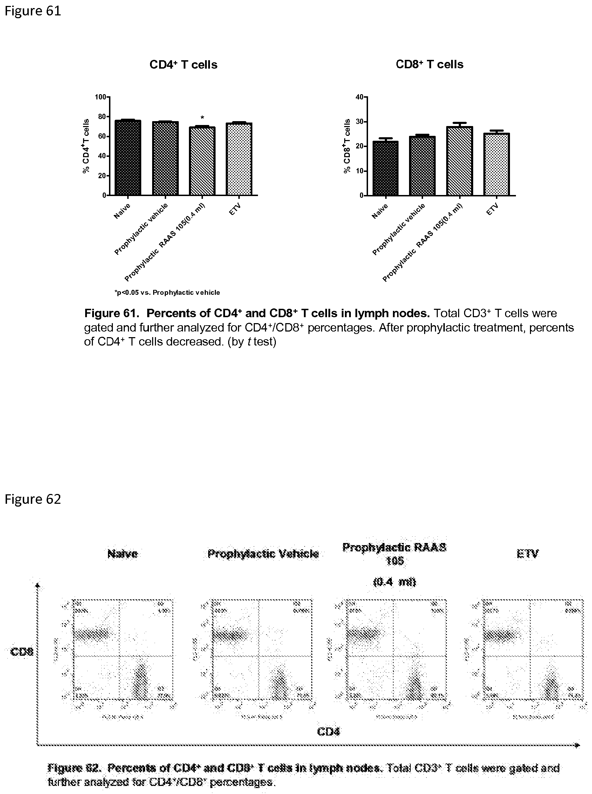

Further analysis of the percentages of CD4⁺ and CD8⁻T cell lineages were performed gating on total CD3⁺ T cells. Percentages of CD4⁺ T cells tended to decrease while CD8⁺ T cells tended to increase after prophylactic treatment, as was seen in therapeutic treatment (Figure 61).The representative FACS profiles from each group were illustrated in Figure 62.

**Figure 61****.** Percentages of CD4 and CD8 T cells in lymph nodes. Total CD3 T cells were gated and further analyzed for CD4/CD8 percentages. After prophylactic treatment, percents of CD4 T cells decreased (by t test)

**Figure 62****.** Percentages of CD4 and CD8 T cells in lymph nodes. Total CD3 T cells were gated and further analyzed for CD4/CD8 percentages.

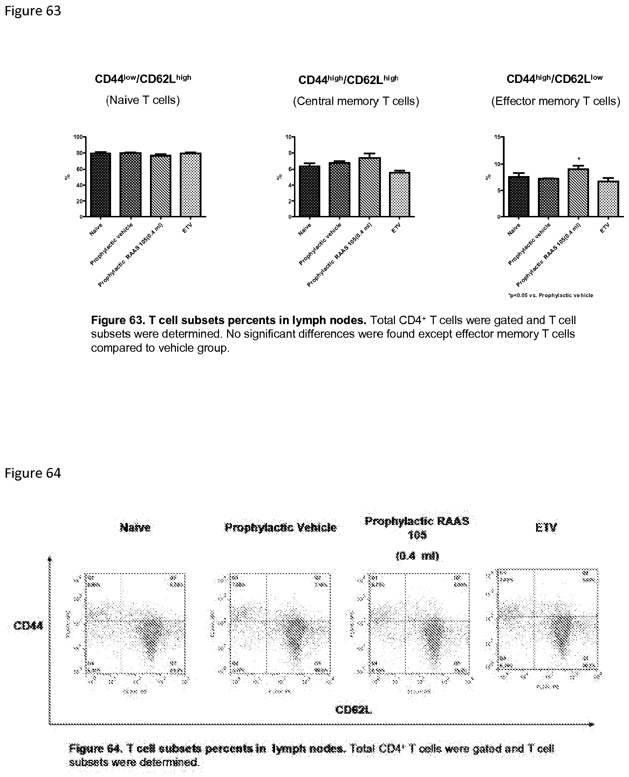

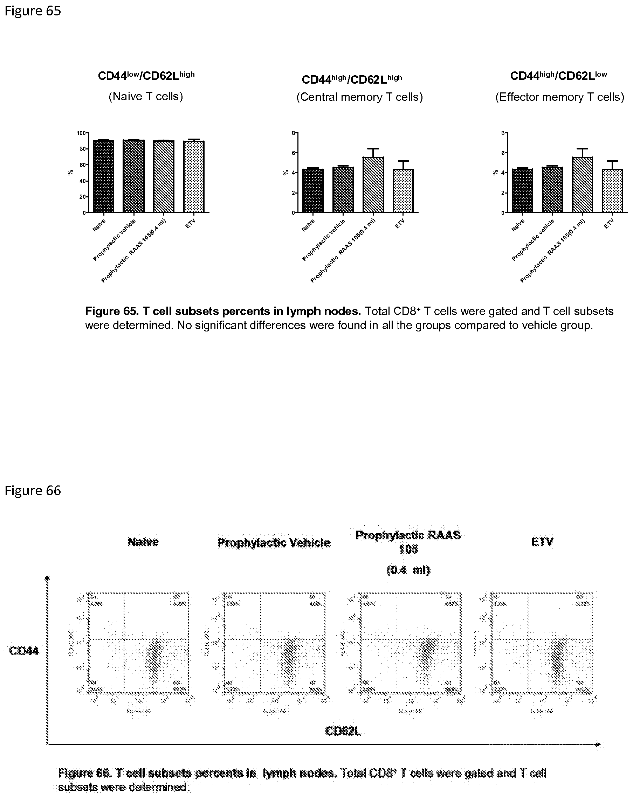

Results of naive T cells, central memory T cells and Effector memory T cells were totally difference with those in therapeutic treatment. Prophylactic treatment did not show significant effects on naive T cells and T_{CM}S, but increased percentages of T_{EM}S (Figure 63 and 65). The representative FACS profiles from each group were illustrated in Figure 64 and 66.

**Figure 63****.** T cell subsets percentages in lymph nodes. Total CD4 cells were gated and T cell subsets were determined. No significant differences were found except effector memory T cells compared to vehicle group.

**Figure 64****.** T cell subsets percentages in lymph nodes. Total CD4 T cells were gated and T cell subsets were determined.

**Figure 65****.** T cell subsets percentages in lymph nodes. Total CD8 T cells were gated and T cell subsets were determined. No significant differences were found in all the groups compared to vehicle group.

**Figure 66****.** T cell subsets percentages in lymph nodes. Total CD8 T cells were gated and T cell subsets were determined.

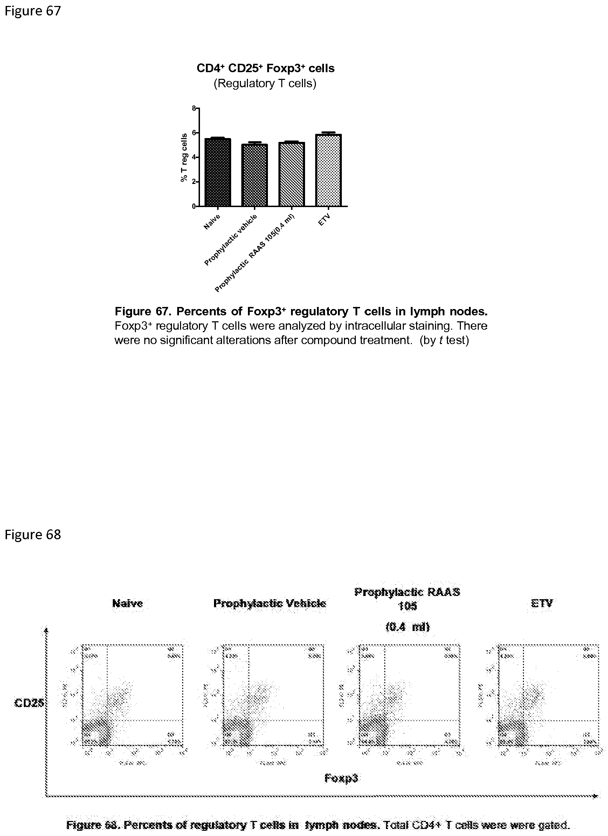

Regulatory T cells were analyzed. There were no significant differences among all groups (Figure 67). The representative FACS profiles from each group were illustrated in Figure 68.

**Figure 67****.** Percentages of Foxp3 regulatory T cells in lymph nodes. Foxp3 regulatory T cells were analyzed by intracellular staining. There were no significant alterations after compound treatment, (by t test)

**Figure 68****.** Percentages of regulatory T cells in lymph nodes. Total CD4 T cells were gated.

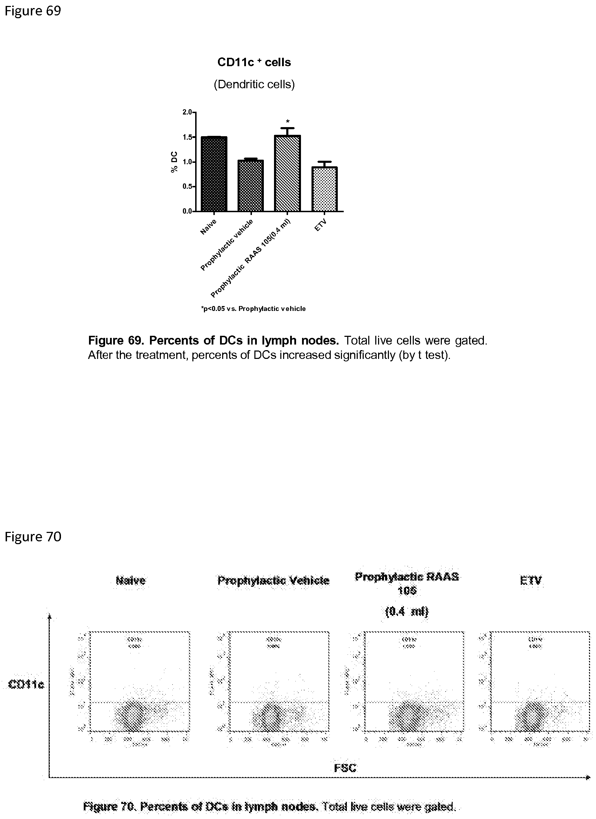

Results of total dendritic cells in lymph nodes were similar with those in therapeutic treatment. Prophylactic treatment of RAAS 105 also increased the percentages of DCs significantly compared with vehicle group (Figure 69). The representative FACS profiles from each group were illustrated in Figure 70.

**Figure 69****.** Percentages of DCs in lymph nodes. Total live cells were gated. After the treatment, percentages of the DCs increased significantly (by t test)

**Figure 70****.** Percentages of DCs in lymph nodes. Total live cells were gated.

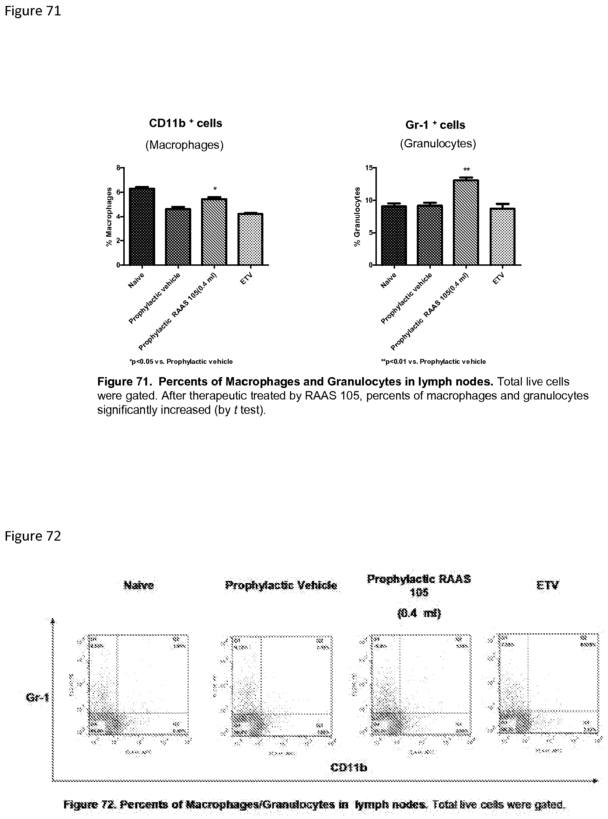

CD11b⁺ macrophages and Gr-1⁺ granulocytes in lymph nodes were analyzed. Both macrophages and granulocytes increased significantly (Figure 71). The representative FACS profiles from each group were illustrated in Figure 72.

**Figure 71****.** Percentages of Macrophages and Granulocytes in lymph nodes. Total live cells were gated. After therapeutic treated by RAAS 105, percents of macrophages and granulocytes significantly increased. (by t test)

**Figure 72****.** Percentages of Macrophages/Granulocytes in lymph nodes. Total live cells were gated.

### Conclusions

The effects of RAAS 105 on different cell lineages in lymphoid tissues and peripheral blood in HBV infected mice were investigated by FACS analysis. T cell lineages (including CD4⁺/CD8⁺ T cells,naïve T cells, memory T cells and regulatory T cells), B cells, dendritic cells (including mDCs, pDCs), granulocytes and monocytes/macrophages were analyzed. RAAS 105 was administered in two different time schedules for therapeutic and prophylactic treatment.

Therapeutic treatment revealed some interesting findings. The animals treated with RAAS 105 exhibited alterations in multiple immune cells and various lineages compared with that in the vehicle group, including reduction of lymphocytes and increase of granulocytes and monocytes. Prophylactic treatment led to less dramatic alterations in the immune cells.

## Claims

1. An isolated purified intravenous immunoglobin (IVIG) plasma product comprising an isolated purified protein complex containing proteins defined by amino acid sequences SEQ ID NOs 33-37.

2. The isolated purified protein complex of claim 1 further comprising proteins defined by amino acid sequences SEQ ID NOs 22-31.

3. The isolated purified protein complex of claim 1 further comprising a protein defined by amino acid sequence SEQ ID NO: 51.

4. An isolated purified IVIG plasma product comprising an isolated purified protein complex containing:
a first substantially pure protein having at least 90% homology to amino acid sequence SEQ ID NO: 22;
a second substantially pure protein having at least 90% homology to amino acid sequence SEQ ID NO: 23;
a third substantially pure protein having at least 90% homology to amino acid sequence SEQ ID NO: 24;
a fourth substantially pure protein having at least 90% homology to amino acid sequence SEQ ID NO: 25;
a fifth substantially pure protein having at least 90% homology to amino acid sequence SEQ ID NO: 26;
a sixth substantially pure protein having at least 90% homology to amino acid sequence SEQ ID NO: 27;
a seventh substantially pure protein having at least 90% homology to amino acid sequence SEQ ID NO: 28;
an eighth substantially pure protein having at least 90% homology to amino acid sequence SEQ ID NO: 29;
a ninth substantially pure protein having at least 90% homology to amino acid sequence SEQ ID NO: 30;
a tenth substantially pure protein having at least 90% homology to amino acid sequence SEQ ID NO: 31;
an eleventh substantially pure protein having at least 90% homology to amino acid sequence SEQ ID NO: 32;
a twelfth substantially pure protein having at least 90% homology to amino acid sequence SEQ ID NO: 33;
a thirteenth substantially pure protein having at least 90% homology to amino acid sequence SEQ ID NO: 34;
a fourteenth substantially pure protein having at least 90% homology to amino acid sequence SEQ ID NO: 35;
a fifteenth substantially pure protein having at least 90% homology to amino acid sequence SEQ ID NO: 36; and
a sixteenth substantially pure protein having at least 90% homology to amino acid sequence SEQ ID NO: 37.

5. An isolated purified IVIG plasma product comprising an isolated purified protein complex containing:
a first substantially pure protein having at least 90% homology to amino acid sequence SEQ ID NO: 33;
a second substantially pure protein having at least 90% homology to amino acid sequence SEQ ID NO: 34;
a third substantially pure protein having at least 90% homology to amino acid SEQ ID NO: 35;
a fourth substantially pure protein having at least 90% homology to amino acid sequence SEQ ID NO: 36; and
a fifth substantially pure protein having at least 90% homology to amino acid sequence SEQ ID NO: 37.

6. An isolated purified IVIG plasma product comprising an isolated purified protein complex containing:
a first substantially pure protein having at least 80% homology to amino acid sequence SEQ ID NO: 22, wherein said substantially pure protein has the same activity as the protein defined by amino acid sequence SEQ ID NO: 22;
a second substantially pure protein having at least 80% homology to amino acid sequence SEQ ID NO: 23, wherein said substantially pure protein has the same activity as the protein defined by amino acid sequence SEQ ID NO: 23;
a third substantially pure protein having at least 80% homology to amino acid sequence SEQ ID NO: 24, wherein said substantially pure protein has the same activity as the protein defined by amino acid sequence SEQ ID NO: 24;
a fourth substantially pure protein having at least 80% homology to amino acid sequence SEQ ID NO: 25, wherein said substantially pure protein has the same activity as the protein defined by amino acid sequence SEQ ID NO: 25;
a fifth substantially pure protein having at least 80% homology to amino acid sequence SEQ ID NO: 26, wherein said substantially pure protein has the same activity as the protein defined by amino acid sequence SEQ ID NO: 26;
a sixth substantially pure protein having at least 80% homology to amino acid sequence SEQ ID NO: 27, wherein said substantially pure protein has the same activity as the protein defined by amino acid sequence SEQ ID NO: 27;
a seventh substantially pure protein having at least 80% homology to amino acid sequence SEQ ID NO: 28, wherein said substantially pure protein has the same activity as the protein defined by amino acid sequence SEQ ID NO: 28;
an eighth substantially pure protein having at least 80% homology to amino acid sequence SEQ ID NO: 29, wherein said substantially pure protein has the same activity as the protein defined by amino acid sequence SEQ ID NO: 29;
a ninth substantially pure protein having at least 80% homology to amino acid sequence SEQ ID NO: 30, wherein said substantially pure protein has the same activity as the protein defined by amino acid sequence SEQ ID NO: 30;
a tenth substantially pure protein having at least 80% homology to amino acid sequence SEQ ID NO: 31, wherein said substantially pure protein has the same activity as the protein defined by amino acid sequence SEQ ID NO: 31;
an eleventh substantially pure protein having at least 80% homology to amino acid sequence SEQ ID NO: 32, wherein said substantially pure protein has the same activity as the protein defined by amino acid sequence SEQ ID NO: 32;
a twelfth substantially pure protein having at least 80% homology to amino acid sequence SEQ ID NO: 33, wherein said substantially pure protein has the same activity as the protein defined by amino acid sequence SEQ ID NO: 33;
a thirteenth substantially pure protein having at least 80% homology to amino acid sequence SEQ ID NO: 34, wherein said substantially pure protein has the same activity as the protein defined by amino acid sequence SEQ ID NO: 34;
a fourteenth substantially pure protein having at least 80% homology to amino acid sequence SEQ ID NO: 35, wherein said substantially pure protein has the same activity as the protein defined by amino acid sequence SEQ ID NO: 35;
a fifteenth substantially pure protein having at least 80% homology to amino acid sequence SEQ ID NO: 36, wherein said substantially pure protein has the same activity as the protein defined by amino acid sequence SEQ ID NO: 36; and
a sixteenth substantially pure protein having at least 80% homology to amino acid sequence SEQ ID NO: 37, wherein said substantially pure protein has the same activity as the protein defined by amino acid sequence SEQ ID NO: 37.

7. An isolated purified IVIG plasma product comprising an isolated purified protein complex containing:
a first substantially pure protein having at least 80% homology to amino acid sequence SEQ ID NO: 33, wherein said substantially pure protein has the same activity as the protein defined by amino acid sequence SEQ ID NO: 33;
a second substantially pure protein having at least 80% homology to amino acid sequence SEQ ID NO: 34, wherein said substantially pure protein has the same activity as the protein defined by amino acid sequence SEQ ID NO: 34;
a third substantially pure protein having at least 80% homology to amino acid SEQ ID NO: 35, wherein said substantially pure protein has the same activity as the protein defined by amino acid sequence SEQ ID NO: 35;
a fourth substantially pure protein having at least 80% homology to amino acid sequence SEQ ID NO: 36, wherein said substantially pure protein has the same activity as the protein defined by amino acid sequence SEQ ID NO: 36; and
a fifth substantially pure protein having at least 80% homology to amino acid sequence SEQ ID NO: 37, wherein said substantially pure protein has the same activity as the protein defined by amino acid sequence SEQ ID NO: 37.

8. The isolated purified IVIG plasma product of claim 1 wherein the proteins defined by amino acid sequences SEQ ID NOs 33-37 have a concentration of at least 30%.

9. The isolated purified IVIG plasma product of claim 2 wherein the proteins defined by amino acid sequences SEQ ID NOs 22-37 have a concentration of at least 30%.

10. The isolated purified IVIG plasma product of claim 3 wherein the proteins defined by amino acid sequences SEQ ID NOs 22-37, and 51 have a concentration of at least 30%.

11. An isolated purified IVIG plasma product for use in the treatment of hepatitis B by administrating an effective dose of the isolated purified IVIG plasma to a patient, the isolated purified IVIG plasma product comprising:
a first protein defined by amino acid sequence SEQ ID NO: 33;
a second protein defined by amino acid sequence SEQ ID NO: 34;
a third protein defined by amino acid sequence SEQ ID NO 35;
a fourth protein defined by amino acid sequence SEQ ID NO 36; and
a fifth protein defined by amino acid sequence SEQ ID NO 37.

12. The isolated purified IVIG plasma product for use according to claim 11, wherein the isolated purified IVIG plasma product further comprises:
a sixth protein defined by amino acid sequence SEQ ID NO: 22;
a seventh protein defined by amino acid sequence SEQ ID NO: 23;
an eighth protein defined by amino acid sequence SEQ ID NO: 24;
a ninth protein defined by amino acid sequence SEQ ID NO: 25;
a tenth protein defined by amino acid sequence SEQ ID NO: 26;
an eleventh protein defined by amino acid sequence SEQ ID NO: 27;
a twelfth protein defined by amino acid sequence SEQ ID NO: 28;
a thirteenth protein defined by amino acid sequence SEQ ID NO: 29;
a fourteenth protein defined by amino acid sequence SEQ ID NO: 30;
a fifteenth protein defined by amino acid sequence SEQ ID NO: 31; and
a sixteenth protein defined by amino acid sequence SEQ ID NO: 32.

13. The isolated purified IVIG plasma product for use according to claim 12 wherein the isolated IVIG plasma product further comprises a sixteenth protein defined by amino acid sequence SEQ ID NO: 51.

14. The isolated purified IVIG plasma product for use according to claim 13 wherein CD62L is regulated to an effective range on CD4+ T cells and/or CD8+ cells in peripheral blood and/or in the spleen of a hepatitis B patient for treating HBV.

15. The isolated purified IVIG plasma product for use according to claim 13 wherein levels selected from one or more of CD4+ T cells, CD28+ T cells, Foxp3+ T cells, B cell, granulocyte, or macrophage are regulated to an effective range in peripheral blood and/or the in the spleen of a hepatitis B patient for treating HBV.

16. A method of manufacturing an isolated purified IVIG plasma product containing proteins defined by amino acid sequences SEQ ID NOs 33-37 comprising:
a) obtaining a fraction II+III paste from human blood plasma;
b) suspending and diluting the fraction II+III paste at a ratio of 1:17;
c) adjusting the pH of the diluted fraction II+III paste to a pH of 5.2 to produce an adjusted fraction II+III paste;
d) adding ethanol to the adjusted fraction II+III paste to produce a 17% ethanol fraction II+III product;
e) press filtering the 17% ethanol fraction II+III product and collecting a filtrate comprising fraction III;
f) concentrating the filtrate by ultrafiltration to form a 100k ultrafiltrate;
g) adjusting the pH of the 100k ultrafiltrate to a pH of 4.0;
h) filtering the pH adjusted ultrafiltrate using a .45 um filter and collecting a filtered product; and
i) nanofiltrating the filtered product with a 50 nm filter to obtain a final product.

17. A method of manufacturing an isolated purified IVIG plasma product containing proteins defined by amino acid sequences SEQ ID NOs 22-37 comprising:
a) obtaining a fraction II+III paste from human hepatitis B immune globulin having high levels of hepatitis B surface antigen;
b) suspending and diluting the fraction II+III paste at a ratio of 1:17;
c) adjusting the pH of the diluted fraction II+III paste to a pH of 5.2 to produce an adjusted fraction II+III paste;
d) adding ethanol to the adjusted fraction II+III paste to produce a 17% ethanol fraction II+III product;
e) press filtering the 17% ethanol fraction II+III product and collecting a filtrate comprising fraction III;
f) concentrating the filtrate by ultrafiltration to form a 100k ultrafiltrate;
g) adjusting the pH of the 100k ultrafiltrate to a pH of 4.0;
h) filtering the pH adjusted ultrafiltrate using a .45 um filter and collecting a filtered product; and
i) nanofiltrating the filtered product with a 50 nm filter to obtain a final product.

18. A method of producing an isolated purified IVIG plasma product containing proteins defined by amino acid sequences SEQ ID NOs 22-37, and 51 comprising:
a) manufacturing a non-sterile final bulk of normal immunoglobulin;
b) manufacturing a non-sterile final bulk of hepatitis B immune globulin;
c) creating a immunoglobulin mixture comprising 80% of the non-sterile final bulk of normal immunoglobulin and 20% of the non-sterile final bulk of hepatitis B immune globulin;
d) performing sterile filtration on the immunoglobulin mixture and collecting a final product.

## Patentansprüche

1. Isoliertes gereinigtes IVIG(Intravenous Immunoglobin)-Plasmaprodukt, umfassend einen isolierten gereinigten Proteinkomplex, der durch Aminosäuresequenzen SEQ ID NO 33-37 definierte Proteine enthält.

2. Isolierter gereinigter Proteinkomplex nach Anspruch 1, ferner umfassend Proteine, die durch Aminosäuresequenzen SEQ ID NO 22-31 definiert sind.

3. Isolierter gereinigter Proteinkomplex nach Anspruch 1, ferner umfassend ein Protein, das durch Aminosäuresequenz SEQ ID NO: 51 definiert ist.

4. Isoliertes gereinigtes IVIG-Plasmaprodukt, umfassend einen isolierten gereinigten Proteinkomplex, der Folgendes enthält:
ein erstes weitgehend reines Protein mit einer Homologie von wenigstens 90% zu Aminosäuresequenz SEQ ID NO: 22;
ein zweites weitgehend reines Protein mit einer Homologie von wenigstens 90% zu Aminosäuresequenz SEQ ID NO: 23;
ein drittes weitgehend reines Protein mit einer Homologie von wenigstens 90% zu Aminosäuresequenz SEQ ID NO: 24;
ein viertes weitgehend reines Protein mit einer Homologie von wenigstens 90% zu Aminosäuresequenz SEQ ID NO: 25;
ein fünftes weitgehend reines Protein mit einer Homologie von wenigstens 90% zu Aminosäuresequenz SEQ ID NO: 26;
ein sechstes weitgehend reines Protein mit einer Homologie von wenigstens 90% zu Aminosäuresequenz SEQ ID NO: 27;
ein siebtes weitgehend reines Protein mit einer Homologie von wenigstens 90% zu Aminosäuresequenz SEQ ID NO: 28;
ein achtes weitgehend reines Protein mit einer Homologie von wenigstens 90% zu Aminosäuresequenz SEQ ID NO: 29;
ein neuntes weitgehend reines Protein mit einer Homologie von wenigstens 90% zu Aminosäuresequenz SEQ ID NO: 30;
ein zehntes weitgehend reines Protein mit einer Homologie von wenigstens 90% zu Aminosäuresequenz SEQ ID NO: 31;
ein elftes weitgehend reines Protein mit einer Homologie von wenigstens 90% zu Aminosäuresequenz SEQ ID NO: 32;
ein zwölftes weitgehend reines Protein mit einer Homologie von wenigstens 90% zu Aminosäuresequenz SEQ ID NO: 33;
ein dreizehntes weitgehend reines Protein mit einer Homologie von wenigstens 90% zu Aminosäuresequenz SEQ ID NO: 34;
ein vierzehntes weitgehend reines Protein mit einer Homologie von wenigstens 90% zu Aminosäuresequenz SEQ ID NO: 35;
ein fünfzehntes weitgehend reines Protein mit einer Homologie von wenigstens 90% zu Aminosäuresequenz SEQ ID NO: 36; und
ein sechzehntes weitgehend reines Protein mit einer Homologie von wenigstens 90% zu Aminosäuresequenz SEQ ID NO: 37.

5. Isoliertes gereinigtes IVIG-Plasmaprodukt, umfassend einen isolierten gereinigten Proteinkomplex, der Folgendes enthält:
ein erstes weitgehend reines Protein mit einer Homologie von wenigstens 90% zu Aminosäuresequenz SEQ ID NO: 33;
ein zweites weitgehend reines Protein mit einer Homologie von wenigstens 90% zu Aminosäuresequenz SEQ ID NO: 34;
ein drittes weitgehend reines Protein mit einer Homologie von wenigstens 90% zu Aminosäure SEQ ID NO: 35;
ein viertes weitgehend reines Protein mit einer Homologie von wenigstens 90% zu Aminosäuresequenz SEQ ID NO: 36; und
ein fünftes weitgehend reines Protein mit einer Homologie von wenigstens 90% zu Aminosäuresequenz SEQ ID NO: 37.

6. Isoliertes gereinigtes IVIG-Plasmaprodukt, umfassend einen isolierten gereinigten Proteinkomplex, der Folgendes enthält:
ein erstes weitgehend reines Protein mit einer Homologie von wenigstens 80% zu Aminosäuresequenz SEQ ID NO: 22, wobei das weitgehend reine Protein die gleiche Aktivität aufweist wie das durch Aminosäuresequenz SEQ ID NO: 22 definierte Protein;
ein zweites weitgehend reines Protein mit einer Homologie von wenigstens 80% zu Aminosäuresequenz SEQ ID NO: 23, wobei das weitgehend reine Protein die gleiche Aktivität aufweist wie das durch Aminosäuresequenz SEQ ID NO: 23 definierte Protein;
ein drittes weitgehend reines Protein mit einer Homologie von wenigstens 80% zu Aminosäuresequenz SEQ ID NO: 24, wobei das weitgehend reine Protein die gleiche Aktivität aufweist wie das durch Aminosäuresequenz SEQ ID NO: 24 definierte Protein;
ein viertes weitgehend reines Protein mit einer Homologie von wenigstens 80% zu Aminosäuresequenz SEQ ID NO: 25, wobei das weitgehend reine Protein die gleiche Aktivität aufweist wie das durch Aminosäuresequenz SEQ ID NO: 25 definierte Protein;
ein fünftes weitgehend reines Protein mit einer Homologie von wenigstens 80% zu Aminosäuresequenz SEQ ID NO: 26, wobei das weitgehend reine Protein die gleiche Aktivität aufweist wie das durch Aminosäuresequenz SEQ ID NO: 26 definierte Protein;
ein sechstes weitgehend reines Protein mit einer Homologie von wenigstens 80% zu Aminosäuresequenz SEQ ID NO: 27, wobei das weitgehend reine Protein die gleiche Aktivität aufweist wie das durch Aminosäuresequenz SEQ ID NO: 27 definierte Protein;
ein siebtes weitgehend reines Protein mit einer Homologie von wenigstens 80% zu Aminosäuresequenz SEQ ID NO: 28, wobei das weitgehend reine Protein die gleiche Aktivität aufweist wie das durch Aminosäuresequenz SEQ ID NO: 28 definierte Protein;
ein achtes weitgehend reines Protein mit einer Homologie von wenigstens 80% zu Aminosäuresequenz SEQ ID NO: 29, wobei das weitgehend reine Protein die gleiche Aktivität aufweist wie das durch Aminosäuresequenz SEQ ID NO: 29 definierte Protein;
ein neuntes weitgehend reines Protein mit einer Homologie von wenigstens 80% zu Aminosäuresequenz SEQ ID NO: 30, wobei das weitgehend reine Protein die gleiche Aktivität aufweist wie das durch Aminosäuresequenz SEQ ID NO: 30 definierte Protein;
ein zehntes weitgehend reines Protein mit einer Homologie von wenigstens 80% zu Aminosäuresequenz SEQ ID NO: 31, wobei das weitgehend reine Protein die gleiche Aktivität aufweist wie das durch Aminosäuresequenz SEQ ID NO: 31 definierte Protein;
ein elftes weitgehend reines Protein mit einer Homologie von wenigstens 80% zu Aminosäuresequenz SEQ ID NO: 32, wobei das weitgehend reine Protein die gleiche Aktivität aufweist wie das durch Aminosäuresequenz SEQ ID NO: 32 definierte Protein;
ein zwölftes weitgehend reines Protein mit einer Homologie von wenigstens 80% zu Aminosäuresequenz SEQ ID NO: 33, wobei das weitgehend reine Protein die gleiche Aktivität aufweist wie das durch Aminosäuresequenz SEQ ID NO: 33 definierte Protein;
ein dreizehntes weitgehend reines Protein mit einer Homologie von wenigstens 80% zu Aminosäuresequenz SEQ ID NO: 34, wobei das weitgehend reine Protein die gleiche Aktivität aufweist wie das durch Aminosäuresequenz SEQ ID NO: 34 definierte Protein;
ein vierzehntes weitgehend reines Protein mit einer Homologie von wenigstens 80% zu Aminosäuresequenz SEQ ID NO: 35, wobei das weitgehend reine Protein die gleiche Aktivität aufweist wie das durch Aminosäuresequenz SEQ ID NO: 35 definierte Protein;
ein fünfzehntes weitgehend reines Protein mit einer Homologie von wenigstens 80% zu Aminosäuresequenz SEQ ID NO: 36, wobei das weitgehend reine Protein die gleiche Aktivität aufweist wie das durch Aminosäuresequenz SEQ ID NO: 36 definierte Protein; und
ein sechzehntes weitgehend reines Protein mit einer Homologie von wenigstens 80% zu Aminosäuresequenz SEQ ID NO: 37, wobei das weitgehend reine Protein die gleiche Aktivität aufweist wie das durch Aminosäuresequenz SEQ ID NO: 37 definierte Protein.

7. Isoliertes gereinigtes IVIG-Plasmaprodukt, umfassend einen isolierten gereinigten Proteinkomplex, der Folgendes enthält:
ein erstes weitgehend reines Protein mit einer Homologie von wenigstens 80% zu Aminosäuresequenz SEQ ID NO: 33, wobei das weitgehend reine Protein die gleiche Aktivität aufweist wie das durch Aminosäuresequenz SEQ ID NO: 33 definierte Protein;
ein zweites weitgehend reines Protein mit einer Homologie von wenigstens 80% zu Aminosäuresequenz SEQ ID NO: 34, wobei das weitgehend reine Protein die gleiche Aktivität aufweist wie das durch Aminosäuresequenz SEQ ID NO: 34 definierte Protein;
ein drittes weitgehend reines Protein mit einer Homologie von wenigstens 80% zu Aminosäure SEQ ID NO: 35, wobei das weitgehend reine Protein die gleiche Aktivität aufweist wie das durch Aminosäuresequenz SEQ ID NO: 35 definierte Protein;
ein viertes weitgehend reines Protein mit einer Homologie von wenigstens 80% zu Aminosäuresequenz SEQ ID NO: 36, wobei das weitgehend reine Protein die gleiche Aktivität aufweist wie das durch Aminosäuresequenz SEQ ID NO: 36 definierte Protein; und
ein fünftes weitgehend reines Protein mit einer Homologie von wenigstens 80% zu Aminosäuresequenz SEQ ID NO: 37, wobei das weitgehend reine Protein die gleiche Aktivität aufweist wie das durch Aminosäuresequenz SEQ ID NO: 37 definierte Protein.

8. Isoliertes gereinigtes IVIG-Plasmaprodukt nach Anspruch 1, wobei die durch Aminosäuresequenzen SEQ ID NO 33-37 definierten Proteine eine Konzentration von wenigstens 30% aufweisen.

9. Isoliertes gereinigtes IVIG-Plasmaprodukt nach Anspruch 2, wobei die durch Aminosäuresequenzen SEQ ID NO 22-37 definierten Proteine eine Konzentration von wenigstens 30% aufweisen.

10. Isoliertes gereinigtes IVIG-Plasmaprodukt nach Anspruch 3, wobei die durch Aminosäuresequenzen SEQ ID NO 22-37 und 51 definierten Proteine eine Konzentration von wenigstens 30% aufweisen.

11. Isoliertes gereinigtes IVIG-Plasmaprodukt zur Verwendung bei der Behandlung von Hepatitis B durch Verabreichen einer wirksamen Dosis des isolierten gereinigten IVIG-Plasmas an einen Patienten, wobei das isolierte gereinigte IVIG-Plasmaprodukt Folgendes umfasst:
ein durch Aminosäuresequenz SEQ ID NO: 33 definiertes erstes Protein;
ein durch Aminosäuresequenz SEQ ID NO: 34 definiertes zweites Protein;
ein durch Aminosäuresequenz SEQ ID NO: 35 definiertes drittes Protein;
ein durch Aminosäuresequenz SEQ ID NO: 36 definiertes viertes Protein; und
ein durch Aminosäuresequenz SEQ ID NO: 37 definiertes fünftes Protein.

12. Isoliertes gereinigtes IVIG-Plasmaprodukt zur Verwendung gemäß Anspruch 11, wobei das isolierte gereinigte IVIG-Plasmaprodukt ferner Folgendes umfasst:
ein durch Aminosäuresequenz SEQ ID NO: 22 definiertes sechstes Protein;
ein durch Aminosäuresequenz SEQ ID NO: 23 definiertes siebtes Protein;
ein durch Aminosäuresequenz SEQ ID NO: 24 definiertes achtes Protein;
ein durch Aminosäuresequenz SEQ ID NO: 25 definiertes neuntes Protein;
ein durch Aminosäuresequenz SEQ ID NO: 26 definiertes zehntes Protein;
ein durch Aminosäuresequenz SEQ ID NO: 27 definiertes elftes Protein;
ein durch Aminosäuresequenz SEQ ID NO: 28 definiertes zwölftes Protein;
ein durch Aminosäuresequenz SEQ ID NO: 29 definiertes dreizehntes Protein;
ein durch Aminosäuresequenz SEQ ID NO: 30 definiertes vierzehntes Protein;
ein durch Aminosäuresequenz SEQ ID NO: 31 definiertes fünfzehntes Protein; und
ein durch Aminosäuresequenz SEQ ID NO: 32 definiertes sechzehntes Protein.

13. Isoliertes gereinigtes IVIG-Plasmaprodukt zur Verwendung gemäß Anspruch 12, wobei das isolierte gereinigte IVIG-Plasmaprodukt ferner ein durch Aminosäuresequenz SEQ ID NO: 51 definiertes sechzehntes Protein umfasst.

14. Isoliertes gereinigtes IVIG-Plasmaprodukt zur Verwendung gemäß Anspruch 13, wobei CD62L auf einen wirksamen Bereich auf CD4+-T-Zellen und/oder CD8+-Zellen in peripherem Blut und/oder in der Milz eines Hepatitis-B-Patienten zur Behandlung von HBV reguliert ist.

15. Isoliertes gereinigtes IVIG-Plasmaprodukt zur Verwendung gemäß Anspruch 13, wobei Spiegel ausgewählt aus einem oder mehreren von CD4+-T-Zellen, CD28+-T-Zellen, Foxp3+-T-Zellen, B-Zelle, Granulozyt oder Makrophage auf einen wirksamen Bereich in peripherem Blut und/oder in der Milz eines Hepatitis-B-Patienten zur Behandlung von HBV reguliert sind.

16. Verfahren zur Herstellung eines isolierten gereinigten IVIG-Plasmaprodukts, das durch Aminosäuresequenzen SEQ ID NO 33-37 definierte Proteine enthält, umfassend:
a) Gewinnen einer Fraktion-II+III-Paste von menschlichem Blutplasma;
b) Suspendieren und Verdünnen der Fraktion-II+III-Paste in einem Verhältnis von 1:17;
c) Einstellen des pH der verdünnten Fraktion-II+III-Paste auf einen pH von 5,2 unter Erhalt einer eingestellten Fraktion-II+III-Paste;
d) Versetzen der eingestellten Fraktion-II+III-Paste mit Ethanol unter Erhalt eines 17%-Ethanol-Fraktion-II+III-Produkts;
e) Druckfiltrieren des 17%-Ethanol-Fraktion-II+III-Produkts und Sammeln eines Fraktion III umfassenden Filtrats;
f) Konzentrieren des Filtrats durch Ultrafiltration unter Bildung eines 100k-Ultrafiltrats;
g) Einstellen des pH des 100k-Ultrafiltrats auf einen pH von 4,0;
h) Filtrieren des pH-eingestellten Ultrafiltrats unter Verwendung eines 0,45-um-Filters und Sammeln eines filtrierten Produkts; und
i) Nanofiltrieren des filtrierten Produkts mit einem 50-nm-Filter, so dass ein Endprodukt gewonnen wird.

17. Verfahren zur Herstellung eines isolierten gereinigten IVIG-Plasmaprodukts, das durch Aminosäuresequenzen SEQ ID NO 22-37 definierte Proteine enthält, umfassend:
a) Gewinnen einer Fraktion-II+III-Paste von menschlichem Hepatitis-B-Immunglobulin mit hohen Spiegeln von Hepatitis-B-Oberflächenantigen;
b) Suspendieren und Verdünnen der Fraktion-II+III-Paste in einem Verhältnis von 1:17;
c) Einstellen des pH der verdünnten Fraktion-II+III-Paste auf einen pH von 5,2 unter Erhalt einer eingestellten Fraktion-II+III-Paste;
d) Versetzen der eingestellten Fraktion-II+III-Paste mit Ethanol unter Erhalt eines 17%-Ethanol-Fraktion-II+III-Produkts;
e) Druckfiltrieren des 17%-Ethanol-Fraktion-II+III-Produkts und Sammeln eines Fraktion III umfassenden Filtrats;
f) Konzentrieren des Filtrats durch Ultrafiltration unter Bildung eines 100k-Ultrafiltrats;
g) Einstellen des pH des 100k-Ultrafiltrats auf einen pH von 4,0;
h) Filtrieren des pH-eingestellten Ultrafiltrats unter Verwendung eines 0,45-um-Filters und Sammeln eines filtrierten Produkts; und
i) Nanofiltrieren des filtrierten Produkts mit einem 50-nm-Filter, so dass ein Endprodukt gewonnen wird.

18. Verfahren zur Erzeugung eines isolierten gereinigten IVIG-Plasmaprodukts, das durch Aminosäuresequenzen SEQ ID NO 22-37 und 51 definierte Proteine enthält, umfassend:
a) Herstellen einer nicht sterilen Endmasse von normalem Immunglobulin;
b) Herstellen einer nicht sterilen Endmasse von Hepatitis-B-Immunglobulin;
c) Schaffen eines Immunglobulingemischs, das 80% der nicht sterilen Endmasse von normalem Immunglobulin und 20% der nicht sterilen Endmasse von Hepatitis-B-Immunglobulin umfasst;
d) Durchführen von Sterilfiltration mit dem Immunglobulingemisch und Sammeln eines Endprodukts.

## Revendications

1. Produit plasmatique d'immunoglobulines intraveineuses (IGIV) purifié isolé comprenant un complexe protéique purifié isolé contenant des protéines définies par les séquences d'acides aminés SEQ ID n° : 33 à 37.

2. Complexe protéique purifié isolé de la revendication 1 comprenant en outre des protéines définies par les séquences d'acides aminés SEQ ID n° : 22 à 31.

3. Complexe protéique purifié isolé de la revendication 1 comprenant en outre une protéine définie par la séquence d'acides aminés SEQ ID n° : 51.

4. Produit plasmatique d'IGIV purifié isolé comprenant un complexe protéique purifié isolé contenant :
une première protéine substantiellement pure ayant une homologie d'au moins 90% avec la séquence d'acides aminés SEQ ID n° : 22 ;
une deuxième protéine substantiellement pure ayant une homologie d'au moins 90% avec la séquence d'acides aminés SEQ ID n° : 23 ;
une troisième protéine substantiellement pure ayant une homologie d'au moins 90% avec la séquence d'acides aminés SEQ ID n° : 24 ;
une quatrième protéine substantiellement pure ayant une homologie d'au moins 90% avec la séquence d'acides aminés SEQ ID n° : 25 ;
une cinquième protéine substantiellement pure ayant une homologie d'au moins 90% avec la séquence d'acides aminés SEQ ID n° : 26 ;
une sixième protéine substantiellement pure ayant une homologie d'au moins 90% avec la séquence d'acides aminés SEQ ID n° : 27 ;
une septième protéine substantiellement pure ayant une homologie d'au moins 90% avec la séquence d'acides aminés SEQ ID n° : 28 ;
une huitième protéine substantiellement pure ayant une homologie d'au moins 90% avec la séquence d'acides aminés SEQ ID n° : 29 ;
une neuvième protéine substantiellement pure ayant une homologie d'au moins 90% avec la séquence d'acides aminés SEQ ID n° : 30 ;
une dixième protéine substantiellement pure ayant une homologie d'au moins 90% avec la séquence d'acides aminés SEQ ID n° : 31 ;
une onzième protéine substantiellement pure ayant une homologie d'au moins 90% avec la séquence d'acides aminés SEQ ID n° : 32 ;
une douzième protéine substantiellement pure ayant une homologie d'au moins 90% avec la séquence d'acides aminés SEQ ID n° : 33 ;
une treizième protéine substantiellement pure ayant une homologie d'au moins 90% avec la séquence d'acides aminés SEQ ID n° : 34 ;
une quatorzième protéine substantiellement pure ayant une homologie d'au moins 90% avec la séquence d'acides aminés SEQ ID n° : 35 ;
une quinzième protéine substantiellement pure ayant une homologie d'au moins 90% avec la séquence d'acides aminés SEQ ID n° : 36 ; et
une seizième protéine substantiellement pure ayant une homologie d'au moins 90% avec la séquence d'acides aminés SEQ ID n° : 37.

5. Produit plasmatique d'IGIV purifié isolé comprenant un complexe protéique purifié isolé contenant :
une première protéine substantiellement pure ayant une homologie d'au moins 90% avec la séquence d'acides aminés SEQ ID n° : 33 ;
une deuxième protéine substantiellement pure ayant une homologie d'au moins 90% avec la séquence d'acides aminés SEQ ID n° : 34 ;
une troisième protéine substantiellement pure ayant une homologie d'au moins 90% avec l'acide aminé SEQ ID n° : 35 ;
une quatrième protéine substantiellement pure ayant une homologie d'au moins 90% avec la séquence d'acides aminés SEQ ID n° : 36 ; et
une cinquième protéine substantiellement pure ayant une homologie d'au moins 90% avec la séquence d'acides aminés SEQ ID n° : 37.

6. Produit plasmatique d'IGIV purifié isolé comprenant un complexe protéique purifié isolé contenant :
une première protéine substantiellement pure ayant une homologie d'au moins 80% avec la séquence d'acides aminés SEQ ID n° : 22, dans lequel ladite protéine substantiellement pure a la même activité que la protéine définie par la séquence d'acides aminés SEQ ID n° : 22 ;
une deuxième protéine substantiellement pure ayant une homologie d'au moins 80% avec la séquence d'acides aminés SEQ ID n° : 23, dans lequel ladite protéine substantiellement pure a la même activité que la protéine définie par la séquence d'acides aminés SEQ ID n° : 23 ;
une troisième protéine substantiellement pure ayant une homologie d'au moins 80% avec la séquence d'acides aminés SEQ ID n° : 24, dans lequel ladite protéine substantiellement pure a la même activité que la protéine définie par la séquence d'acides aminés SEQ ID n° : 24 ;
une quatrième protéine substantiellement pure ayant une homologie d'au moins 80% avec la séquence d'acides aminés SEQ ID n° : 25, dans lequel ladite protéine substantiellement pure a la même activité que la protéine définie par la séquence d'acides aminés SEQ ID n° : 25 ;
une cinquième protéine substantiellement pure ayant une homologie d'au moins 80% avec la séquence d'acides aminés SEQ ID n° : 26, dans lequel ladite protéine substantiellement pure a la même activité que la protéine définie par la séquence d'acides aminés SEQ ID n° : 26 ;
une sixième protéine substantiellement pure ayant une homologie d'au moins 80% avec la séquence d'acides aminés SEQ ID n° : 27, dans lequel ladite protéine substantiellement pure a la même activité que la protéine définie par la séquence d'acides aminés SEQ ID n° : 27 ;
une septième protéine substantiellement pure ayant une homologie d'au moins 80% avec la séquence d'acides aminés SEQ ID n° : 28, dans lequel ladite protéine substantiellement pure a la même activité que la protéine définie par la séquence d'acides aminés SEQ ID n° : 28 ;
une huitième protéine substantiellement pure ayant une homologie d'au moins 80% avec la séquence d'acides aminés SEQ ID n° : 29, dans lequel ladite protéine substantiellement pure a la même activité que la protéine définie par la séquence d'acides aminés SEQ ID n° : 29 ;
une neuvième protéine substantiellement pure ayant une homologie d'au moins 80% avec la séquence d'acides aminés SEQ ID n° : 30, dans lequel ladite protéine substantiellement pure a la même activité que la protéine définie par la séquence d'acides aminés SEQ ID n° : 30 ;
une dixième protéine substantiellement pure ayant une homologie d'au moins 80% avec la séquence d'acides aminés SEQ ID n° : 31, dans lequel ladite protéine substantiellement pure a la même activité que la protéine définie par la séquence d'acides aminés SEQ ID n° : 31 ;
une onzième protéine substantiellement pure ayant une homologie d'au moins 80% avec la séquence d'acides aminés SEQ ID n° : 32, dans lequel ladite protéine substantiellement pure a la même activité que la protéine définie par la séquence d'acides aminés SEQ ID n° : 32 ;
une douzième protéine substantiellement pure ayant une homologie d'au moins 80% avec la séquence d'acides aminés SEQ ID n° : 33, dans lequel ladite protéine substantiellement pure a la même activité que la protéine définie par la séquence d'acides aminés SEQ ID n° : 33 ;
une treizième protéine substantiellement pure ayant une homologie d'au moins 80% avec la séquence d'acides aminés SEQ ID n° : 34, dans lequel ladite protéine substantiellement pure a la même activité que la protéine définie par la séquence d'acides aminés SEQ ID n° : 34 ;
une quatorzième protéine substantiellement pure ayant une homologie d'au moins 80% avec la séquence d'acides aminés SEQ ID n° : 35, dans lequel ladite protéine substantiellement pure a la même activité que la protéine définie par la séquence d'acides aminés SEQ ID n° : 35 ;
une quinzième protéine substantiellement pure ayant une homologie d'au moins 80% avec la séquence d'acides aminés SEQ ID n° : 36, dans lequel ladite protéine substantiellement pure a la même activité que la protéine définie par la séquence d'acides aminés SEQ ID n° : 36 ; et
une seizième protéine substantiellement pure ayant une homologie d'au moins 80% avec la séquence d'acides aminés SEQ ID n° : 37, dans lequel ladite protéine substantiellement pure a la même activité que la protéine définie par la séquence d'acides aminés SEQ ID n° : 37.

7. Produit plasmatique d'IGIV purifié isolé comprenant un complexe protéique purifié isolé contenant :
une première protéine substantiellement pure ayant une homologie d'au moins 80% avec la séquence d'acides aminés SEQ ID n° : 33, dans lequel ladite protéine substantiellement pure a la même activité que la protéine définie par la séquence d'acides aminés SEQ ID n° : 33 ;
une deuxième protéine substantiellement pure ayant une homologie d'au moins 80% avec la séquence d'acides aminés SEQ ID n° : 34, dans lequel ladite protéine substantiellement pure a la même activité que la protéine définie par la séquence d'acides aminés SEQ ID n° : 34 ;
une troisième protéine substantiellement pure ayant une homologie d'au moins 80% avec l'acide aminé SEQ ID n° : 35, dans lequel ladite protéine substantiellement pure a la même activité que la protéine définie par la séquence d'acides aminés SEQ ID n° : 35 ;
une quatrième protéine substantiellement pure ayant une homologie d'au moins 80% avec la séquence d'acides aminés SEQ ID n° : 36, dans lequel ladite protéine substantiellement pure a la même activité que la protéine définie par la séquence d'acides aminés SEQ ID n° : 36 ; et
une cinquième protéine substantiellement pure ayant une homologie d'au moins 80% avec la séquence d'acides aminés SEQ ID n° : 37, dans lequel ladite protéine substantiellement pure a la même activité que la protéine définie par la séquence d'acides aminés SEQ ID n° : 37.

8. Produit plasmatique d'IGIV purifié isolé de la revendication 1, dans lequel les protéines définies par les séquences d'acides aminés SEQ ID n° : 33 à 37 ont une concentration d'au moins 30%.

9. Produit plasmatique d'IGIV purifié isolé de la revendication 2, dans lequel les protéines définies par les séquences d'acides aminés SEQ ID n° : 22 à 37 ont une concentration d'au moins 30%.

10. Produit plasmatique d'IGIV purifié isolé de la revendication 3, dans lequel les protéines définies par les séquences d'acides aminés SEQ ID n° : 22 à 37 et 51 ont une concentration d'au moins 30%.

11. Produit plasmatique d'IGIV purifié isolé destiné à être utilisé dans le traitement de l'hépatite B par une administration d'une dose efficace du plasma d'IGIV purifié isolé à un patient, le produit plasmatique d'IGIV purifié isolé comprenant :
une première protéine définie par la séquence d'acides aminés SEQ ID n° : 33 ;
une deuxième protéine définie par la séquence d'acides aminés SEQ ID n° : 34 ;
une troisième protéine définie par la séquence d'acides aminés SEQ ID n° : 35 ;
une quatrième protéine définie par la séquence d'acides aminés SEQ ID n° : 36 ; et
une cinquième protéine définie par la séquence d'acides aminés SEQ ID n° : 37.

12. Produit plasmatique d'IGIV purifié isolé destiné à être utilisé selon la revendication 11, le produit plasmatique d'IGIV purifié isolé comprenant en outre :
une sixième protéine définie par la séquence d'acides aminés SEQ ID n° : 22 ;
une septième protéine définie par la séquence d'acides aminés SEQ ID n° : 23 ;
une huitième protéine définie par la séquence d'acides aminés SEQ ID n° : 24 ;
une neuvième protéine définie par la séquence d'acides aminés SEQ ID n° : 25 ;
une dixième protéine définie par la séquence d'acides aminés SEQ ID n° : 26 ;
une onzième protéine définie par la séquence d'acides aminés SEQ ID n° : 27 ;
une douzième protéine définie par la séquence d'acides aminés SEQ ID n° : 28 ;
une treizième protéine définie par la séquence d'acides aminés SEQ ID n° : 29 ;
une quatorzième protéine définie par la séquence d'acides aminés SEQ ID n° : 30 ;
une quinzième protéine définie par la séquence d'acides aminés SEQ ID n° : 31 ; et
une seizième protéine définie par la séquence d'acides aminés SEQ ID n° : 32.

13. Produit plasmatique d'IGIV purifié isolé destiné à être utilisé selon la revendication 12, le produit plasmatique d'IGIV purifié isolé comprenant en outre une seizième protéine définie par la séquence d'acides aminés SEQ ID n° : 51.

14. Produit plasmatique d'IGIV purifié isolé destiné à être utilisé selon la revendication 13, dans lequel le CD62L est régulé jusqu'à une plage efficace sur des lymphocytes T CD4+ et/ou des lymphocytes CD8+ dans le sang périphérique et/ou dans la rate d'un patient à hépatite B pour traiter un VHB.

15. Produit plasmatique d'IGIV purifié isolé destiné à être utilisé selon la revendication 13, dans lequel des taux choisis à partir d'un ou de plusieurs d'entre des lymphocytes T CD4+, lymphocytes T CD28+, lymphocytes T Foxp3+, lymphocytes B, granulocytes ou macrophages sont régulés jusqu'à une plage efficace dans le sang périphérique et/ou dans la rate d'un patient à hépatite B pour traiter un VHB.

16. Procédé de fabrication d'un produit plasmatique d'IGIV purifié isolé contenant des protéines définies par les séquences d'acides aminés SEQ ID n° : 33 à 37 comprenant les étapes consistant à :
a) obtenir une pâte des fractions II+III à partir de plasma sanguin humain ;
b) mettre en suspension et diluer la pâte des fractions II+III à un rapport de 1:17 ;
c) ajuster le pH de la pâte des fractions II+III diluée jusqu'à un pH de 5,2 pour produire une pâte des fractions II+III ajustée ;
d) ajouter de l'éthanol à la pâte des fractions II+III ajustée pour produire un produit éthanolique à 17% des fractions II+III ;
e) filtrer par presse le produit éthanolique à 17% des fractions II+III et recueillir un filtrat comprenant la fraction III ;
f) concentrer le filtrat par ultrafiltration pour former un ultrafiltrat de 100k ;
g) ajuster le pH de l'ultrafiltrat de 100k jusqu'à un pH de 4,0 ;
h) filtrer l'ultrafiltrat à pH ajusté en utilisant un filtre de 0,45 um et recueillir un produit filtré ; et
i) nano-filtrer le produit filtré avec un filtre de 50 nm pour obtenir un produit final.

17. Procédé de fabrication d'un produit plasmatique d'IGIV purifié isolé contenant des protéines définies par les séquences d'acides aminés SEQ ID n° : 22 à 37 comprenant les étapes consistant à :
a) obtenir une pâte des fractions II+III à partir d'immunoglobulines d'hépatite B humaines ayant des taux élevés d'un antigène de surface de l'hépatite B ;
b) mettre en suspension et diluer la pâte des fractions II+III à un rapport de 1:17 ;
c) ajuster le pH de la pâte des fractions II+III diluée jusqu'à un pH de 5,2 pour produire une pâte des fractions II+III ajustée ;
d) ajouter de l'éthanol à la pâte des fractions II+III ajustée pour produire un produit éthanolique à 17% des fractions II+III ;
e) filtrer par presse le produit éthanolique à 17% des fractions II+III et recueillir un filtrat comprenant la fraction III ;
f) concentrer le filtrat par ultrafiltration pour former un ultrafiltrat de 100k ;
g) ajuster le pH de l'ultrafiltrat de 100k jusqu'à un pH de 4,0 ;
h) filtrer l'ultrafiltrat à pH ajusté en utilisant un filtre de 0,45 um et recueillir un produit filtré ; et
i) nano-filtrer le produit filtré avec un filtre de 50 nm pour obtenir un produit final.

18. Procédé de production d'un produit plasmatique d'IGIV purifié isolé contenant des protéines définies par les séquences d'acides aminés SEQ ID n° : 22 à 37 et 51 comprenant les étapes consistant à :
a) fabriquer un volume final non stérile d'immunoglobulines normales ;
b) fabriquer un volume final non stérile d'immunoglobulines d'hépatite B ;
c) créer un mélange d'immunoglobulines comprenant 80% du volume final non stérile d'immunoglobulines normales et 20% du volume final non stérile d'immunoglobulines d'hépatite B ;
d) effectuer une filtration stérile sur le mélange d'immunoglobulines et recueillir un produit final.


### SEQUENCE LISTING

<110> Hoang, Kieu
<120> Purified compositions of IVIG and KH proteins for modulating lymphocytes and treating hepatitis B virus
<130> RAA.031US
<160> 54
<170> PatentIn version 3.5
<210> 1
   <211> 775
   <212> PRT
   <213> Homo sapiens
<400> 1
   Met Asp Thr Tyr Ile Glu Ser His Phe Ala Gly Ala Leu Ala Tyr Arg
   1               5                   10                  15
   Asp Leu Ile Lys Val Leu Lys Trp Tyr Val Asp Arg Ile Thr Glu Ala
               20                  25                  30
   Glu Arg Gln Glu His Ile Gln Glu Val Leu Lys Ala Gln Glu Tyr Ile
           35                  40                  45
   Phe Lys Tyr Ile Val Gln Ser Arg Arg Leu Phe Ser Leu Ala Thr Gly
       50                  55                  60
   Gly Gln Asn Glu Glu Glu Phe Arg Cys Cys Ile Gln Glu Leu Leu Met
   65                  70                  75                  80
   Ser Val Arg Phe Phe Leu Ser Gln Glu Ser Lys Gly Ser Gly Ala Leu
                   85                  90                  95
   Ser Gln Ser Gln Ala Val Phe Leu Ser Ser Phe Pro Ala Val Tyr Ser
               100                 105                 110
   Glu Leu Leu Lys Leu Phe Asp Val Arg Glu Val Ala Asn Leu Val Gln
           115                 120                 125
   Asp Thr Leu Gly Ser Leu Pro Thr Ile Leu His Val Asp Asp Ser Leu
       130                 135                 140
   Gln Ala Ile Lys Leu Gln Cys Ile Gly Lys Thr Val Glu Ser Gln Leu
   145                 150                 155                 160
   Tyr Thr Asn Pro Asp Ser Arg Tyr Ile Leu Leu Pro Val Val Leu His
                   165                 170                 175
   His Leu His Ile His Leu Gln Glu Gln Lys Asp Leu Ile Met Cys Ala
   180 185 190
   Arg Ile Leu Ser Asn Val Phe Cys Leu Ile Lys Lys Asn Ser Ser Glu
   195 200 205
   Lys Ser Val Leu Glu Glu Ile Asp Val Ile Val Ala Ser Leu Leu Asp
   210 215 220
   Ile Leu Leu Arg Thr Ile Leu Glu Ile Thr Ser Arg Pro Gln Pro Ser
   225 230 235 240
   Ser Ser Ala Met Arg Phe Gln Phe Gln Asp Val Thr Gly Glu Phe Val
   245 250 255
   Ala Cys Leu Leu Ser Leu Leu Arg Gln Met Thr Asp Arg His Tyr Gln
   260 265 270
   Gln Leu Leu Asp Ser Phe Asn Thr Lys Glu Glu Leu Arg Asp Phe Leu
   275 280 285
   Leu Gln Ile Phe Thr Val Phe Arg Ile Leu Ile Arg Pro Glu Met Phe
   290 295 300
   Pro Lys Asp Trp Thr Val Met Arg Leu Val Ala Asn Asn Val Ile Ile
   305 310 315 320
   Thr Thr Val Leu Tyr Leu Ser Asp Ala Leu Arg Lys Asn Phe Leu Asn
   325 330 335
   Glu Asn Phe Asp Tyr Lys Ile Trp Asp Ser Tyr Phe Tyr Leu Ala Val
   340 345 350
   Ile Phe Ile Asn Gln Leu Cys Leu Gln Leu Glu Met Phe Thr Pro Ser
   355 360 365
   Lys Lys Lys Lys Val Leu Glu Lys Tyr Gly Asp Met Arg Val Thr Met
   370 375 380
   Gly Cys Glu Ile Phe Ser Met Trp Gln Asn Leu Gly Glu His Lys Leu
   385 390 395 400
   His Phe Ile Pro Ala Leu Ile Gly Pro Phe Leu Glu Val Thr Leu Ile
   405 410 415
   Pro Gln Pro Asp Leu Arg Asn Val Met Ile Pro Ile Phe His Asp Met
   420 425 430
   Met Asp Trp Glu Gln Arg Arg Ser Gly Asn Phe Lys Gln Val Glu Ala
   435 440 445
   Lys Leu Ile Asp Lys Leu Asp Ser Leu Met Ser Glu Gly Glu Gly Asp
   450 455 460
   Glu Thr Tyr Arg Glu Leu Phe Asn Ser Ile Ile Pro Leu Phe Gly Pro
   465 470 475 480
   Tyr Pro Ser Leu Leu Lys Lys Ile Glu Arg Glu Thr Trp Arg Glu Ser
   485 490 495
   Gly Val Ser Leu Ile Ala Thr Val Thr Arg Leu Met Glu Arg Leu Leu
   500 505 510
   Asp Tyr Arg Asp Cys Met Lys Met Gly Glu Val Asp Gly Lys Lys Ile
   515 520 525
   Gly Cys Thr Val Ser Leu Leu Asn Phe Tyr Lys Thr Glu Leu Asn Lys
   530 535 540
   Glu Glu Met Tyr Ile Arg Tyr Ile His Lys Leu Tyr Asp Leu His Leu
   545 550 555 560
   Lys Ala Gln Asn Phe Thr Glu Ala Ala Tyr Thr Leu Leu Leu Tyr Asp
   565 570 575
   Glu Leu Leu Glu Trp Ser Asp Arg Pro Leu Arg Glu Phe Leu Thr Tyr
   580 585 590
   Pro Met Gln Thr Glu Trp Gln Arg Lys Glu His Leu His Leu Thr Ile
   595 600 605
   Ile Gln Asn Phe Asp Arg Gly Lys Cys Trp Glu Asn Gly Ile Ile Leu
   610 615 620
   Cys Arg Lys Ile Ala Glu Gln Tyr Glu Ser Tyr Tyr Asp Tyr Arg Asn
   625 630 635 640
   Leu Ser Lys Met Arg Met Met Glu Ala Ser Leu Tyr Asp Lys Ile Met
   645 650 655
   Asp Gln Gln Arg Leu Glu Pro Glu Phe Phe Arg Val Gly Phe Tyr Gly
   660 665 670
   Lys Lys Phe Pro Phe Phe Leu Arg Asn Lys Glu Phe Val Cys Arg Gly
               675                 680                 685
   His Asp Tyr Glu Arg Leu Glu Ala Phe Gln Gln Arg Met Leu Asn Glu
       690                 695                 700
   Phe Pro His Ala Ile Ala Met Gln His Ala Asn Gln Pro Asp Glu Thr
   705                 710                 715                 720
   Ile Phe Gln Ala Glu Ala Gln Tyr Leu Gln Ile Tyr Ala Val Thr Pro
                   725                 730                 735
   Ile Pro Glu Ser Gln Glu Val Leu Gln Arg Glu Gly Val Pro Asp Asn
               740                 745                 750
   Ile Lys Ser Phe Tyr Lys Val Asn His Ile Trp Lys Phe Arg Tyr Asp
           755                 760                 765
   Arg Pro Phe His Lys Gly Thr
       770                 775
<210> 2
   <211> 241
   <212> PRT
   <213> Homo Sapiens
<400> 2
   Asp Glu Pro Pro Gln Ser Pro Trp Asp Arg Val Lys Asp Leu Ala Thr
   1               5                   10                  15
   Val Tyr Val Asp Val Leu Lys Asp Ser Gly Arg Asp Tyr Val Ser Gln
               20                  25                  30
   Phe Glu Gly Ser Ala Leu Gly Lys Gln Leu Asn Leu Lys Leu Leu Asp
           35                  40                  45
   Asn Trp Asp Ser Val Thr Ser Thr Phe Ser Lys Leu Arg Glu Gln Leu
       50                  55                  60
   Gly Pro Val Thr Gln Glu Phe Trp Asp Asn Leu Glu Lys Glu Thr Glu
   65                  70                  75                  80
   Gly Leu Arg Gln Glu Met Ser Lys Asp Leu Glu Glu Val Lys Ala Lys
                   85                  90                  95
   Val Gln Pro Tyr Leu Asp Asp Phe Gln Lys Lys Trp Gln Glu Glu Met
               100                 105                 110
   Glu Leu Tyr Arg Gln Lys Val Glu Pro Leu Arg Ala Glu Leu Gln Glu
               115                 120                 125
   Gly Ala Arg Gln Lys Leu His Glu Leu Gln Glu Lys Leu Ser Pro Leu
       130                 135                 140
   Gly Glu Glu Met Arg Asp Cys Ala Arg Ala His Val Asp Ala Leu Arg
   145                 150                 155                 160
   Thr His Leu Ala Pro Tyr Ser Asp Glu Leu Arg Gln Arg Leu Ala Ala
                   165                 170                 175
   Arg Leu Glu Ala Leu Lys Glu Asn Gly Gly Ala Arg Leu Ala Glu Tyr
               180                 185                 190
   His Ala Lys Ala Thr Glu His Leu Ser Thr Leu Ser Glu Lys Ala Lys
           195                 200                 205
   Pro Ala Leu Glu Asp Leu Arg Gln Gly Leu Leu Pro Val Leu Glu Ser
       210                 215                 220
   Phe Lys Val Ser Phe Leu Ser Ala Leu Glu Glu Tyr Thr Lys Lys Leu
   225                 230                 235                 240
   Asn
<210> 3
   <211> 243
   <212> PRT
   <213> Homo Sapiens
<400> 3
   Asp Glu Pro Pro Gln Ser Pro Trp Asp Arg Val Lys Asp Leu Ala Thr
   1               5                   10                  15
   Val Tyr Val Asp Val Leu Lys Asp Ser Gly Arg Asp Tyr Val Ser Gln
               20                  25                  30
   Phe Glu Gly Ser Ala Leu Gly Lys Gln Leu Asn Leu Lys Leu Leu Asp
           35                  40                  45
   Asn Trp Asp Ser Val Thr Ser Thr Phe Ser Lys Leu Arg Glu Gln Leu
       50                  55                  60
   Gly Pro Val Thr Gln Glu Phe Trp Asp Asn Leu Glu Lys Glu Thr Glu
   65                  70                  75                  80
   Gly Leu Cys Gln Glu Met Ser Lys Asp Leu Glu Glu Val Lys Ala Lys
           85                  90                  95
   Val Gln Pro Tyr Leu Asp Asp Phe Gln Lys Lys Trp Gln Glu Glu Met
               100                 105                 110
   Glu Leu Tyr Arg Gln Lys Val Glu Pro Leu Arg Ala Glu Leu Gln Glu
           115                 120                 125
   Gly Ala Arg Gln Lys Leu His Glu Leu Gln Glu Lys Leu Ser Pro Leu
       130                 135                 140
   Gly Glu Glu Met Arg Asp Arg Ala Arg Ala His Val Asp Ala Leu Arg
   145                 150                 155                 160
   Thr His Leu Ala Pro Tyr Ser Asp Glu Leu Arg Gln Arg Leu Ala Ala
                   165                 170                 175
   Arg Leu Glu Ala Leu Lys Glu Asn Gly Gly Ala Arg Leu Ala Glu Tyr
               180                 185                 190
   His Ala Lys Ala Thr Glu His Leu Ser Thr Leu Ser Glu Lys Ala Lys
           195                 200                 205
   Pro Ala Leu Glu Asp Leu Arg Gln Gly Leu Leu Pro Val Leu Glu Ser
       210                 215                 220
   Phe Lys Val Ser Phe Leu Ser Ala Leu Glu Glu Tyr Thr Lys Lys Leu
   225                 230                 235                 240
   Asn Thr Gln
<210> 4
   <211> 72
   <212> PRT
   <213> Homo sapiens
<220>
   <221> misc_feature
   <222> (12)..(12)
   <223> Xaa can be any naturally occurring amino acid
<400> 4
   Met Gly Cys Lys Arg Ala Ser Glu Val Cys Gly Xaa Ala Val Glu Gly
   1               5                   10                  15
   Leu Arg Asp Pro Leu Lys Pro Ser Glu Pro Ser Gln Gly Ala Ala Gly
               20                  25                  30
   Lys Arg Lys Gly Thr Glu Tyr Leu Met Lys Gln Lys Leu Glu Phe Gly
           35                  40                  45
   Gly Arg Gly Glu Glu Leu Leu Leu Gly Val His Leu Arg Gly Ala Gln
       50                  55                  60
   Lys Thr Gly Gly Gly Trp Arg Arg
   65                  70
<210> 5
   <211> 605
   <212> PRT
   <213> Homo sapiens
<400> 5
   Met Ala His Val Arg Gly Leu Gln His Val Phe Leu Ala Pro Gln Gln
   1               5                   10                  15
   Ala Arg Ser Leu Leu Gln Arg Val Arg Arg Ala Asn Thr Phe Leu Glu
               20                  25                  30
   Glu Val Arg Lys Gly Asn Leu Glu Arg Glu Cys Val Glu Glu Thr Cys
           35                  40                  45
   Ser Tyr Glu Glu Ala Phe Glu Ala Leu Glu Ser Ser Thr Ala Thr Asp
       50                  55                  60
   Val Phe Trp Ala Lys Tyr Thr Ala Cys Glu Thr Ala Arg Thr Pro Arg
   65                  70                  75                  80
   Asp Lys Leu Ala Ala Cys Leu Glu Gly Asn Cys Ala Glu Gly Leu Gly
                   85                  90                  95
   Thr Asn Tyr Arg Gly His Val Asn Ile Thr Arg Ser Gly Ile Glu Cys
               100                 105                 110
   Gln Leu Trp Arg Ser Arg Tyr Pro His Lys Pro Glu Ile Asn Ser Thr
           115                 120                 125
   Thr His Pro Gly Ala Asp Leu Gln Glu Asn Phe Cys Arg Asn Pro Asp
       130                 135                 140
   Ser Ser Thr Thr Gly Pro Trp Cys Tyr Thr Thr Asp Pro Thr Val Arg
   145                 150                 155                 160
   Arg Gln Glu Cys Ser Ile Pro Val Cys Gly Gln Asp Gln Val Thr Val
                   165                 170                 175
   Ala Met Thr Pro Arg Ser Glu Gly Ser Ser Val Asn Leu Ser Pro Pro
   180 185 190
   Leu Glu Gln Cys Val Pro Asp Arg Gly Gln Gln Tyr Gln Gly Arg Leu
   195 200 205
   Ala Val Thr Thr His Gly Leu Pro Cys Leu Ala Trp Ala Ser Ala Gln
   210 215 220
   Ala Lys Ala Leu Ser Lys His Gln Asp Phe Asn Ser Ala Val Gln Leu
   225 230 235 240
   Val Glu Asn Phe Cys Arg Asn Pro Asp Gly Asp Glu Glu Gly Val Trp
   245 250 255
   Cys Tyr Val Ala Gly Lys Pro Gly Asp Phe Gly Tyr Cys Asp Leu Asn
   260 265 270
   Tyr Cys Glu Glu Ala Val Glu Glu Glu Thr Gly Asp Gly Leu Asp Glu
   275 280 285
   Asp Ser Asp Arg Ala Ile Glu Gly Arg Thr Ala Thr Ser Glu Tyr Gln
   290 295 300
   Thr Phe Phe Asn Pro Arg Thr Phe Gly Ser Gly Glu Ala Asp Cys Gly
   305 310 315 320
   Leu Arg Pro Leu Phe Glu Lys Lys Ser Leu Glu Asp Lys Thr Glu Arg
   325 330 335
   Glu Leu Leu Glu Ser Tyr Ile Asp Gly Arg Ile Val Glu Gly Ser Asp
   340 345 350
   Ala Glu Ile Gly Met Ser Pro Trp Gln Val Met Leu Phe Arg Lys Ser
   355 360 365
   Pro Gln Glu Leu Leu Cys Gly Ala Ser Leu Ile Ser Asp Arg Trp Val
   370 375 380
   Leu Thr Ala Ala His Cys Leu Leu Tyr Pro Pro Trp Asp Lys Asn Phe
   385 390 395 400
   Thr Glu Asn Asp Leu Leu Val Arg Ile Gly Lys His Ser Arg Thr Arg
   405 410 415
   Tyr Glu Arg Asn Ile Glu Lys Ile Ser Met Leu Glu Lys Ile Tyr Ile
   420 425 430
   His Pro Arg Tyr Asn Trp Arg Glu Asn Leu Asp Arg Asp Ile Ala Leu
   435 440 445
   Met Lys Leu Lys Lys Pro Val Ala Phe Ser Asp Tyr Ile His Pro Val
   450 455 460
   Cys Leu Pro Asp Arg Glu Thr Ala Ala Ser Leu Leu Gln Ala Gly Tyr
   465 470 475 480
   Lys Gly Arg Val Thr Gly Trp Gly Asn Leu Lys Glu Thr Trp Thr Ala
   485 490 495
   Asn Val Gly Lys Gly Gln Pro Ser Val Leu Gln Val Val Asn Leu Pro
   500 505 510
   Ile Val Glu Arg Pro Val Cys Lys Asp Ser Thr Arg Ile Arg Ile Thr
   515 520 525
   Asp Asn Met Phe Cys Ala Gly Tyr Lys Pro Asp Glu Gly Lys Arg Gly
   530 535 540
   Asp Ala Cys Glu Gly Asp Ser Gly Gly Pro Phe Val Met Lys Ser Pro
   545 550 555 560
   Phe Asn Asn Arg Trp Tyr Gln Met Gly Ile Val Ser Trp Gly Glu Gly
   565 570 575
   Cys Asp Arg Asp Gly Lys Tyr Gly Phe Tyr Thr His Val Phe Arg Leu
   580 585 590
   Lys Lys Trp Ile Gln Lys Val Ile Asp Gln Phe Gly Glu
   595 600 605
<210> 6
   <211> 375
   <212> PRT
   <213> Homo sapiens
<400> 6
<210> 7
   <211> 595
   <212> PRT
   <213> Homo sapiens
<400> 7
<210> 8
   <211> 439
   <212> PRT
   <213> Homo sapiens
<400> 8
   Thr Pro Leu Pro Pro Thr Ser Ala His Gly Asn Val Ala Glu Gly Glu
   1               5                   10                  15
   Thr Lys Pro Asp Pro Asp Val Thr Glu Arg Cys Ser Asp Gly Trp Ser
               20                  25                  30
   Phe Asp Ala Thr Thr Leu Asp Asp Asn Gly Thr Met Leu Phe Phe Lys
           35                  40                  45
   Gly Glu Phe Val Trp Lys Ser His Lys Trp Asp Arg Glu Leu Ile Ser
       50                  55                  60
   Glu Arg Trp Lys Asn Phe Pro Ser Pro Val Asp Ala Ala Phe Arg Gln
   65                  70                  75                  80
   Gly His Asn Ser Val Phe Leu Ile Lys Gly Asp Lys Val Trp Val Tyr
                   85                  90                  95
   Pro Pro Glu Lys Lys Glu Lys Gly Tyr Pro Lys Leu Leu Gln Asp Glu
               100                 105                 110
   Phe Pro Gly Ile Pro Ser Pro Leu Asp Ala Ala Val Glu Cys His Arg
           115                 120                 125
   Gly Glu Cys Gln Ala Glu Gly Val Leu Phe Phe Gln Gly Asp Arg Glu
       130                 135                 140
   Trp Phe Trp Asp Leu Ala Thr Gly Thr Met Lys Glu Arg Ser Trp Pro
   145                 150                 155                 160
   Ala Val Gly Asn Cys Ser Ser Ala Leu Arg Trp Leu Gly Arg Tyr Tyr
                   165                 170                 175
   Cys Phe Gln Gly Asn Gln Phe Leu Arg Phe Asp Pro Val Arg Gly Glu
               180                 185                 190
   Val Pro Pro Arg Tyr Pro Arg Asp Val Arg Asp Tyr Phe Met Pro Cys
           195                 200                 205
   Pro Gly Arg Gly His Gly His Arg Asn Gly Thr Gly His Gly Asn Ser
       210                 215                 220
   Thr His His Gly Pro Glu Tyr Met Arg Cys Ser Pro His Leu Val Leu
   225                 230                 235                 240
   Ser Ala Leu Thr Ser Asp Asn His Gly Ala Thr Tyr Ala Phe Ser Gly
                   245                 250                 255
   Thr His Tyr Trp Arg Leu Asp Thr Ser Arg Asp Gly Trp His Ser Trp
               260                 265                 270
   Pro Ile Ala His Gln Trp Pro Gln Gly Pro Ser Ala Val Asp Ala Ala
           275                 280                 285
   Phe Ser Trp Glu Glu Lys Leu Tyr Leu Val Gln Gly Thr Gln Val Tyr
       290                 295                 300
   Val Phe Leu Thr Lys Gly Gly Tyr Thr Leu Val Ser Gly Tyr Pro Lys
   305                 310                 315                 320
   Arg Leu Glu Lys Glu Val Gly Thr Pro His Gly Ile Ile Leu Asp Ser
                   325                 330                 335
   Val Asp Ala Ala Phe Ile Cys Pro Gly Ser Ser Arg Leu His Ile Met
               340                 345                 350
   Ala Gly Arg Arg Leu Trp Trp Leu Asp Leu Lys Ser Gly Ala Gln Ala
           355                 360                 365
   Thr Trp Thr Glu Leu Pro Trp Pro His Glu Lys Val Asp Gly Ala Leu
       370                 375                 380
   Cys Met Glu Lys Ser Leu Gly Pro Asn Ser Cys Ser Ala Asn Gly Pro
   385                 390                 395                 400
   Gly Leu Tyr Leu Ile His Gly Pro Asn Leu Tyr Cys Tyr Ser Asp Val
                   405                 410                 415
   Glu Lys Leu Asn Ala Ala Lys Ala Leu Pro Gln Pro Gln Asn Val Thr
               420                 425                 430
   Ser Leu Leu Gly Cys Thr His
           435
<210> 9
   <211> 371
   <212> PRT
   <213> Homo sapiens
<400> 9
   Met Glu Pro Arg Ala Val Gly Val Ser Lys Gln Asp Ile Arg Glu Gln
   1               5                   10                  15
   Ile Trp Gly Tyr Met Glu Ser Gln Asn Leu Ala Asp Phe Pro Arg Pro
               20                  25                  30
   Val His His Arg Ile Pro Asn Phe Lys Gly Ser Tyr Leu Ala Cys Gln
           35                  40                  45
   Asn Ile Lys Asp Leu Asp Val Phe Ala Arg Ala Gln Glu Val Lys Val
       50                  55                  60
   Asp Pro Asp Lys Pro Leu Glu Gly Val Arg Leu Leu Val Leu Gln Ser
   65                  70                  75                  80
   Lys Lys Thr Leu Leu Val Pro Thr Pro Arg Leu Arg Thr Gly Leu Phe
                   85                  90                  95
   Asn Lys Ile Thr Pro Pro Pro Gly Ala Thr Lys Asp Ile Leu Arg Lys
               100                 105                 110
   Cys Ala Thr Ser Gln Gly Val Arg Asn Tyr Ser Val Pro Ile Gly Leu
           115                 120                 125
   Asp Ser Arg Val Leu Val Asp Leu Val Val Val Gly Ser Val Ala Ala
       130                 135                 140
   Ser Glu Lys Gly Trp Arg Ile Gly Lys Gly Glu Gly Tyr Ala Asp Leu
   145                 150                 155                 160
   Glu Tyr Ala Met Met Val Ser Met Gly Ala Val Ser Lys Glu Thr Pro
                   165                 170                 175
   Val Val Thr Ile Val His Asp Cys Gln Val Val Asp Ile Pro Glu Glu
               180                 185                 190
   Leu Val Glu Glu His Asp Ile Thr Val Asp Tyr Ile Leu Thr Pro Thr
           195                 200                 205
   Arg Val Ile Ala Thr Gly Cys Lys Arg Pro Lys Pro Met Gly Ile Thr
       210                 215                 220
   Trp Phe Lys Ile Ser Leu Glu Met Met Glu Lys Ile Pro Ile Leu Arg
   225                 230                 235                 240
   Ser Leu Arg Ala Arg Glu Gln Gln Ala Gly Lys Asp Val Thr Leu Gln
                   245                 250                 255
   Gly Glu His Gln His Leu Pro Glu Pro Gly Cys Gln Gln Thr Val Pro
               260                 265                 270
   Leu Ser Val Gly Arg Arg Pro Pro Asp Thr Pro Gly Pro Glu Thr Asn
           275                 280                 285
   Ser Met Glu Ala Ala Pro Gly Ser Pro Pro Gly Glu Gly Ala Pro Leu
   290 295 300
   Ala Ala Asp Val Tyr Val Gly Asn Leu Pro Arg Asp Ala Arg Val Ser
   305 310 315 320
   Asp Leu Lys Arg Ala Leu Arg Glu Leu Gly Ser Val Pro Leu Arg Leu
   325 330 335
   Thr Trp Gln Gly Pro Arg Arg Arg Ala Phe Leu His Tyr Pro Asp Ser
   340 345 350
   Ala Ala Ala Ser Arg Pro Ser Pro Ala Cys Arg Ala Cys Ala Trp Ala
   355 360 365
   Pro Thr Pro
   370
<210> 10
   <211> 142
   <212> PRT
   <213> Homo sapiens
<400> 10
<210> 11
   <211> 243
   <212> PRT
   <213> Homo sapiens
<400> 11
   Asp Glu Pro Pro Gln Ser Pro Trp Asp Arg Val Lys Asp Leu Ala Thr
   1               5                   10                  15
   Val Tyr Val Asp Val Leu Lys Asp Ser Gly Arg Asp Tyr Val Ser Gln
               20                  25                  30
   Phe Glu Gly Ser Ala Leu Gly Lys Gln Leu Asn Leu Lys Leu Leu Asp
           35                  40                  45
   Asn Trp Asp Ser Val Thr Ser Thr Phe Ser Lys Leu Arg Glu Gln Leu
       50                  55                  60
   Gly Pro Val Thr Gln Glu Phe Trp Asp Asn Leu Glu Lys Glu Thr Glu
   65                  70                  75                  80
   Gly Leu Cys Gln Glu Met Ser Lys Asp Leu Glu Glu Val Lys Ala Lys
                   85                  90                  95
   Val Gln Pro Tyr Leu Asp Asp Phe Gln Lys Lys Trp Gln Glu Glu Met
               100                 105                 110
   Glu Leu Tyr Arg Gln Lys Val Glu Pro Leu Arg Ala Glu Leu Gln Glu
           115                 120                 125
   Gly Ala Arg Gln Lys Leu His Glu Leu Gln Glu Lys Leu Ser Pro Leu
       130                 135                 140
   Gly Glu Glu Met Arg Asp Arg Ala Arg Ala His Val Asp Ala Leu Arg
   145                 150                 155                 160
   Thr His Leu Ala Pro Tyr Ser Asp Glu Leu Arg Gln Arg Leu Ala Ala
                   165                 170                 175
   Arg Leu Glu Ala Leu Lys Glu Asn Gly Gly Ala Arg Leu Ala Glu Tyr
               180                 185                 190
   His Ala Lys Ala Thr Glu His Leu Ser Thr Leu Ser Glu Lys Ala Lys
           195                 200                 205
   Pro Ala Leu Glu Asp Leu Arg Gln Gly Leu Leu Pro Val Leu Glu Ser
       210                 215                 220
   Phe Lys Val Ser Phe Leu Ser Ala Leu Glu Glu Tyr Thr Lys Lys Leu
   225                 230                 235                 240
   Asn Thr Gln
<210> 12
   <211> 622
   <212> PRT
   <213> Homo sapiens
<400> 12
<210> 13
   <211> 415
   <212> PRT
   <213> Homo sapiens
<400> 13
<210> 14
   <211> 427
   <212> PRT
   <213> Homo sapiens
<400> 14
<210> 15
   <211> 427
   <212> PRT
   <213> Homo sapiens
<400> 15
<210> 16
   <211> 427
   <212> PRT
   <213> Homo sapiens
<400> 16
<210> 17
   <211> 347
   <212> PRT
   <213> Homo sapiens
<400> 17
   Met Asp Asp Asp Ile Ala Ala Leu Val Val Asp Asn Gly Ser Gly Met
   1               5                   10                  15
   Cys Lys Ala Gly Phe Ala Gly Asp Asp Ala Pro Arg Ala Val Phe Pro
               20                  25                  30
   Ser Ile Val Gly Arg Pro Arg His Gln Gly Val Met Val Gly Ile Val
           35                  40                  45
   Thr Asn Trp Asp Asp Met Glu Lys Ile Trp His His Thr Phe Tyr Asn
       50                  55                  60
   Glu Leu Arg Val Ala Pro Glu Glu His Pro Val Leu Leu Thr Glu Ala
   65                  70                  75                  80
   Pro Leu Asn Pro Lys Ala Asn Arg Glu Lys Met Thr Gln Ile Met Phe
                   85                  90                  95
   Glu Thr Phe Asn Thr Pro Ala Met Tyr Val Ala Ile Gln Ala Val Leu
               100                 105                 110
   Ser Leu Tyr Ala Ser Gly Arg Thr Thr Gly Ile Val Met Asp Ser Gly
           115                 120                 125
   Asp Gly Val Thr His Thr Val Pro Ile Tyr Glu Gly Tyr Ala Leu Pro
       130                 135                 140
   His Ala Ile Leu Arg Leu Asp Leu Ala Gly Arg Asp Leu Thr Asp Tyr
   145                 150                 155                 160
   Leu Met Lys Ile Leu Thr Glu Arg Gly Tyr Ser Phe Thr Thr Thr Ala
                   165                 170                 175
   Glu Arg Glu Ile Val Arg Asp Ile Lys Glu Lys Leu Cys Tyr Val Ala
               180                 185                 190
   Leu Asp Phe Glu Gln Glu Met Ala Thr Ala Ala Ser Ser Ser Ser Leu
           195                 200                 205
   Glu Lys Ser Tyr Glu Leu Pro Asp Gly Gln Val Ile Thr Ile Gly Asn
           210                 215                 220
   Glu Arg Phe Arg Cys Pro Glu Ala Leu Phe Gln Pro Ser Phe Leu Gly
   225                 230                 235                 240
   Met Glu Ser Cys Gly Ile His Glu Thr Thr Phe Asn Ser Ile Met Lys
                   245                 250                 255
   Cys Asp Val Asp Ile Arg Lys Asp Leu Tyr Ala Asn Thr Val Leu Ser
               260                 265                 270
   Gly Gly Thr Thr Met Tyr Pro Gly Ile Ala Asp Arg Met Gln Lys Glu
           275                 280                 285
   Ile Thr Ala Leu Ala Pro Ser Thr Met Lys Ile Lys Ile Ile Ala Pro
       290                 295                 300
   Pro Glu Arg Lys Tyr Ser Val Trp Ile Gly Gly Ser Ile Leu Ala Ser
   305                 310                 315                 320
   Leu Ser Thr Phe Gln Gln Met Trp Ile Ser Lys Gln Glu Tyr Asp Glu
                   325                 330                 335
   Ser Gly Pro Ser Ile Val His Arg Lys Cys Phe
               340                 345
<210> 18
   <211> 356
   <212> PRT
   <213> Homo sapiens
<400> 18
   Met Glu Glu Glu Ile Ala Ala Leu Val Ile Asp Asn Gly Ser Gly Met
   1               5                   10                  15
   Cys Lys Ala Gly Phe Ala Gly Asp Asp Ala Pro Arg Ala Val Phe Pro
               20                  25                  30
   Ser Ile Val Gly Arg Pro Arg His Gln Gly Val Met Val Gly Met Gly
           35                  40                  45
   Gln Lys Asp Ser Tyr Val Gly Asp Glu Ala Gln Ser Lys Arg Gly Ile
       50                  55                  60
   Leu Thr Leu Lys Tyr Pro Ile Glu His Gly Ile Val Thr Asn Trp Asp
   65                  70                  75                  80
   Asp Met Glu Lys Ile Trp His His Thr Phe Tyr Asn Glu Leu Arg Val
                   85                  90                  95
   Ala Pro Glu Glu His Pro Val Leu Leu Thr Glu Ala Pro Leu Asn Pro
               100                 105                 110
   Lys Ala Asn Arg Glu Lys Met Thr Gln Ile Met Phe Glu Thr Phe Asn
           115                 120                 125
   Thr Thr Gly Ile Val Met Asp Ser Gly Asp Gly Val Thr His Thr Val
       130                 135                 140
   Pro Ile Tyr Glu Gly Tyr Ala Leu Pro His Ala Ile Leu Arg Leu Asp
   145                 150                 155                 160
   Leu Ala Gly Arg Asp Leu Thr Asp Tyr Leu Met Lys Ile Leu Thr Glu
                   165                 170                 175
   Arg Gly Tyr Ser Phe Thr Thr Thr Ala Glu Arg Glu Ile Val Arg Asp
               180                 185                 190
   Ile Lys Glu Lys Leu Cys Tyr Val Ala Leu Asp Phe Glu Gln Glu Met
           195                 200                 205
   Ala Thr Ala Ala Ser Ser Ser Ser Leu Glu Lys Ser Tyr Glu Leu Pro
       210                 215                 220
   Asp Gly Gln Val Ile Thr Ile Gly Asn Glu Arg Phe Arg Cys Pro Glu
   225                 230                 235                 240
   Ala Leu Phe Gln Pro Ser Phe Leu Gly Met Glu Ser Cys Gly Ile His
                   245                 250                 255
   Glu Thr Thr Phe Asn Ser Ile Met Lys Cys Asp Val Asp Ile Arg Lys
               260                 265                 270
   Asp Leu Tyr Ala Asn Thr Val Leu Ser Gly Gly Thr Thr Met Tyr Pro
           275                 280                 285
   Gly Ile Ala Asp Arg Met Gln Lys Glu Ile Thr Ala Leu Ala Pro Ser
       290                 295                 300
   Thr Met Lys Ile Lys Ile Ile Ala Pro Pro Glu Arg Lys Tyr Ser Val
   305                 310                 315                 320
   Trp Ile Gly Gly Ser Ile Leu Ala Ser Leu Ser Thr Phe Gln Gln Met
                   325                 330                 335
   Trp Ile Ser Lys Gln Glu Tyr Asp Glu Ser Gly Pro Ser Ile Val His
               340                 345                 350
   Arg Lys Cys Phe
           355
<210> 19
   <211> 248
   <212> PRT
   <213> Homo sapiens
<400> 19
   Met Gln Lys Ser Glu Gly Ser Gly Gly Thr Gln Leu Lys Asn Arg Ala
   1               5                   10                  15
   Thr Gly Asn Tyr Asp Gln Arg Thr Ser Ser Ser Thr Gln Leu Lys His
               20                  25                  30
   Arg Asn Ala Val Gln Gly Ser Lys Ser Ser Leu Ser Thr Ser Ser Pro
           35                  40                  45
   Glu Ser Ala Arg Lys Leu His Pro Arg Pro Ser Asp Lys Leu Asn Pro
       50                  55                  60
   Lys Thr Ile Asn Pro Phe Gly Glu Gln Ser Arg Val Pro Ser Ala Phe
   65                  70                  75                  80
   Ala Ala Ile Tyr Ser Lys Gly Gly Ile Pro Cys Arg Leu Val His Gly
                   85                  90                  95
   Ser Val Lys His Arg Leu Gln Trp Glu Cys Pro Pro Glu Ser Leu Ser
               100                 105                 110
   Phe Asp Pro Leu Leu Ile Thr Leu Ala Glu Gly Leu Arg Glu Thr Lys
           115                 120                 125
   His Pro Tyr Thr Phe Val Ser Lys Glu Gly Phe Arg Glu Leu Leu Leu
       130                 135                 140
   Val Lys Gly Ala Pro Glu Lys Ala Ile Pro Leu Leu Pro Arg Leu Ile
   145                 150                 155                 160
   Pro Val Leu Lys Ala Ala Leu Val His Ser Asp Asp Glu Val Phe Glu
                   165                 170                 175
   Arg Gly Leu Asn Ala Leu Val Gln Leu Ser Val Val Val Gly Pro Ser
               180                 185                 190
   Leu Asn Asp His Leu Lys His Leu Leu Thr Ser Leu Ser Lys Arg Leu
   195 200 205
   Met Asp Lys Lys Phe Lys Glu Pro Ile Thr Ser Ala Leu Gln Lys Leu
   210 215 220
   Glu Gln His Gly Gly Ser Gly Ser Leu Ser Ile Ile Lys Ser Lys Ile
   225 230 235 240
   Pro Thr Tyr Cys Ser Ile Cys Cys
   245
<210> 20
   <211> 215
   <212> PRT
   <213> Homo sapiens
<400> 20
   Met Ala Ser Glu Thr Glu Lys Thr His Ala Leu Leu Gln Thr Cys Ser
   1 5 10 15
   Thr Glu Ser Leu Ile Ser Ser Leu Gly Leu Gly Ala Phe Cys Leu Val
   20 25 30
   Ala Asp Arg Leu Leu Gln Phe Ser Thr Ile Gln Gln Asn Asp Trp Leu
   35 40 45
   Arg Ala Leu Ser Asp Asn Ala Val His Cys Val Ile Gly Met Trp Ser
   50 55 60
   Trp Ala Val Val Thr Gly Ile Lys Lys Lys Thr Asp Phe Gly Glu Ile
   65 70 75 80
   Ile Leu Ala Gly Phe Leu Ala Ser Val Ile Asp Val Asp His Phe Phe
   85 90 95
   Leu Ala Gly Ser Met Ser Leu Lys Ala Ala Leu Thr Leu Pro Arg Arg
   100 105 110
   Pro Phe Leu His Cys Ser Thr Val Ile Pro Val Val Val Leu Thr Leu
   115 120 125
   Lys Phe Thr Met His Leu Phe Lys Leu Lys Asp Ser Trp Cys Phe Leu
   130 135 140
   Pro Trp Met Leu Phe Ile Ser Trp Thr Ser His His Ile Arg Asp Gly
   145 150 155 160
   Ile Arg His Gly Leu Trp Ile Cys Pro Phe Gly Lys Thr Ser Pro Leu
                   165                 170                 175
   Pro Phe Trp Leu Tyr Val Ile Ile Thr Ser Ser Leu Pro His Ile Cys
               180                 185                 190
   Ser Phe Val Met Tyr Leu Thr Gly Thr Arg Gln Met Met Ser Ser Lys
           195                 200                 205
   His Gly Val Arg Ile Asp Val
       210                 215
<210> 21
   <211> 1337
   <212> PRT
   <213> Homo sapiens
<400> 21
   Met Asp Phe Thr Ala Gln Pro Lys Pro Ala Thr Ala Leu Cys Gly Val
   1               5                   10                  15
   Val Ser Ala Asp Gly Lys Ile Ala Tyr Pro Pro Gly Val Lys Glu Ile
               20                  25                  30
   Thr Asp Lys Ile Thr Thr Asp Glu Met Ile Lys Arg Leu Lys Met Val
           35                  40                  45
   Val Lys Thr Phe Met Asp Met Asp Gln Asp Ser Glu Asp Glu Lys Gln
       50                  55                  60
   Gln Tyr Leu Pro Leu Ala Leu His Leu Ala Ser Glu Phe Phe Leu Arg
   65                  70                  75                  80
   Asn Pro Asn Lys Asp Val Arg Leu Leu Val Ala Cys Cys Leu Ala Asp
                   85                  90                  95
   Ile Phe Arg Ile Tyr Ala Pro Glu Ala Pro Tyr Thr Ser His Asp Lys
               100                 105                 110
   Leu Lys Asp Ile Phe Leu Phe Ile Thr Arg Gln Leu Lys Gly Leu Glu
           115                 120                 125
   Asp Thr Lys Ser Pro Gln Phe Asn Arg Tyr Phe Tyr Leu Leu Glu Asn
       130                 135                 140
   Leu Ala Trp Val Lys Ser Tyr Asn Ile Cys Phe Glu Leu Glu Asp Cys
   145                 150                 155                 160
   Asn Glu Ile Phe Ile Gln Leu Phe Arg Thr Leu Phe Ser Val Ile Asn
                   165                 170                 175
   Asn Ser His Asn Lys Lys Val Gln Met His Met Leu Asp Leu Met Ser
               180                 185                 190
   Ser Ile Ile Met Glu Gly Asp Gly Val Thr Gln Glu Leu Leu Asp Ser
           195                 200                 205
   Ile Leu Ile Asn Leu Ile Pro Ala His Lys Asn Leu Asn Lys Gln Ser
       210                 215                 220
   Phe Asp Leu Ala Lys Val Leu Leu Lys Arg Thr Val Gln Thr Ile Glu
   225                 230                 235                 240
   Ala Cys Ile Ala Asn Phe Phe Asn Gln Val Leu Val Leu Gly Arg Ser
                   245                 250                 255
   Ser Val Ser Asp Leu Ser Glu His Val Phe Asp Leu Ile Gln Glu Leu
               260                 265                 270
   Phe Ala Ile Asp Pro His Leu Leu Leu Ser Val Met Pro Gln Leu Glu
           275                 280                 285
   Phe Lys Leu Lys Ser Asn Asp Gly Glu Glu Arg Leu Ala Val Val Arg
       290                 295                 300
   Leu Leu Ala Lys Leu Phe Gly Ser Lys Asp Ser Asp Leu Ala Thr Gln
   305                 310                 315                 320
   Asn Arg Pro Leu Trp Gln Cys Phe Leu Gly Arg Phe Asn Asp Ile His
                   325                 330                 335
   Val Pro Val Arg Leu Glu Ser Val Lys Phe Ala Ser His Cys Leu Met
               340                 345                 350
   Asn His Pro Asp Leu Ala Lys Asp Leu Thr Glu Tyr Leu Lys Val Arg
           355                 360                 365
   Ser His Asp Pro Glu Glu Ala Ile Arg His Asp Val Ile Val Thr Ile
       370                 375                 380
   Ile Thr Ala Ala Lys Arg Asp Leu Ala Leu Val Asn Asp Gln Leu Leu
   385                 390                 395                 400
   Gly Phe Val Arg Glu Arg Thr Leu Asp Lys Arg Trp Arg Val Arg Lys
                   405                 410                 415
   Glu Ala Met Met Gly Leu Ala Gln Leu Tyr Lys Lys Tyr Cys Leu His
               420                 425                 430
   Gly Glu Ala Gly Lys Glu Ala Ala Glu Lys Val Ser Trp Ile Lys Asp
           435                 440                 445
   Lys Leu Leu His Ile Tyr Tyr Gln Asn Ser Ile Asp Asp Lys Leu Leu
       450                 455                 460
   Val Glu Lys Ile Phe Ala Gln Tyr Leu Val Pro His Asn Leu Glu Thr
   465                 470                 475                 480
   Glu Glu Arg Met Lys Cys Leu Tyr Tyr Leu Tyr Ala Ser Leu Asp Pro
                   485                 490                 495
   Asn Ala Val Lys Ala Leu Asn Glu Met Trp Lys Cys Gln Asn Met Leu
               500                 505                 510
   Arg Ser His Val Arg Glu Leu Leu Asp Leu His Lys Gln Pro Thr Ser
           515                 520                 525
   Glu Ala Asn Cys Ser Ala Met Phe Gly Lys Leu Met Thr Ile Ala Lys
       530                 535                 540
   Asn Leu Pro Asp Pro Gly Lys Ala Gln Asp Phe Val Lys Lys Phe Asn
   545                 550                 555                 560
   Gln Val Leu Gly Asp Asp Glu Lys Leu Arg Ser Gln Leu Glu Leu Leu
                   565                 570                 575
   Ile Ser Pro Thr Cys Ser Cys Lys Gln Ala Asp Ile Cys Val Arg Glu
               580                 585                 590
   Ile Ala Arg Lys Leu Ala Asn Pro Lys Gln Pro Thr Asn Pro Phe Leu
           595                 600                 605
   Glu Met Val Lys Phe Leu Leu Glu Arg Ile Ala Pro Val His Ile Asp
       610                 615                 620
   Ser Glu Ala Ile Ser Ala Leu Val Lys Leu Met Asn Lys Ser Ile Glu
   625                 630                 635                 640
   Gly Thr Ala Asp Asp Glu Glu Glu Gly Val Ser Pro Asp Thr Ala Ile
                   645                 650                 655
   Arg Ser Gly Leu Glu Leu Leu Lys Val Leu Ser Phe Thr His Pro Thr
               660                 665                 670
   Ser Phe His Ser Ala Glu Thr Tyr Glu Ser Leu Leu Gln Cys Leu Arg
   675 680 685
   Met Glu Asp Asp Lys Val Ala Glu Ala Ala Ile Gln Ile Phe Arg Asn
   690 695 700
   Thr Gly His Lys Ile Glu Thr Asp Leu Pro Gln Ile Arg Ser Thr Leu
   705 710 715 720
   Ile Pro Ile Leu His Gln Lys Ala Lys Arg Gly Thr Pro His Gln Ala
   725 730 735
   Lys Gln Ala Val His Cys Ile His Ala Ile Phe Thr Asn Lys Glu Val
   740 745 750
   Gln Leu Ala Gln Ile Phe Glu Pro Leu Ser Arg Ser Leu Asn Ala Asp
   755 760 765
   Val Pro Glu Gln Leu Ile Thr Pro Leu Val Ser Leu Gly His Ile Ser
   770 775 780
   Met Leu Ala Pro Asp Gln Phe Ala Ser Pro Met Lys Ser Val Val Ala
   785 790 795 800
   Asn Phe Ile Val Lys Asp Leu Leu Met Asn Asp Arg Ser Thr Gly Glu
   805 810 815
   Lys Asn Gly Lys Leu Trp Ser Pro Asp Glu Glu Val Ser Pro Glu Val
   820 825 830
   Leu Ala Lys Val Gln Ala Ile Lys Leu Leu Val Arg Trp Leu Leu Gly
   835 840 845
   Met Lys Asn Asn Gln Ser Lys Ser Ala Asn Ser Thr Leu Arg Leu Leu
   850 855 860
   Ser Ala Met Leu Val Ser Glu Gly Asp Leu Thr Glu Gln Lys Arg Ile
   865 870 875 880
   Ser Lys Ser Asp Met Ser Arg Leu Arg Leu Ala Ala Gly Ser Ala Ile
   885 890 895
   Met Lys Leu Ala Gln Glu Pro Cys Tyr His Glu Ile Ile Thr Pro Glu
   900 905 910
   Gln Phe Gln Leu Cys Ala Leu Val Ile Asn Asp Glu Cys Tyr Gln Val
   915 920 925
   Arg Gln Ile Phe Ala Gln Lys Leu His Lys Ala Leu Val Lys Leu Leu
   930 935 940
   Leu Pro Leu Glu Tyr Met Ala Ile Phe Ala Leu Cys Ala Lys Asp Pro
   945 950 955 960
   Val Lys Glu Arg Arg Ala His Ala Arg Gln Cys Leu Leu Lys Asn Ile
   965 970 975
   Ser Ile Arg Arg Glu Tyr Ile Lys Gln Asn Pro Met Ala Thr Glu Lys
   980 985 990
   Leu Leu Ser Leu Leu Pro Glu Tyr Val Val Pro Tyr Met Ile His Leu
   995 1000 1005
   Leu Ala His Asp Pro Asp Phe Thr Arg Ser Gln Asp Val Asp Gln
   1010 1015 1020
   Leu Arg Asp Ile Lys Glu Cys Leu Trp Phe Met Leu Glu Val Leu
   1025 1030 1035
   Met Thr Lys Asn Glu Asn Asn Ser His Ala Phe Met Lys Lys Met
   1040 1045 1050
   Ala Glu Asn Ile Lys Leu Thr Arg Asp Ala Gln Ser Pro Asp Glu
   1055 1060 1065
   Ser Lys Thr Asn Glu Lys Leu Tyr Thr Val Cys Asp Val Ala Leu
   1070 1075 1080
   Cys Val Ile Asn Ser Lys Ser Ala Leu Cys Asn Ala Asp Ser Pro
   1085 1090 1095
   Lys Asp Pro Val Leu Pro Met Lys Phe Phe Thr Gln Pro Glu Lys
   1100 1105 1110
   Asp Phe Cys Asn Asp Lys Ser Tyr Ile Ser Glu Glu Thr Arg Val
   1115 1120 1125
   Leu Leu Leu Thr Gly Lys Pro Lys Pro Ala Gly Val Leu Gly Ala
   1130 1135 1140
   Val Asn Lys Pro Leu Ser Ala Thr Gly Arg Lys Pro Tyr Val Arg
   1145 1150 1155
   Ser Thr Gly Thr Glu Thr Gly Ser Asn Ile Asn Val Asn Ser Glu
       1160                1165                1170
   Leu Asn Pro Ser Thr Gly Asn Arg Ser Arg Glu Gln Ser Ser Glu
       1175                1180                1185
   Ala Ala Glu Thr Gly Val Ser Glu Asn Glu Glu Asn Pro Val Arg
       1190                1195                1200
   Ile Ile Ser Val Thr Pro Val Lys Asn Ile Asp Pro Val Lys Asn
       1205                1210                1215
   Lys Glu Ile Asn Ser Asp Gln Ala Thr Gln Gly Asn Ile Ser Ser
       1220                1225                1230
   Asp Arg Gly Lys Lys Arg Thr Val Thr Ala Ala Gly Ala Glu Asn
       1235                1240                1245
   Ile Gln Gln Lys Thr Asp Glu Lys Val Asp Glu Ser Gly Pro Pro
       1250                1255                1260
   Ala Pro Ser Lys Pro Arg Arg Gly Arg Arg Pro Lys Ser Glu Ser
       1265                1270                1275
   Gln Gly Asn Ala Thr Lys Asn Asp Asp Leu Asn Lys Pro Ile Asn
       1280                1285                1290
   Lys Gly Arg Lys Arg Ala Ala Val Gly Gln Glu Ser Pro Gly Gly
       1295                1300                1305
   Leu Glu Ala Gly Asn Ala Lys Ala Pro Lys Leu Gln Asp Leu Ala
       1310                1315                1320
   Lys Lys Ala Ala Pro Ala Glu Arg Gln Ile Asp Leu Gln Arg
       1325                1330                1335
<210> 22
   <211> 634
   <212> PRT
   <213> Homo sapiens
<400> 22
   Met Ala Glu Glu Gln Glu Phe Thr Gln Leu Cys Lys Leu Pro Ala Gln
   1               5                   10                  15
   Pro Ser His Pro His Cys Val Asn Asn Thr Tyr Arg Ser Ala Gln His
               20                  25                  30
   Ser Gln Ala Leu Leu Arg Gly Leu Leu Ala Leu Arg Asp Ser Gly Ile
           35                  40                  45
   Leu Phe Asp Val Val Leu Val Val Glu Gly Arg His Ile Glu Ala His
       50                  55                  60
   Arg Ile Leu Leu Ala Ala Ser Cys Asp Tyr Phe Arg Gly Met Phe Ala
   65                  70                  75                  80
   Gly Gly Leu Lys Glu Met Glu Gln Glu Glu Val Leu Ile His Gly Val
                   85                  90                  95
   Ser Tyr Asn Ala Met Cys Gln Ile Leu His Phe Ile Tyr Thr Ser Glu
               100                 105                 110
   Leu Glu Leu Ser Leu Ser Asn Val Gln Glu Thr Leu Val Ala Ala Cys
           115                 120                 125
   Gln Leu Gln Ile Pro Glu Ile Ile His Phe Cys Cys Asp Phe Leu Met
       130                 135                 140
   Ser Trp Val Asp Glu Glu Asn Ile Leu Asp Val Tyr Arg Leu Ala Glu
   145                 150                 155                 160
   Leu Phe Asp Leu Ser Arg Leu Thr Glu Gln Leu Asp Thr Tyr Ile Leu
                   165                 170                 175
   Lys Asn Phe Val Ala Phe Ser Arg Thr Asp Lys Tyr Arg Gln Leu Pro
               180                 185                 190
   Leu Glu Lys Val Tyr Ser Leu Leu Ser Ser Asn Arg Leu Glu Val Ser
           195                 200                 205
   Cys Glu Thr Glu Val Tyr Glu Gly Ala Leu Leu Tyr His Tyr Ser Leu
       210                 215                 220
   Glu Gln Val Gln Ala Asp Gln Ile Ser Leu His Glu Pro Pro Lys Leu
   225                 230                 235                 240
   Leu Glu Thr Val Arg Phe Pro Leu Met Glu Ala Glu Val Leu Gln Arg
                   245                 250                 255
   Leu His Asp Lys Leu Asp Pro Ser Pro Leu Arg Asp Thr Val Ala Ser
               260                 265                 270
   Ala Leu Met Tyr His Arg Asn Glu Ser Leu Gln Pro Ser Leu Gln Ser
           275                 280                 285
   Pro Gln Thr Glu Leu Arg Ser Asp Phe Gln Cys Val Val Gly Phe Gly
       290                 295                 300
   Gly Ile His Ser Thr Pro Ser Thr Val Leu Ser Asp Gln Ala Lys Tyr
   305                 310                 315                 320
   Leu Asn Pro Leu Leu Gly Glu Trp Lys His Phe Thr Ala Ser Leu Ala
                   325                 330                 335
   Pro Arg Met Ser Asn Gln Gly Ile Ala Val Leu Asn Asn Phe Val Tyr
               340                 345                 350
   Leu Ile Gly Gly Asp Asn Asn Val Gln Gly Phe Arg Ala Glu Ser Arg
           355                 360                 365
   Cys Trp Arg Tyr Asp Pro Arg His Asn Arg Trp Phe Gln Ile Gln Ser
       370                 375                 380
   Leu Gln Gln Glu His Ala Asp Leu Ser Val Cys Val Val Gly Arg Tyr
   385                 390                 395                 400
   Ile Tyr Ala Val Ala Gly Arg Asp Tyr His Asn Asp Leu Asn Ala Val
                   405                 410                 415
   Glu Arg Tyr Asp Pro Ala Thr Asn Ser Trp Ala Tyr Val Ala Pro Leu
               420                 425                 430
   Lys Arg Glu Val Tyr Ala His Ala Gly Ala Thr Leu Glu Gly Lys Met
           435                 440                 445
   Tyr Ile Thr Cys Gly Arg Arg Gly Glu Asp Tyr Leu Lys Glu Thr His
       450                 455                 460
   Cys Tyr Asp Pro Gly Ser Asn Thr Trp His Thr Leu Ala Asp Gly Pro
   465                 470                 475                 480
   Val Arg Arg Ala Trp His Gly Met Ala Thr Leu Leu Asn Lys Leu Tyr
                   485                 490                 495
   Val Ile Gly Gly Ser Asn Asn Asp Ala Gly Tyr Arg Arg Asp Val His
               500                 505                 510
   Gln Val Ala Cys Tyr Ser Cys Thr Ser Gly Gln Trp Ser Ser Val Cys
           515                 520                 525
   Pro Leu Pro Ala Gly His Gly Glu Pro Gly Ile Ala Val Leu Asp Asn
       530                 535                 540
   Arg Ile Tyr Val Leu Gly Gly Arg Ser His Asn Arg Gly Ser Arg Thr
   545 550 555 560
   Gly Tyr Val His Ile Tyr Asp Val Glu Lys Asp Cys Trp Glu Glu Gly
   565 570 575
   Pro Gln Leu Asp Asn Ser Ile Ser Gly Leu Ala Ala Cys Val Leu Thr
   580 585 590
   Leu Pro Arg Ser Leu Leu Leu Glu Pro Pro Arg Gly Thr Pro Asp Arg
   595 600 605
   Ser Gln Ala Asp Pro Asp Phe Ala Ser Glu Val Met Ser Val Ser Asp
   610 615 620
   Trp Glu Glu Phe Asp Asn Ser Ser Glu Asp
   625 630
<210> 23
   <211> 2268
   <212> PRT
   <213> Homo sapiens
<400> 23
   Met Lys Gln Leu Gln Pro Gln Pro Pro Pro Lys Met Gly Asp Phe Tyr
   1 5 10 15
   Asp Pro Glu His Pro Thr Pro Glu Glu Glu Glu Asn Glu Ala Lys Ile
   20 25 30
   Glu Asn Val Gln Lys Thr Gly Phe Ile Lys Gly Pro Met Phe Lys Gly
   35 40 45
   Val Ala Ser Ser Arg Phe Leu Pro Lys Gly Thr Lys Thr Lys Val Asn
   50 55 60
   Leu Glu Glu Gln Gly Arg Gln Lys Val Ser Phe Ser Phe Ser Leu Thr
   65 70 75 80
   Lys Lys Thr Leu Gln Asn Arg Phe Leu Thr Ala Leu Gly Asn Glu Lys
   85 90 95
   Gln Ser Asp Thr Pro Asn Pro Pro Ala Val Pro Leu Gln Val Asp Ser
   100 105 110
   Thr Pro Lys Met Lys Met Glu Ile Gly Asp Thr Leu Ser Thr Ala Glu
   115 120 125
   Glu Ser Ser Pro Pro Lys Ser Arg Val Glu Leu Gly Lys Ile His Phe
       130                 135                 140
   Lys Lys His Leu Leu His Val Thr Ser Arg Pro Leu Leu Ala Thr Thr
   145                 150                 155                 160
   Thr Ala Val Ala Ser Pro Pro Thr His Ala Ala Pro Leu Pro Ala Val
                   165                 170                 175
   Ile Ala Glu Ser Thr Thr Val Asp Ser Pro Pro Ser Ser Pro Pro Pro
               180                 185                 190
   Pro Pro Pro Pro Ala Gln Ala Thr Thr Leu Ser Ser Pro Ala Pro Val
           195                 200                 205
   Thr Glu Pro Val Ala Leu Pro His Thr Pro Ile Thr Val Leu Met Ala
       210                 215                 220
   Ala Pro Val Pro Leu Pro Val Asp Val Ala Val Arg Ser Leu Lys Glu
   225                 230                 235                 240
   Pro Pro Ile Ile Ile Val Pro Glu Ser Leu Glu Ala Asp Thr Lys Gln
                   245                 250                 255
   Asp Thr Ile Ser Asn Ser Leu Glu Glu His Val Thr Gln Ile Leu Asn
               260                 265                 270
   Glu Gln Ala Asp Ile Ser Ser Lys Lys Glu Asp Ser His Ile Gly Lys
           275                 280                 285
   Asp Glu Glu Ile Pro Asp Ser Ser Lys Ile Ser Leu Ser Cys Lys Lys
       290                 295                 300
   Thr Gly Ser Lys Lys Lys Ser Ser Gln Ser Glu Gly Ile Phe Leu Gly
   305                 310                 315                 320
   Ser Glu Ser Asp Glu Asp Ser Val Arg Thr Ser Ser Ser Gln Arg Ser
                   325                 330                 335
   His Asp Leu Lys Phe Ser Ala Ser Ile Glu Lys Glu Arg Asp Phe Lys
               340                 345                 350
   Lys Ser Ser Ala Pro Leu Lys Ser Glu Asp Leu Gly Lys Pro Ser Arg
           355                 360                 365
   Ser Lys Thr Asp Arg Asp Asp Lys Tyr Phe Ser Tyr Ser Lys Leu Glu
   370 375 380
   Arg Asp Thr Arg Tyr Val Ser Ser Arg Cys Arg Ser Glu Arg Glu Arg
   385 390 395 400
   Arg Arg Ser Arg Ser His Ser Arg Ser Glu Arg Gly Ser Arg Thr Asn
   405 410 415
   Leu Ser Tyr Ser Arg Ser Glu Arg Ser His Tyr Tyr Asp Ser Asp Arg
   420 425 430
   Arg Tyr His Arg Ser Ser Pro Tyr Arg Glu Arg Thr Arg Tyr Ser Arg
   435 440 445
   Pro Tyr Thr Asp Asn Arg Ala Arg Glu Ser Ser Asp Ser Glu Glu Glu
   450 455 460
   Tyr Lys Lys Thr Tyr Ser Arg Arg Thr Ser Ser His Ser Ser Ser Tyr
   465 470 475 480
   Arg Asp Leu Arg Thr Ser Ser Tyr Ser Lys Ser Asp Arg Asp Cys Lys
   485 490 495
   Thr Glu Thr Ser Tyr Leu Glu Met Glu Arg Arg Gly Lys Tyr Ser Ser
   500 505 510
   Lys Leu Glu Arg Glu Ser Lys Arg Thr Ser Glu Asn Glu Ala Ile Lys
   515 520 525
   Arg Cys Cys Ser Pro Pro Asn Glu Leu Gly Phe Arg Arg Gly Ser Ser
   530 535 540
   Tyr Ser Lys His Asp Ser Ser Ala Ser Arg Tyr Lys Ser Thr Leu Ser
   545 550 555 560
   Lys Pro Ile Pro Lys Ser Asp Lys Phe Lys Asn Ser Phe Cys Cys Thr
   565 570 575
   Glu Leu Asn Glu Glu Ile Lys Gln Ser His Ser Phe Ser Leu Gln Thr
   580 585 590
   Pro Cys Ser Lys Gly Ser Glu Leu Arg Met Ile Asn Lys Asn Pro Glu
   595 600 605
   Arg Glu Lys Ala Gly Ser Pro Ala Pro Ser Asn Arg Leu Asn Asp Ser
   610 615 620
   Pro Thr Leu Lys Lys Leu Asp Glu Leu Pro Ile Phe Lys Ser Glu Phe
   625                 630                 635                 640
   Ile Thr His Asp Ser His Asp Ser Ile Lys Glu Leu Asp Ser Leu Ser
                   645                 650                 655
   Lys Val Lys Asn Asp Gln Leu Arg Ser Phe Cys Pro Ile Glu Leu Asn
               660                 665                 670
   Ile Asn Gly Ser Pro Gly Ala Glu Ser Asp Leu Ala Thr Phe Cys Thr
           675                 680                 685
   Ser Lys Thr Asp Ala Val Leu Met Thr Ser Asp Asp Ser Val Thr Gly
       690                 695                 700
   Ser Glu Leu Ser Pro Leu Val Lys Ala Cys Met Leu Ser Ser Asn Gly
   705                 710                 715                 720
   Phe Gln Asn Ile Ser Arg Cys Lys Glu Lys Asp Leu Asp Asp Thr Cys
                   725                 730                 735
   Met Leu His Lys Lys Ser Glu Ser Pro Phe Arg Glu Thr Glu Pro Leu
               740                 745                 750
   Val Ser Pro His Gln Asp Lys Leu Met Ser Met Pro Val Met Thr Val
           755                 760                 765
   Asp Tyr Ser Lys Thr Val Val Lys Glu Pro Val Asp Thr Arg Val Ser
       770                 775                 780
   Cys Cys Lys Thr Lys Asp Ser Asp Ile Tyr Cys Thr Leu Asn Asp Ser
   785                 790                 795                 800
   Asn Pro Ser Leu Cys Asn Ser Glu Ala Glu Asn Ile Glu Pro Ser Val
                   805                 810                 815
   Met Lys Ile Ser Ser Asn Ser Phe Met Asn Val His Leu Glu Ser Lys
               820                 825                 830
   Pro Val Ile Cys Asp Ser Arg Asn Leu Thr Asp His Ser Lys Phe Ala
           835                 840                 845
   Cys Glu Glu Tyr Lys Gln Ser Ile Gly Ser Thr Ser Ser Ala Ser Val
       850                 855                 860
   Asn His Phe Asp Asp Leu Tyr Gln Pro Ile Gly Ser Ser Gly Ile Ala
   865                 870                 875                 880
   Ser Ser Leu Gln Ser Leu Pro Pro Gly Ile Lys Val Asp Ser Leu Thr
   885 890 895
   Leu Leu Lys Cys Gly Glu Asn Thr Ser Pro Val Leu Asp Ala Val Leu
   900 905 910
   Lys Ser Lys Lys Ser Ser Glu Phe Leu Lys His Ala Gly Lys Glu Thr
   915 920 925
   Ile Val Glu Val Gly Ser Asp Leu Pro Asp Ser Gly Lys Gly Phe Ala
   930 935 940
   Ser Arg Glu Asn Arg Arg Asn Asn Gly Leu Ser Gly Lys Cys Leu Gln
   945 950 955 960
   Glu Ala Gln Glu Glu Gly Asn Ser Ile Leu Pro Glu Arg Arg Gly Arg
   965 970 975
   Pro Glu Ile Ser Leu Asp Glu Arg Gly Glu Gly Gly His Val His Thr
   980 985 990
   Ser Asp Asp Ser Glu Val Val Phe Ser Ser Cys Asp Leu Asn Leu Thr
   995 1000 1005
   Met Glu Asp Ser Asp Gly Val Thr Tyr Ala Leu Lys Cys Asp Ser
   1010 1015 1020
   Ser Gly His Ala Pro Glu Ile Val Ser Thr Val His Glu Asp Tyr
   1025 1030 1035
   Ser Gly Ser Ser Glu Ser Ser Asn Asp Glu Ser Asp Ser Glu Asp
   1040 1045 1050
   Thr Asp Ser Asp Asp Ser Ser Ile Pro Arg Asn Arg Leu Gln Ser
   1055 1060 1065
   Val Val Val Val Pro Lys Asn Ser Thr Leu Pro Met Glu Glu Thr
   1070 1075 1080
   Ser Pro Cys Ser Ser Arg Ser Ser Gln Ser Tyr Arg His Tyr Ser
   1085 1090 1095
   Asp His Trp Glu Asp Glu Arg Leu Glu Ser Arg Arg His Leu Tyr
   1100 1105 1110
   Glu Glu Lys Phe Glu Ser Ile Ala Ser Lys Ala Cys Pro Gln Thr
   1115 1120 1125
   Asp Lys Phe Phe Leu His Lys Gly Thr Glu Lys Asn Pro Glu Ile
   1130 1135 1140
   Ser Phe Thr Gln Ser Ser Arg Lys Gln Ile Asp Asn Arg Leu Pro
   1145 1150 1155
   Glu Leu Ser His Pro Gln Ser Asp Gly Val Asp Ser Thr Ser His
   1160 1165 1170
   Thr Asp Val Lys Ser Asp Pro Leu Gly His Pro Asn Ser Glu Glu
   1175 1180 1185
   Thr Val Lys Ala Lys Ile Pro Ser Arg Gln Gln Glu Glu Leu Pro
   1190 1195 1200
   Ile Tyr Ser Ser Asp Phe Glu Asp Val Pro Asn Lys Ser Trp Gln
   1205 1210 1215
   Gln Thr Thr Phe Gln Asn Arg Pro Asp Ser Arg Leu Gly Lys Thr
   1220 1225 1230
   Glu Leu Ser Phe Ser Ser Ser Cys Glu Ile Pro His Val Asp Gly
   1235 1240 1245
   Leu His Ser Ser Glu Glu Leu Arg Asn Leu Gly Trp Asp Phe Ser
   1250 1255 1260
   Gln Glu Lys Pro Ser Thr Thr Tyr Gln Gln Pro Asp Ser Ser Tyr
   1265 1270 1275
   Gly Ala Cys Gly Gly His Lys Tyr Gln Gln Asn Ala Glu Gln Tyr
   1280 1285 1290
   Gly Gly Thr Arg Asp Tyr Trp Gln Gly Asn Gly Tyr Trp Asp Pro
   1295 1300 1305
   Arg Ser Gly Arg Pro Pro Gly Thr Gly Val Val Tyr Asp Arg Thr
   1310 1315 1320
   Gln Gly Gln Val Pro Asp Ser Leu Thr Asp Asp Arg Glu Glu Glu
   1325 1330 1335
   Glu Asn Trp Asp Gln Gln Asp Gly Ser His Phe Ser Asp Gln Ser
   1340 1345 1350
   Asp Lys Phe Leu Leu Ser Leu Gln Lys Asp Lys Gly Ser Val Gln
   1355 1360 1365
   Ala Pro Glu Ile Ser Ser Asn Ser Ile Lys Asp Thr Leu Ala Val
   1370 1375 1380
   Asn Glu Lys Lys Asp Phe Ser Lys Asn Leu Glu Lys Asn Asp Ile
   1385 1390 1395
   Lys Asp Arg Gly Pro Leu Lys Lys Arg Arg Gln Glu Ile Glu Ser
   1400 1405 1410
   Asp Ser Glu Ser Asp Gly Glu Leu Gln Asp Arg Lys Lys Val Arg
   1415 1420 1425
   Val Glu Val Glu Gln Gly Glu Thr Ser Val Pro Pro Gly Ser Ala
   1430 1435 1440
   Leu Val Gly Pro Ser Cys Val Met Asp Asp Phe Arg Asp Pro Gln
   1445 1450 1455
   Arg Trp Lys Glu Cys Ala Lys Gln Gly Lys Met Pro Cys Tyr Phe
   1460 1465 1470
   Asp Leu Ile Glu Glu Asn Val Tyr Leu Thr Glu Arg Lys Lys Asn
   1475 1480 1485
   Lys Ser His Arg Asp Ile Lys Arg Met Gln Cys Glu Cys Thr Pro
   1490 1495 1500
   Leu Ser Lys Asp Glu Arg Ala Gln Gly Glu Ile Ala Cys Gly Glu
   1505 1510 1515
   Asp Cys Leu Asn Arg Leu Leu Met Ile Glu Cys Ser Ser Arg Cys
   1520 1525 1530
   Pro Asn Gly Asp Tyr Cys Ser Asn Arg Arg Phe Gln Arg Lys Gln
   1535 1540 1545
   His Ala Asp Val Glu Val Ile Leu Thr Glu Lys Lys Gly Trp Gly
   1550 1555 1560
   Leu Arg Ala Ala Lys Asp Leu Pro Ser Asn Thr Phe Val Leu Glu
   1565 1570 1575
   Tyr Cys Gly Glu Val Leu Asp His Lys Glu Phe Lys Ala Arg Val
   1580 1585 1590
   Lys Glu Tyr Ala Arg Asn Lys Asn Ile His Tyr Tyr Phe Met Ala
   1595 1600 1605
   Leu Lys Asn Asp Glu Ile Ile Asp Ala Thr Gln Lys Gly Asn Cys
   1610 1615 1620
   Ser Arg Phe Met Asn His Ser Cys Glu Pro Asn Cys Glu Thr Gln
   1625 1630 1635
   Lys Trp Thr Val Asn Gly Gln Leu Arg Val Gly Phe Phe Thr Thr
   1640 1645 1650
   Lys Leu Val Pro Ser Gly Ser Glu Leu Thr Phe Asp Tyr Gln Phe
   1655 1660 1665
   Gln Arg Tyr Gly Lys Glu Ala Gln Lys Cys Phe Cys Gly Ser Ala
   1670 1675 1680
   Asn Cys Arg Gly Tyr Leu Gly Gly Glu Asn Arg Val Ser Ile Arg
   1685 1690 1695
   Ala Ala Gly Gly Lys Met Lys Lys Glu Arg Ser Arg Lys Lys Asp
   1700 1705 1710
   Ser Val Asp Gly Glu Leu Glu Ala Leu Met Glu Asn Gly Glu Gly
   1715 1720 1725
   Leu Ser Asp Lys Asn Gln Val Leu Ser Leu Ser Arg Leu Met Val
   1730 1735 1740
   Arg Ile Glu Thr Leu Glu Gln Lys Leu Thr Cys Leu Glu Leu Ile
   1745 1750 1755
   Gln Asn Thr His Ser Gln Ser Cys Leu Lys Ser Phe Leu Glu Arg
   1760 1765 1770
   His Gly Leu Ser Leu Leu Trp Ile Trp Met Ala Glu Leu Gly Asp
   1775 1780 1785
   Gly Arg Glu Ser Asn Gln Lys Leu Gln Glu Glu Ile Ile Lys Thr
   1790 1795 1800
   Leu Glu His Leu Pro Ile Pro Thr Lys Asn Met Leu Glu Glu Ser
   1805 1810 1815
   Lys Val Leu Pro Ile Ile Gln Arg Trp Ser Gln Thr Lys Thr Ala
   1820 1825 1830
   Val Pro  Pro Leu Ser Glu Gly  Asp Gly Tyr Ser Ser  Glu Asn Thr
       1835                 1840                 1845
   Ser Arg  Ala His Thr Pro Leu  Asn Thr Pro Asp Pro  Ser Thr Lys
       1850                 1855                 1860
   Leu Ser  Thr Glu Ala Asp Thr  Asp Thr Pro Lys Lys  Leu Met Phe
       1865                 1870                 1875
   Arg Arg  Leu Lys Ile Ile Ser  Glu Asn Ser Met Asp  Ser Ala Ile
       1880                 1885                 1890
   Ser Asp  Ala Thr Ser Glu Leu  Glu Gly Lys Asp Gly  Lys Glu Asp
       1895                 1900                 1905
   Leu Asp  Gln Leu Glu Asn Val  Pro Val Glu Glu Glu  Glu Glu Leu
       1910                 1915                 1920
   Gln Ser  Gln Gln Leu Leu Pro  Gln Gln Leu Pro Glu  Cys Lys Val
       1925                 1930                 1935
   Asp Ser  Glu Thr Asn Ile Glu  Ala Ser Lys Leu Pro  Thr Ser Glu
       1940                 1945                 1950
   Pro Glu  Ala Asp Ala Glu Ile  Glu Pro Lys Glu Ser  Asn Gly Thr
       1955                 1960                 1965
   Lys Leu  Glu Glu Pro Ile Asn  Glu Glu Thr Pro Ser  Gln Asp Glu
       1970                 1975                 1980
   Glu Glu  Gly Val Ser Asp Val  Glu Ser Glu Arg Ser  Gln Glu Gln
       1985                 1990                 1995
   Pro Asp  Lys Thr Val Asp Ile  Ser Asp Leu Ala Thr  Lys Leu Leu
       2000                 2005                 2010
   Asp Ser  Trp Lys Asp Leu Lys  Glu Val Tyr Arg Ile  Pro Lys Lys
       2015                 2020                 2025
   Ser Gln  Thr Glu Lys Glu Asn  Thr Thr Thr Glu Arg  Gly Arg Asp
       2030                 2035                 2040
   Ala Val  Gly Phe Arg Asp Gln  Thr Pro Ala Pro Lys  Thr Pro Asn
       2045                 2050                 2055
   Arg Ser  Arg Glu Arg Asp Pro  Asp Lys Gln Thr Gln  Asn Lys Glu
       2060                 2065                 2070
   Lys Arg Lys Arg Arg Ser Ser Leu Ser Pro Pro Ser Ser Ala Tyr
   2075 2080 2085
   Glu Arg Gly Thr Lys Arg Pro Asp Asp Arg Tyr Asp Thr Pro Thr
   2090 2095 2100
   Ser Lys Lys Lys Val Arg Ile Lys Asp Arg Asn Lys Leu Ser Thr
   2105 2110 2115
   Glu Glu Arg Arg Lys Leu Phe Glu Gln Glu Val Ala Gln Arg Glu
   2120 2125 2130
   Ala Gln Lys Gln Gln Gln Gln Met Gln Asn Leu Gly Met Thr Ser
   2135 2140 2145
   Pro Leu Pro Tyr Asp Ser Leu Gly Tyr Asn Ala Pro His His Pro
   2150 2155 2160
   Phe Ala Gly Tyr Pro Pro Gly Tyr Pro Met Gln Ala Tyr Val Asp
   2165 2170 2175
   Pro Ser Asn Pro Asn Ala Gly Lys Val Leu Leu Pro Thr Pro Ser
   2180 2185 2190
   Met Asp Pro Val Cys Ser Pro Ala Pro Tyr Asp His Ala Gln Pro
   2195 2200 2205
   Leu Val Gly His Ser Thr Glu Pro Leu Ser Ala Pro Pro Pro Val
   2210 2215 2220
   Pro Val Val Pro His Val Ala Ala Pro Val Glu Val Ser Ser Ser
   2225 2230 2235
   Gln Tyr Val Ala Gln Ser Asp Gly Val Val His Gln Asp Ser Ser
   2240 2245 2250
   Val Ala Val Leu Pro Val Pro Ala Pro Gly Pro Val Gln Gly Gln
   2255 2260 2265
<210> 24
   <211> 215
   <212> PRT
   <213> Homo sapiens
<400> 24
   Met Ala Ser Glu Thr Glu Lys Thr His Ala Leu Leu Gln Thr Cys Ser
   1 5 10 15
   Thr Glu Ser Leu Ile Ser Ser Leu Gly Leu Gly Ala Phe Cys Leu Val
   20 25 30
   Ala Asp Arg Leu Leu Gln Phe Ser Thr Ile Gln Gln Asn Asp Trp Leu
   35 40 45
   Arg Ala Leu Ser Asp Asn Ala Val His Cys Val Ile Gly Met Trp Ser
   50 55 60
   Trp Ala Val Val Thr Gly Ile Lys Lys Lys Thr Asp Phe Gly Glu Ile
   65 70 75 80
   Ile Leu Ala Gly Phe Leu Ala Ser Val Ile Asp Val Asp His Phe Phe
   85 90 95
   Leu Ala Gly Ser Met Ser Leu Lys Ala Ala Leu Thr Leu Pro Arg Arg
   100 105 110
   Pro Phe Leu His Cys Ser Thr Val Ile Pro Val Val Val Leu Thr Leu
   115 120 125
   Lys Phe Thr Met His Leu Phe Lys Leu Lys Asp Ser Trp Cys Phe Leu
   130 135 140
   Pro Trp Met Leu Phe Ile Ser Trp Thr Ser His His Ile Arg Asp Gly
   145 150 155 160
   Ile Arg His Gly Leu Trp Ile Cys Pro Phe Gly Lys Thr Ser Pro Leu
   165 170 175
   Pro Phe Trp Leu Tyr Val Ile Ile Thr Ser Ser Leu Pro His Ile Cys
   180 185 190
   Ser Phe Val Met Tyr Leu Thr Gly Thr Arg Gln Met Met Ser Ser Lys
   195 200 205
   His Gly Val Arg Ile Asp Val
   210 215
<210> 25
   <211> 840
   <212> PRT
   <213> Homo sapiens
<400> 25
   Met Asn Gly Glu Tyr Arg Gly Arg Gly Phe Gly Arg Gly Arg Phe Gln
   1 5 10 15
   Ser Trp Lys Arg Gly Arg Gly Gly Gly Asn Phe Ser Gly Lys Trp Arg
               20                  25                  30
   Glu Arg Glu His Arg Pro Asp Leu Ser Lys Thr Thr Gly Lys Arg Thr
           35                  40                  45
   Ser Glu Gln Thr Pro Gln Phe Leu Leu Ser Thr Lys Thr Pro Gln Ser
       50                  55                  60
   Met Gln Ser Thr Leu Asp Arg Phe Ile Pro Tyr Lys Gly Trp Lys Leu
   65                  70                  75                  80
   Tyr Phe Ser Glu Val Tyr Ser Asp Ser Ser Pro Leu Ile Glu Lys Ile
                   85                  90                  95
   Gln Ala Phe Glu Lys Phe Phe Thr Arg His Ile Asp Leu Tyr Asp Lys
               100                 105                 110
   Asp Glu Ile Glu Arg Lys Gly Ser Ile Leu Val Asp Phe Lys Glu Leu
           115                 120                 125
   Thr Glu Gly Gly Glu Val Thr Asn Leu Ile Pro Asp Ile Ala Thr Glu
       130                 135                 140
   Leu Arg Asp Ala Pro Glu Lys Thr Leu Ala Cys Met Gly Leu Ala Ile
   145                 150                 155                 160
   His Gln Val Leu Thr Lys Asp Leu Glu Arg His Ala Ala Glu Leu Gln
                   165                 170                 175
   Ala Gln Glu Gly Leu Ser Asn Asp Gly Glu Thr Met Val Asn Val Pro
               180                 185                 190
   His Ile His Ala Arg Val Tyr Asn Tyr Glu Pro Leu Thr Gln Leu Lys
           195                 200                 205
   Asn Val Arg Ala Asn Tyr Tyr Gly Lys Tyr Ile Ala Leu Arg Gly Thr
       210                 215                 220
   Val Val Arg Val Ser Asn Ile Lys Pro Leu Cys Thr Lys Met Ala Phe
   225                 230                 235                 240
   Leu Cys Ala Ala Cys Gly Glu Ile Gln Ser Phe Pro Leu Pro Asp Gly
                   245                 250                 255
   Lys Tyr Ser Leu Pro Thr Lys Cys Pro Val Pro Val Cys Arg Gly Arg
   260 265 270
   Ser Phe Thr Ala Leu Arg Ser Ser Pro Leu Thr Val Thr Met Asp Trp
   275 280 285
   Gln Ser Ile Lys Ile Gln Glu Leu Met Ser Asp Asp Gln Arg Glu Ala
   290 295 300
   Gly Arg Ile Pro Arg Thr Ile Glu Cys Glu Leu Val His Asp Leu Val
   305 310 315 320
   Asp Ser Cys Val Pro Gly Asp Thr Val Thr Ile Thr Gly Ile Val Lys
   325 330 335
   Val Ser Asn Ala Glu Glu Gly Ser Arg Asn Lys Asn Asp Lys Cys Met
   340 345 350
   Phe Leu Leu Tyr Ile Glu Ala Asn Ser Ile Ser Asn Ser Lys Gly Gln
   355 360 365
   Lys Thr Lys Ser Ser Glu Asp Gly Cys Lys His Gly Met Leu Met Glu
   370 375 380
   Phe Ser Leu Lys Asp Leu Tyr Ala Ile Gln Glu Ile Gln Ala Glu Glu
   385 390 395 400
   Asn Leu Phe Lys Leu Ile Val Asn Ser Leu Cys Pro Val Ile Phe Gly
   405 410 415
   His Glu Leu Val Lys Ala Gly Leu Ala Leu Ala Leu Phe Gly Gly Ser
   420 425 430
   Gln Lys Tyr Ala Asp Asp Lys Asn Arg Ile Pro Ile Arg Gly Asp Pro
   435 440 445
   His Ile Leu Val Val Gly Asp Pro Gly Leu Gly Lys Ser Gln Met Leu
   450 455 460
   Gln Ala Ala Cys Asn Val Ala Pro Arg Gly Val Tyr Val Cys Gly Asn
   465 470 475 480
   Thr Thr Thr Thr Ser Gly Leu Thr Val Thr Leu Ser Lys Asp Ser Ser
   485 490 495
   Ser Gly Asp Phe Ala Leu Glu Ala Gly Ala Leu Val Leu Gly Asp Gln
   500 505 510
   Gly Ile Cys Gly Ile Asp Glu Phe Asp Lys Met Gly Asn Gln His Gln
           515                 520                 525
   Ala Leu Leu Glu Ala Met Glu Gln Gln Ser Ile Ser Leu Ala Lys Ala
       530                 535                 540
   Gly Val Val Cys Ser Leu Pro Ala Arg Thr Ser Ile Ile Ala Ala Ala
   545                 550                 555                 560
   Asn Pro Val Gly Gly His Tyr Asn Lys Ala Lys Thr Val Ser Glu Asn
                   565                 570                 575
   Leu Lys Met Gly Ser Ala Leu Leu Ser Arg Phe Asp Leu Val Phe Ile
               580                 585                 590
   Leu Leu Asp Thr Pro Asn Glu His His Asp His Leu Leu Ser Glu His
           595                 600                 605
   Val Ile Ala Ile Arg Ala Gly Lys Gln Arg Thr Ile Ser Ser Ala Thr
       610                 615                 620
   Val Ala Arg Met Asn Ser Gln Asp Ser Asn Thr Ser Val Leu Glu Val
   625                 630                 635                 640
   Val Ser Glu Lys Pro Leu Ser Glu Arg Leu Lys Val Val Pro Gly Glu
                   645                 650                 655
   Thr Ile Asp Pro Ile Pro His Gln Leu Leu Arg Lys Tyr Ile Gly Tyr
               660                 665                 670
   Ala Arg Gln Tyr Val Tyr Pro Arg Leu Ser Thr Glu Ala Ala Arg Val
           675                 680                 685
   Leu Gln Asp Phe Tyr Leu Glu Leu Arg Lys Gln Ser Gln Arg Leu Asn
       690                 695                 700
   Ser Ser Pro Ile Thr Thr Arg Gln Leu Glu Ser Leu Ile Arg Leu Thr
   705                 710                 715                 720
   Glu Ala Arg Ala Arg Leu Glu Leu Arg Glu Glu Ala Thr Lys Glu Asp
                   725                 730                 735
   Ala Glu Asp Ile Val Glu Ile Met Lys Tyr Ser Met Leu Gly Thr Tyr
               740                 745                 750
   Ser Asp Glu Phe Gly Asn Leu Asp Phe Glu Arg Ser Gln His Gly Ser
           755                 760                 765
   Gly Met Ser Asn Arg Ser Thr Ala Lys Arg Phe Ile Ser Ala Leu Asn
   770 775 780
   Asn Val Ala Glu Arg Thr Tyr Asn Asn Ile Phe Gln Phe His Gln Leu
   785 790 795 800
   Arg Gln Ile Ala Lys Glu Leu Asn Ile Gln Val Ala Asp Phe Glu Asn
   805 810 815
   Phe Ile Gly Ser Leu Asn Asp Gln Gly Tyr Leu Leu Lys Lys Gly Pro
   820 825 830
   Lys Val Tyr Gln Leu Gln Thr Met
   835 840
<210> 26
   <211> 261
   <212> PRT
   <213> Homo sapiens
<400> 26
   Met Val Val Leu Ser Val Pro Ala Glu Val Thr Val Ile Leu Leu Asp
   1 5 10 15
   Ile Glu Gly Thr Thr Thr Pro Ile Ala Phe Val Lys Asp Ile Leu Phe
   20 25 30
   Pro Tyr Ile Glu Glu Asn Val Lys Glu Tyr Leu Gln Thr His Trp Glu
   35 40 45
   Glu Glu Glu Cys Gln Gln Asp Val Ser Leu Leu Arg Lys Gln Ala Glu
   50 55 60
   Glu Asp Ala His Leu Asp Gly Ala Val Pro Ile Pro Ala Ala Ser Gly
   65 70 75 80
   Asn Gly Val Asp Asp Leu Gln Gln Met Ile Gln Ala Val Val Asp Asn
   85 90 95
   Val Cys Trp Gln Met Ser Leu Asp Arg Lys Thr Thr Ala Leu Lys Gln
   100 105 110
   Leu Gln Gly His Met Trp Arg Ala Ala Phe Thr Ala Gly Arg Met Lys
   115 120 125
   Ala Glu Phe Phe Ala Asp Val Val Pro Ala Val Arg Lys Trp Arg Glu
   130 135 140
   Ala Gly Met Lys Val Tyr Ile Tyr Ser Ser Gly Ser Val Glu Ala Gln
   145                 150                 155                 160
   Lys Leu Leu Phe Gly His Ser Thr Glu Gly Asp Ile Leu Glu Leu Val
                   165                 170                 175
   Asp Gly His Phe Asp Thr Lys Ile Gly His Lys Val Glu Ser Glu Ser
               180                 185                 190
   Tyr Arg Lys Ile Ala Asp Ser Ile Gly Cys Ser Thr Asn Asn Ile Leu
           195                 200                 205
   Phe Leu Thr Asp Val Thr Arg Glu Ala Ser Ala Ala Glu Glu Ala Asp
       210                 215                 220
   Val His Val Ala Val Val Val Arg Pro Gly Asn Ala Gly Leu Thr Asp
   225                 230                 235                 240
   Asp Glu Lys Thr Tyr Tyr Ser Leu Ile Thr Ser Phe Ser Glu Leu Tyr
                   245                 250                 255
   Leu Pro Ser Ser Thr
               260
<210> 27
   <211> 788
   <212> PRT
   <213> Homo sapiens
<400> 27
   Met Ala Met Glu Ser Thr Ala Thr Ala Ala Val Ala Ala Glu Leu Val
   1               5                   10                  15
   Ser Ala Asp Lys Ile Glu Asp Val Pro Ala Pro Ser Thr Ser Ala Asp
               20                  25                  30
   Lys Val Glu Ser Leu Asp Val Asp Ser Glu Ala Lys Lys Leu Leu Gly
           35                  40                  45
   Leu Gly Gln Lys His Leu Val Met Gly Asp Ile Pro Ala Ala Val Asn
       50                  55                  60
   Ala Phe Gln Glu Ala Ala Ser Leu Leu Gly Lys Lys Tyr Gly Glu Thr
   65                  70                  75                  80
   Ala Asn Glu Cys Gly Glu Ala Phe Phe Phe Tyr Gly Lys Ser Leu Leu
                   85                  90                  95
   Glu Leu Ala Arg Met Glu Asn Gly Val Leu Gly Asn Ala Leu Glu Gly
               100                 105                 110
   Val His Val Glu Glu Glu Glu Gly Glu Lys Thr Glu Asp Glu Ser Leu
           115                 120                 125
   Val Glu Asn Asn Asp Asn Ile Asp Glu Glu Ala Arg Glu Glu Leu Arg
       130                 135                 140
   Glu Gln Val Tyr Asp Ala Met Gly Glu Lys Glu Glu Ala Lys Lys Thr
   145                 150                 155                 160
   Glu Asp Lys Ser Leu Ala Lys Pro Glu Thr Asp Lys Glu Gln Asp Ser
                   165                 170                 175
   Glu Met Glu Lys Gly Gly Arg Glu Asp Met Asp Ile Ser Lys Ser Ala
               180                 185                 190
   Glu Glu Pro Gln Glu Lys Val Asp Leu Thr Leu Asp Trp Leu Thr Glu
           195                 200                 205
   Thr Ser Glu Glu Ala Lys Gly Gly Ala Ala Pro Glu Gly Pro Asn Glu
       210                 215                 220
   Ala Glu Val Thr Ser Gly Lys Pro Glu Gln Glu Val Pro Asp Ala Glu
   225                 230                 235                 240
   Glu Glu Lys Ser Val Ser Gly Thr Asp Val Gln Glu Glu Cys Arg Glu
                   245                 250                 255
   Lys Gly Gly Gln Glu Lys Gln Gly Glu Val Ile Val Ser Ile Glu Glu
               260                 265                 270
   Lys Pro Lys Glu Val Ser Glu Glu Gln Pro Val Val Thr Leu Glu Lys
           275                 280                 285
   Gln Gly Thr Ala Val Glu Val Glu Ala Glu Ser Leu Asp Pro Thr Val
       290                 295                 300
   Lys Pro Val Asp Val Gly Gly Asp Glu Pro Glu Glu Lys Val Val Thr
   305                 310                 315                 320
   Ser Glu Asn Glu Ala Gly Lys Ala Val Leu Glu Gln Leu Val Gly Gln
                   325                 330                 335
   Glu Val Pro Pro Ala Glu Glu Ser Pro Glu Val Thr Thr Glu Ala Ala
               340                 345                 350
   Glu Ala Ser Ala Val Glu Ala Gly Ser Glu Val Ser Glu Lys Pro Gly
           355                 360                 365
   Gln Glu Ala Pro Val Leu Pro Lys Asp Gly Ala Val Asn Gly Pro Ser
       370                 375                 380
   Val Val Gly Asp Gln Thr Pro Ile Glu Pro Gln Thr Ser Ile Glu Arg
   385                 390                 395                 400
   Leu Thr Glu Thr Lys Asp Gly Ser Gly Leu Glu Glu Lys Val Arg Ala
                   405                 410                 415
   Lys Leu Val Pro Ser Gln Glu Glu Thr Lys Leu Ser Val Glu Glu Ser
               420                 425                 430
   Glu Ala Ala Gly Asp Gly Val Asp Thr Lys Val Ala Gln Gly Ala Thr
           435                 440                 445
   Glu Lys Ser Pro Glu Asp Lys Val Gln Ile Ala Ala Asn Glu Glu Thr
       450                 455                 460
   Gln Glu Arg Glu Glu Gln Met Lys Glu Gly Glu Glu Thr Glu Gly Ser
   465                 470                 475                 480
   Glu Glu Asp Asp Lys Glu Asn Asp Lys Thr Glu Glu Met Pro Asn Asp
                   485                 490                 495
   Ser Val Leu Glu Asn Lys Ser Leu Gln Glu Asn Glu Glu Glu Glu Ile
               500                 505                 510
   Gly Asn Leu Glu Leu Ala Trp Asp Met Leu Asp Leu Ala Lys Ile Ile
           515                 520                 525
   Phe Lys Arg Gln Glu Thr Lys Glu Ala Gln Leu Tyr Ala Ala Gln Ala
       530                 535                 540
   His Leu Lys Leu Gly Glu Val Ser Val Glu Ser Glu Asn Tyr Val Gln
   545                 550                 555                 560
   Ala Val Glu Glu Phe Gln Ser Cys Leu Asn Leu Gln Glu Gln Tyr Leu
                   565                 570                 575
   Glu Ala His Asp Arg Leu Leu Ala Glu Thr His Tyr Gln Leu Gly Leu
               580                 585                 590
   Ala Tyr Gly Tyr Asn Ser Gln Tyr Asp Glu Ala Val Ala Gln Phe Ser
               595                 600                 605
   Lys Ser Ile Glu Val Ile Glu Asn Arg Met Ala Val Leu Asn Glu Gln
       610                 615                 620
   Val Lys Glu Ala Glu Gly Ser Ser Ala Glu Tyr Lys Lys Glu Ile Glu
   625                 630                 635                 640
   Glu Leu Lys Glu Leu Leu Pro Glu Ile Arg Glu Lys Ile Glu Asp Ala
                   645                 650                 655
   Lys Glu Ser Gln Arg Ser Gly Asn Val Ala Glu Leu Ala Leu Lys Ala
               660                 665                 670
   Thr Leu Val Glu Ser Ser Thr Ser Gly Phe Thr Pro Gly Gly Gly Gly
           675                 680                 685
   Ser Ser Val Ser Met Ile Ala Ser Arg Lys Pro Thr Asp Gly Ala Ser
       690                 695                 700
   Ser Ser Asn Cys Val Thr Asp Ile Ser His Leu Val Arg Lys Lys Arg
   705                 710                 715                 720
   Lys Pro Glu Glu Glu Ser Pro Arg Lys Asp Asp Ala Lys Lys Ala Lys
                   725                 730                 735
   Gln Glu Pro Glu Val Asn Gly Gly Ser Gly Asp Ala Val Pro Ser Gly
               740                 745                 750
   Asn Glu Val Ser Glu Asn Met Glu Glu Glu Ala Glu Asn Gln Ala Glu
           755                 760                 765
   Ser Arg Ala Ala Val Glu Gly Thr Val Glu Ala Gly Ala Thr Val Glu
       770                 775                 780
   Ser Thr Ala Cys
   785
<210> 28
   <211> 609
   <212> PRT
   <213> Homo sapiens
<400> 28
<210> 29
   <211> 609
   <212> PRT
   <213> Homo sapiens
<400> 29
<210> 30
   <211> 453
   <212> PRT
   <213> Homo sapiens
<400> 30
   Met Ser Trp Ser Leu His Pro Arg Asn Leu Ile Leu Tyr Phe Tyr Ala
   1               5                   10                  15
   Leu Leu Phe Leu Ser Ser Thr Cys Val Ala Tyr Val Ala Thr Arg Asp
               20                  25                  30
   Asn Cys Cys Ile Leu Asp Glu Arg Phe Gly Ser Tyr Cys Pro Thr Thr
           35                  40                  45
   Cys Gly Ile Ala Asp Phe Leu Ser Thr Tyr Gln Thr Lys Val Asp Lys
       50                  55                  60
   Asp Leu Gln Ser Leu Glu Asp Ile Leu His Gln Val Glu Asn Lys Thr
   65                  70                  75                  80
   Ser Glu Val Lys Gln Leu Ile Lys Ala Ile Gln Leu Thr Tyr Asn Pro
                   85                  90                  95
   Asp Glu Ser Ser Lys Pro Asn Met Ile Asp Ala Ala Thr Leu Lys Ser
               100                 105                 110
   Arg Lys Met Leu Glu Glu Ile Met Lys Tyr Glu Ala Ser Ile Leu Thr
           115                 120                 125
   His Asp Ser Ser Ile Arg Tyr Leu Gln Glu Ile Tyr Asn Ser Asn Asn
       130                 135                 140
   Gln Lys Ile Val Asn Leu Lys Glu Lys Val Ala Gln Leu Glu Ala Gln
   145                 150                 155                 160
   Cys Gln Glu Pro Cys Lys Asp Thr Val Gln Ile His Asp Ile Thr Gly
                   165                 170                 175
   Lys Asp Cys Gln Asp Ile Ala Asn Lys Gly Ala Lys Gln Ser Gly Leu
               180                 185                 190
   Tyr Phe Ile Lys Pro Leu Lys Ala Asn Gln Gln Phe Leu Val Tyr Cys
           195                 200                 205
   Glu Ile Asp Gly Ser Gly Asn Gly Trp Thr Val Phe Gln Lys Arg Leu
       210                 215                 220
   Asp Gly Ser Val Asp Phe Lys Lys Asn Trp Ile Gln Tyr Lys Glu Gly
   225 230 235 240
   Phe Gly His Leu Ser Pro Thr Gly Thr Thr Glu Phe Trp Leu Gly Asn
   245 250 255
   Glu Lys Ile His Leu Ile Ser Thr Gln Ser Ala Ile Pro Tyr Ala Leu
   260 265 270
   Arg Val Glu Leu Glu Asp Trp Asn Gly Arg Thr Ser Thr Ala Asp Tyr
   275 280 285
   Ala Met Phe Lys Val Gly Pro Glu Ala Asp Lys Tyr Arg Leu Thr Tyr
   290 295 300
   Ala Tyr Phe Ala Gly Gly Asp Ala Gly Asp Ala Phe Asp Gly Phe Asp
   305 310 315 320
   Phe Gly Asp Asp Pro Ser Asp Lys Phe Phe Thr Ser His Asn Gly Met
   325 330 335
   Gln Phe Ser Thr Trp Asp Asn Asp Asn Asp Lys Phe Glu Gly Asn Cys
   340 345 350
   Ala Glu Gln Asp Gly Ser Gly Trp Trp Met Asn Lys Cys His Ala Gly
   355 360 365
   His Leu Asn Gly Val Tyr Tyr Gln Gly Gly Thr Tyr Ser Lys Ala Ser
   370 375 380
   Thr Pro Asn Gly Tyr Asp Asn Gly Ile Ile Trp Ala Thr Trp Lys Thr
   385 390 395 400
   Arg Trp Tyr Ser Met Lys Lys Thr Thr Met Lys Ile Ile Pro Phe Asn
   405 410 415
   Arg Leu Thr Ile Gly Glu Gly Gln Gln His His Leu Gly Gly Ala Lys
   420 425 430
   Gln Val Arg Pro Glu His Pro Ala Glu Thr Glu Tyr Asp Ser Leu Tyr
   435 440 445
   Pro Glu Asp Asp Leu
   450
<210> 31
   <211> 453
   <212> PRT
   <213> Homo sapiens
<400> 31
   Met Ser Trp Ser Leu His Pro Arg Asn Leu Ile Leu Tyr Phe Tyr Ala
   1               5                   10                  15
   Leu Leu Phe Leu Ser Ser Thr Cys Val Ala Tyr Val Ala Thr Arg Asp
               20                  25                  30
   Asn Cys Cys Ile Leu Asp Glu Arg Phe Gly Ser Tyr Cys Pro Thr Thr
           35                  40                  45
   Cys Gly Ile Ala Asp Phe Leu Ser Thr Tyr Gln Thr Lys Val Asp Lys
       50                  55                  60
   Asp Leu Gln Ser Leu Glu Asp Ile Leu His Gln Val Glu Asn Lys Thr
   65                  70                  75                  80
   Ser Glu Val Lys Gln Leu Ile Lys Ala Ile Gln Leu Thr Tyr Asn Pro
                   85                  90                  95
   Asp Glu Ser Ser Lys Pro Asn Met Ile Asp Ala Ala Thr Leu Lys Ser
               100                 105                 110
   Arg Lys Met Leu Glu Glu Ile Met Lys Tyr Glu Ala Ser Ile Leu Thr
           115                 120                 125
   His Asp Ser Ser Ile Arg Tyr Leu Gln Glu Ile Tyr Asn Ser Asn Asn
       130                 135                 140
   Gln Lys Ile Val Asn Leu Lys Glu Lys Val Ala Gln Leu Glu Ala Gln
   145                 150                 155                 160
   Cys Gln Glu Pro Cys Lys Asp Thr Val Gln Ile His Asp Ile Thr Gly
                   165                 170                 175
   Lys Asp Cys Gln Asp Ile Ala Asn Lys Gly Ala Lys Gln Ser Gly Leu
               180                 185                 190
   Tyr Phe Ile Lys Pro Leu Lys Ala Asn Gln Gln Phe Leu Val Tyr Cys
           195                 200                 205
   Glu Ile Asp Gly Ser Gly Asn Gly Trp Thr Val Phe Gln Lys Arg Leu
       210                 215                 220
   Asp Gly Ser Val Asp Phe Lys Lys Asn Trp Ile Gln Tyr Lys Glu Gly
   225                 230                 235                 240
   Phe Gly His Leu Ser Pro Thr Gly Thr Thr Glu Phe Trp Leu Gly Asn
   245 250 255
   Glu Lys Ile His Leu Ile Ser Thr Gln Ser Ala Ile Pro Tyr Ala Leu
   260 265 270
   Arg Val Glu Leu Glu Asp Trp Asn Gly Arg Thr Ser Thr Ala Asp Tyr
   275 280 285
   Ala Met Phe Lys Val Gly Pro Glu Ala Asp Lys Tyr Arg Leu Thr Tyr
   290 295 300
   Ala Tyr Phe Ala Gly Gly Asp Ala Gly Asp Ala Phe Asp Gly Phe Asp
   305 310 315 320
   Phe Gly Asp Asp Pro Ser Asp Lys Phe Phe Thr Ser His Asn Gly Met
   325 330 335
   Gln Phe Ser Thr Trp Asp Asn Asp Asn Asp Lys Phe Glu Gly Asn Cys
   340 345 350
   Ala Glu Gln Asp Gly Ser Gly Trp Trp Met Asn Lys Cys His Ala Gly
   355 360 365
   His Leu Asn Gly Val Tyr Tyr Gln Gly Gly Thr Tyr Ser Lys Ala Ser
   370 375 380
   Thr Pro Asn Gly Tyr Asp Asn Gly Ile Ile Trp Ala Thr Trp Lys Thr
   385 390 395 400
   Arg Trp Tyr Ser Met Lys Lys Thr Thr Met Lys Ile Ile Pro Phe Asn
   405 410 415
   Arg Leu Thr Ile Gly Glu Gly Gln Gln His His Leu Gly Gly Ala Lys
   420 425 430
   Gln Val Arg Pro Glu His Pro Ala Glu Thr Glu Tyr Asp Ser Leu Tyr
   435 440 445
   Pro Glu Asp Asp Leu
   450
<210> 32
   <211> 644
   <212> PRT
   <213> Homo sapiens
<400> 32
<210> 33
   <211> 1231
   <212> PRT
   <213> Homo sapiens
<400> 33
   Met Arg Leu Leu Ala Lys Ile Ile Cys Leu Met Leu Trp Ala Ile Cys
   1               5                   10                  15
   Val Ala Glu Asp Cys Asn Glu Leu Pro Pro Arg Arg Asn Thr Glu Ile
               20                  25                  30
   Leu Thr Gly Ser Trp Ser Asp Gln Thr Tyr Pro Glu Gly Thr Gln Ala
           35                  40                  45
   Ile Tyr Lys Cys Arg Pro Gly Tyr Arg Ser Leu Gly Asn Val Ile Met
       50                  55                  60
   Val Cys Arg Lys Gly Glu Trp Val Ala Leu Asn Pro Leu Arg Lys Cys
   65                  70                  75                  80
   Gln Lys Arg Pro Cys Gly His Pro Gly Asp Thr Pro Phe Gly Thr Phe
   85 90 95
   Thr Leu Thr Gly Gly Asn Val Phe Glu Tyr Gly Val Lys Ala Val Tyr
   100 105 110
   Thr Cys Asn Glu Gly Tyr Gln Leu Leu Gly Glu Ile Asn Tyr Arg Glu
   115 120 125
   Cys Asp Thr Asp Gly Trp Thr Asn Asp Ile Pro Ile Cys Glu Val Val
   130 135 140
   Lys Cys Leu Pro Val Thr Ala Pro Glu Asn Gly Lys Ile Val Ser Ser
   145 150 155 160
   Ala Met Glu Pro Asp Arg Glu Tyr His Phe Gly Gln Ala Val Arg Phe
   165 170 175
   Val Cys Asn Ser Gly Tyr Lys Ile Glu Gly Asp Glu Glu Met His Cys
   180 185 190
   Ser Asp Asp Gly Phe Trp Ser Lys Glu Lys Pro Lys Cys Val Glu Ile
   195 200 205
   Ser Cys Lys Ser Pro Asp Val Ile Asn Gly Ser Pro Ile Ser Gln Lys
   210 215 220
   Ile Ile Tyr Lys Glu Asn Glu Arg Phe Gln Tyr Lys Cys Asn Met Gly
   225 230 235 240
   Tyr Glu Tyr Ser Glu Arg Gly Asp Ala Val Cys Thr Glu Ser Gly Trp
   245 250 255
   Arg Pro Leu Pro Ser Cys Glu Glu Lys Ser Cys Asp Asn Pro Tyr Ile
   260 265 270
   Pro Asn Gly Asp Tyr Ser Pro Leu Arg Ile Lys His Arg Thr Gly Asp
   275 280 285
   Glu Ile Thr Tyr Gln Cys Arg Asn Gly Phe Tyr Pro Ala Thr Arg Gly
   290 295 300
   Asn Thr Ala Lys Cys Thr Ser Thr Gly Trp Ile Pro Ala Pro Arg Cys
   305 310 315 320
   Thr Leu Lys Pro Cys Asp Tyr Pro Asp Ile Lys His Gly Gly Leu Tyr
   325 330 335
   His Glu Asn Met Arg Arg Pro Tyr Phe Pro Val Ala Val Gly Lys Tyr
   340 345 350
   Tyr Ser Tyr Tyr Cys Asp Glu His Phe Glu Thr Pro Ser Gly Ser Tyr
   355 360 365
   Trp Asp His Ile His Cys Thr Gln Asp Gly Trp Ser Pro Ala Val Pro
   370 375 380
   Cys Leu Arg Lys Cys Tyr Phe Pro Tyr Leu Glu Asn Gly Tyr Asn Gln
   385 390 395 400
   Asn Tyr Gly Arg Lys Phe Val Gln Gly Lys Ser Ile Asp Val Ala Cys
   405 410 415
   His Pro Gly Tyr Ala Leu Pro Lys Ala Gln Thr Thr Val Thr Cys Met
   420 425 430
   Glu Asn Gly Trp Ser Pro Thr Pro Arg Cys Ile Arg Val Lys Thr Cys
   435 440 445
   Ser Lys Ser Ser Ile Asp Ile Glu Asn Gly Phe Ile Ser Glu Ser Gln
   450 455 460
   Tyr Thr Tyr Ala Leu Lys Glu Lys Ala Lys Tyr Gln Cys Lys Leu Gly
   465 470 475 480
   Tyr Val Thr Ala Asp Gly Glu Thr Ser Gly Ser Ile Thr Cys Gly Lys
   485 490 495
   Asp Gly Trp Ser Ala Gln Pro Thr Cys Ile Lys Ser Cys Asp Ile Pro
   500 505 510
   Val Phe Met Asn Ala Arg Thr Lys Asn Asp Phe Thr Trp Phe Lys Leu
   515 520 525
   Asn Asp Thr Leu Asp Tyr Glu Cys His Asp Gly Tyr Glu Ser Asn Thr
   530 535 540
   Gly Ser Thr Thr Gly Ser Ile Val Cys Gly Tyr Asn Gly Trp Ser Asp
   545 550 555 560
   Leu Pro Ile Cys Tyr Glu Arg Glu Cys Glu Leu Pro Lys Ile Asp Val
   565 570 575
   His Leu Val Pro Asp Arg Lys Lys Asp Gln Tyr Lys Val Gly Glu Val
   580 585 590
   Leu Lys Phe Ser Cys Lys Pro Gly Phe Thr Ile Val Gly Pro Asn Ser
   595 600 605
   Val Gln Cys Tyr His Phe Gly Leu Ser Pro Asp Leu Pro Ile Cys Lys
   610 615 620
   Glu Gln Val Gln Ser Cys Gly Pro Pro Pro Glu Leu Leu Asn Gly Asn
   625 630 635 640
   Val Lys Glu Lys Thr Lys Glu Glu Tyr Gly His Ser Glu Val Val Glu
   645 650 655
   Tyr Tyr Cys Asn Pro Arg Phe Leu Met Lys Gly Pro Asn Lys Ile Gln
   660 665 670
   Cys Val Asp Gly Glu Trp Thr Thr Leu Pro Val Cys Ile Val Glu Glu
   675 680 685
   Ser Thr Cys Gly Asp Ile Pro Glu Leu Glu His Gly Trp Ala Gln Leu
   690 695 700
   Ser Ser Pro Pro Tyr Tyr Tyr Gly Asp Ser Val Glu Phe Asn Cys Ser
   705 710 715 720
   Glu Ser Phe Thr Met Ile Gly His Arg Ser Ile Thr Cys Ile His Gly
   725 730 735
   Val Trp Thr Gln Leu Pro Gln Cys Val Ala Ile Asp Lys Leu Lys Lys
   740 745 750
   Cys Lys Ser Ser Asn Leu Ile Ile Leu Glu Glu His Leu Lys Asn Lys
   755 760 765
   Lys Glu Phe Asp His Asn Ser Asn Ile Arg Tyr Arg Cys Arg Gly Lys
   770 775 780
   Glu Gly Trp Ile His Thr Val Cys Ile Asn Gly Arg Trp Asp Pro Glu
   785 790 795 800
   Val Asn Cys Ser Met Ala Gln Ile Gln Leu Cys Pro Pro Pro Pro Gln
   805 810 815
   Ile Pro Asn Ser His Asn Met Thr Thr Thr Leu Asn Tyr Arg Asp Gly
   820 825 830
   Glu Lys Val Ser Val Leu Cys Gln Glu Asn Tyr Leu Ile Gln Glu Gly
           835                 840                 845
   Glu Glu Ile Thr Cys Lys Asp Gly Arg Trp Gln Ser Ile Pro Leu Cys
       850                 855                 860
   Val Glu Lys Ile Pro Cys Ser Gln Pro Pro Gln Ile Glu His Gly Thr
   865                 870                 875                 880
   Ile Asn Ser Ser Arg Ser Ser Gln Glu Ser Tyr Ala His Gly Thr Lys
                   885                 890                 895
   Leu Ser Tyr Thr Cys Glu Gly Gly Phe Arg Ile Ser Glu Glu Asn Glu
               900                 905                 910
   Thr Thr Cys Tyr Met Gly Lys Trp Ser Ser Pro Pro Gln Cys Glu Gly
           915                 920                 925
   Leu Pro Cys Lys Ser Pro Pro Glu Ile Ser His Gly Val Val Ala His
       930                 935                 940
   Met Ser Asp Ser Tyr Gln Tyr Gly Glu Glu Val Thr Tyr Lys Cys Phe
   945                 950                 955                 960
   Glu Gly Phe Gly Ile Asp Gly Pro Ala Ile Ala Lys Cys Leu Gly Glu
                   965                 970                 975
   Lys Trp Ser His Pro Pro Ser Cys Ile Lys Thr Asp Cys Leu Ser Leu
               980                 985                 990
   Pro Ser Phe Glu Asn Ala Ile Pro  Met Gly Glu Lys Lys  Asp Val Tyr
           995                 1000                 1005
   Lys Ala  Gly Glu Gln Val Thr  Tyr Thr Cys Ala Thr  Tyr Tyr Lys
       1010                 1015                 1020
   Met Asp  Gly Ala Ser Asn Val  Thr Cys Ile Asn Ser  Arg Trp Thr
       1025                 1030                 1035
   Gly Arg  Pro Thr Cys Arg Asp  Thr Ser Cys Val Asn  Pro Pro Thr
       1040                 1045                 1050
   Val Gln  Asn Ala Tyr Ile Val  Ser Arg Gln Met Ser  Lys Tyr Pro
       1055                 1060                 1065
   Ser Gly  Glu Arg Val Arg Tyr  Gln Cys Arg Ser Pro  Tyr Glu Met
       1070                 1075                 1080
   Phe Gly  Asp Glu Glu Val Met  Cys Leu Asn Gly Asn  Trp Thr Glu
       1085                 1090                 1095
   Pro Pro  Gln Cys Lys Asp Ser  Thr Gly Lys Cys Gly  Pro Pro Pro
       1100                 1105                 1110
   Pro Ile  Asp Asn Gly Asp Ile  Thr Ser Phe Pro Leu  Ser Val Tyr
       1115                 1120                 1125
   Ala Pro  Ala Ser Ser Val Glu  Tyr Gln Cys Gln Asn  Leu Tyr Gln
       1130                 1135                 1140
   Leu Glu  Gly Asn Lys Arg Ile  Thr Cys Arg Asn Gly  Gln Trp Ser
       1145                 1150                 1155
   Glu Pro  Pro Lys Cys Leu His  Pro Cys Val Ile Ser  Arg Glu Ile
       1160                 1165                 1170
   Met Glu  Asn Tyr Asn Ile Ala  Leu Arg Trp Thr Ala  Lys Gln Lys
       1175                 1180                 1185
   Leu Tyr  Ser Arg Thr Gly Glu  Ser Val Glu Phe Val  Cys Lys Arg
       1190                 1195                 1200
   Gly Tyr  Arg Leu Ser Ser Arg  Ser His Thr Leu Arg  Thr Thr Cys
       1205                 1210                 1215
   Trp Asp  Gly Lys Leu Glu Tyr  Pro Thr Cys Ala Lys  Arg
       1220                 1225                 1230
<210> 34
   <211> 30
   <212> PRT
   <213> Homo sapiens
<400> 34
   Gln Ala His Gly Arg Cys Ser Ala Gly Ala Gln Phe Val Phe Cys Arg
   1               5                   10                  15
   Arg Ser Ala Gly Ala Ala Cys Thr Gln Gln Ala Leu Ser Arg
               20                  25                  30
<210> 35
   <211> 452
   <212> PRT
   <213> Homo sapiens
<400> 35
   Gly Ser Ala Ser Ala Pro Thr Leu Phe Pro Leu Val Ser Cys Glu Asn
   1               5                   10                  15
   Ser Pro Ser Asp Thr Ser Ser Val Ala Val Gly Cys Leu Ala Gln Asp
               20                  25                  30
   Phe Leu Pro Asp Ser Ile Thr Leu Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro Val Ile Ala Glu Leu Pro Pro Lys
               100                 105                 110
   Val Ser Val Phe Val Pro Pro Arg Asp Gly Phe Phe Gly Asn Pro Arg
           115                 120                 125
   Lys Ser Lys Leu Ile Cys Gln Ala Thr Gly Phe Ser Pro Arg Gln Ile
       130                 135                 140
   Gln Val Ser Trp Leu Arg Glu Gly Lys Gln Val Gly Ser Gly Val Thr
   145                 150                 155                 160
   Thr Asp Gln Val Gln Ala Glu Ala Lys Glu Ser Gly Pro Thr Thr Tyr
                   165                 170                 175
   Lys Val Thr Ser Thr Leu Thr Ile Lys Glu Ser Asp Trp Leu Gly Gln
               180                 185                 190
   Ser Met Phe Thr Cys Arg Val Asp His Arg Gly Leu Thr Phe Gln Gln
           195                 200                 205
   Asn Ala Ser Ser Met Cys Val Pro Asp Gln Asp Thr Ala Ile Arg Val
       210                 215                 220
   Phe Ala Ile Pro Pro Ser Phe Ala Ser Ile Phe Leu Thr Lys Ser Thr
   225                 230                 235                 240
   Lys Leu Thr Cys Leu Val Thr Asp Leu Thr Thr Tyr Asp Ser Val Thr
                   245                 250                 255
   Ile Ser Trp Thr Arg Gln Asn Gly Glu Ala Val Lys Thr His Thr Asn
               260                 265                 270
   Ile Ser Glu Ser His Pro Asn Ala Thr Phe Ser Ala Val Gly Glu Ala
           275                 280                 285
   Ser Ile Cys Glu Asp Asp Trp Asn Ser Gly Glu Arg Phe Thr Cys Thr
       290                 295                 300
   Val Thr His Thr Asp Leu Pro Ser Pro Leu Lys Gln Thr Ile Ser Arg
   305                 310                 315                 320
   Pro Lys Gly Val Ala Leu His Arg Pro Asp Val Tyr Leu Leu Pro Pro
                   325                 330                 335
   Ala Arg Glu Gln Leu Asn Leu Arg Glu Ser Ala Thr Ile Thr Cys Leu
               340                 345                 350
   Val Thr Gly Phe Ser Pro Ala Asp Val Phe Val Gln Trp Met Gln Arg
           355                 360                 365
   Gly Gln Pro Leu Ser Pro Glu Lys Tyr Val Thr Ser Ala Pro Met Pro
       370                 375                 380
   Glu Pro Gln Ala Pro Gly Arg Tyr Phe Ala His Ser Ile Leu Thr Val
   385                 390                 395                 400
   Ser Glu Glu Glu Trp Asn Thr Gly Glu Thr Tyr Thr Cys Val Ala His
                   405                 410                 415
   Glu Ala Leu Pro Asn Arg Val Thr Glu Arg Thr Val Asp Lys Ser Thr
               420                 425                 430
   Gly Lys Pro Thr Leu Tyr Asn Val Ser Leu Val Met Ser Asp Thr Ala
           435                 440                 445
   Gly Thr Cys Tyr
       450
<210> 36
   <211> 491
   <212> PRT
   <213> Homo sapiens
<400> 36
<210> 37
   <211> 267
   <212> PRT
   <213> Homo sapiens
<400> 37
<210> 38
   <211> 1663
   <212> PRT
   <213> Homo sapiens
<400> 38
<210> 39
   <211> 609
   <212> PRT
   <213> Homo sapiens
<400> 39
<210> 40
   <211> 42
   <212> PRT
   <213> Homo sapiens
<400> 40
   Gly Gln Pro Arg Glu Pro Gln Val Tyr Thr Leu Pro Pro Ser Arg Asp
   1               5                   10                  15
   Glu Leu Thr Lys Gly Phe Tyr Pro Ser Asp Ile Ala Val Glu Trp Glu
               20                  25                  30
   Ser Asn Gly Gln Pro Glu Asn Asn Tyr Lys
           35                  40
<210> 41
   <211> 609
   <212> PRT
   <213> Homo sapiens
<400> 41
<210> 42
   <211> 491
   <212> PRT
   <213> Homo sapiens
<400> 42
<210> 43
   <211> 491
   <212> PRT
   <213> Homo sapiens
<400> 43
<210> 44
   <211> 52
   <212> PRT
   <213> Homo sapiens
<400> 44
   Gly Gln Pro Arg Glu Pro Gln Val Tyr Thr Leu Pro Pro Ser Arg Asp
   1               5                   10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys
       50
<210> 45
   <211> 453
   <212> PRT
   <213> Homo sapiens
<400> 45
   Met Ser Trp Ser Leu His Pro Arg Asn Leu Ile Leu Tyr Phe Tyr Ala
   1               5                   10                  15
   Leu Leu Phe Leu Ser Ser Thr Cys Val Ala Tyr Val Ala Thr Arg Asp
               20                  25                  30
   Asn Cys Cys Ile Leu Asp Glu Arg Phe Gly Ser Tyr Cys Pro Thr Thr
           35                  40                  45
   Cys Gly Ile Ala Asp Phe Leu Ser Thr Tyr Gln Thr Lys Val Asp Lys
       50                  55                  60
   Asp Leu Gln Ser Leu Glu Asp Ile Leu His Gln Val Glu Asn Lys Thr
   65                  70                  75                  80
   Ser Glu Val Lys Gln Leu Ile Lys Ala Ile Gln Leu Thr Tyr Asn Pro
                   85                  90                  95
   Asp Glu Ser Ser Lys Pro Asn Met Ile Asp Ala Ala Thr Leu Lys Ser
               100                 105                 110
   Arg Lys Met Leu Glu Glu Ile Met Lys Tyr Glu Ala Ser Ile Leu Thr
           115                 120                 125
   His Asp Ser Ser Ile Arg Tyr Leu Gln Glu Ile Tyr Asn Ser Asn Asn
       130                 135                 140
   Gln Lys Ile Val Asn Leu Lys Glu Lys Val Ala Gln Leu Glu Ala Gln
   145                 150                 155                 160
   Cys Gln Glu Pro Cys Lys Asp Thr Val Gln Ile His Asp Ile Thr Gly
                   165                 170                 175
   Lys Asp Cys Gln Asp Ile Ala Asn Lys Gly Ala Lys Gln Ser Gly Leu
               180                 185                 190
   Tyr Phe Ile Lys Pro Leu Lys Ala Asn Gln Gln Phe Leu Val Tyr Cys
           195                 200                 205
   Glu Ile Asp Gly Ser Gly Asn Gly Trp Thr Val Phe Gln Lys Arg Leu
       210                 215                 220
   Asp Gly Ser Val Asp Phe Lys Lys Asn Trp Ile Gln Tyr Lys Glu Gly
   225                 230                 235                 240
   Phe Gly His Leu Ser Pro Thr Gly Thr Thr Glu Phe Trp Leu Gly Asn
                   245                 250                 255
   Glu Lys Ile His Leu Ile Ser Thr Gln Ser Ala Ile Pro Tyr Ala Leu
               260                 265                 270
   Arg Val Glu Leu Glu Asp Trp Asn Gly Arg Thr Ser Thr Ala Asp Tyr
           275                 280                 285
   Ala Met Phe Lys Val Gly Pro Glu Ala Asp Lys Tyr Arg Leu Thr Tyr
       290                 295                 300
   Ala Tyr Phe Ala Gly Gly Asp Ala Gly Asp Ala Phe Asp Gly Phe Asp
   305                 310                 315                 320
   Phe Gly Asp Asp Pro Ser Asp Lys Phe Phe Thr Ser His Asn Gly Met
                   325                 330                 335
   Gln Phe Ser Thr Trp Asp Asn Asp Asn Asp Lys Phe Glu Gly Asn Cys
               340                 345                 350
   Ala Glu Gln Asp Gly Ser Gly Trp Trp Met Asn Lys Cys His Ala Gly
           355                 360                 365
   His Leu Asn Gly Val Tyr Tyr Gln Gly Gly Thr Tyr Ser Lys Ala Ser
       370                 375                 380
   Thr Pro Asn Gly Tyr Asp Asn Gly Ile Ile Trp Ala Thr Trp Lys Thr
   385                 390                 395                 400
   Arg Trp Tyr Ser Met Lys Lys Thr Thr Met Lys Ile Ile Pro Phe Asn
                   405                 410                 415
   Arg Leu Thr Ile Gly Glu Gly Gln Gln His His Leu Gly Gly Ala Lys
               420                 425                 430
   Gln Val Arg Pro Glu His Pro Ala Glu Thr Glu Tyr Asp Ser Leu Tyr
           435                 440                 445
   Pro Glu Asp Asp Leu
       450
<210> 46
   <211> 525
   <212> PRT
   <213> Homo sapiens
<400> 46
   Met Lys Ala Leu Ile Ala Ala Leu Leu Leu Ile Thr Leu Gln Tyr Ser
   1               5                   10                  15
   Cys Ala Val Ser Pro Thr Asp Cys Ser Ala Val Glu Pro Glu Ala Glu
               20                  25                  30
   Lys Ala Leu Asp Leu Ile Asn Lys Arg Arg Arg Asp Gly Tyr Leu Phe
           35                  40                  45
   Gln Leu Leu Arg Ile Ala Asp Ala His Leu Asp Arg Val Glu Asn Thr
       50                  55                  60
   Thr Val Tyr Tyr Leu Val Leu Asp Val Gln Glu Ser Asp Cys Ser Val
   65                  70                  75                  80
   Leu Ser Arg Lys Tyr Trp Asn Asp Cys Glu Pro Pro Asp Ser Arg Arg
                   85                  90                  95
   Pro Ser Glu Ile Val Ile Gly Gln Cys Lys Val Ile Ala Thr Arg His
               100                 105                 110
   Ser His Glu Ser Gln Asp Leu Arg Val Ile Asp Phe Asn Cys Thr Thr
           115                 120                 125
   Ser Ser Val Ser Ser Ala Leu Ala Asn Thr Lys Asp Ser Pro Val Leu
       130                 135                 140
   Ile Asp Phe Phe Glu Asp Thr Glu Arg Tyr Arg Lys Gln Ala Asn Lys
   145                 150                 155                 160
   Ala Leu Glu Lys Tyr Lys Glu Glu Asn Asp Asp Phe Ala Ser Phe Arg
                   165                 170                 175
   Val Asp Arg Ile Glu Arg Val Ala Arg Val Arg Gly Gly Glu Gly Thr
               180                 185                 190
   Gly Tyr Phe Val Asp Phe Ser Val Arg Asn Cys Pro Arg His His Phe
           195                 200                 205
   Pro Arg His Pro Asn Val Phe Gly Phe Cys Arg Ala Asp Leu Phe Tyr
       210                 215                 220
   Asp Val Glu Ala Leu Asp Leu Glu Ser Pro Lys Asn Leu Val Ile Asn
   225 230 235 240
   Cys Glu Val Phe Asp Pro Gln Glu His Glu Asn Ile Asn Gly Val Pro
   245 250 255
   Pro His Leu Gly His Pro Phe His Trp Gly Gly His Glu Arg Ser Ser
   260 265 270
   Thr Thr Lys Pro Pro Phe Lys Pro His Gly Ser Arg Asp His His His
   275 280 285
   Pro His Lys Pro His Glu His Gly Pro Pro Pro Pro Pro Asp Glu Arg
   290 295 300
   Asp His Ser His Gly Pro Pro Leu Pro Gln Gly Pro Pro Pro Leu Leu
   305 310 315 320
   Pro Met Ser Cys Ser Ser Cys Gln His Ala Thr Phe Gly Thr Asn Gly
   325 330 335
   Ala Gln Arg His Ser His Asn Asn Asn Ser Ser Asp Leu His Pro His
   340 345 350
   Lys His His Ser His Glu Gln His Pro His Gly His His Pro His Ala
   355 360 365
   His His Pro His Glu His Asp Thr His Arg Gln His Pro His Gly His
   370 375 380
   His Pro His Gly His His Pro His Gly His His Pro His Gly His His
   385 390 395 400
   Pro His Gly His His Pro His Cys His Asp Phe Gln Asp Tyr Gly Pro
   405 410 415
   Cys Asp Pro Pro Pro His Asn Gln Gly His Cys Cys His Gly His Gly
   420 425 430
   Pro Pro Pro Gly His Leu Arg Arg Arg Gly Pro Gly Lys Gly Pro Arg
   435 440 445
   Pro Phe His Cys Arg Gln Ile Gly Ser Val Tyr Arg Leu Pro Pro Leu
   450 455 460
   Arg Lys Gly Glu Val Leu Pro Leu Pro Glu Ala Asn Phe Pro Ser Phe
   465 470 475 480
   Pro Leu Pro His His Lys His Pro Leu Lys Pro Asp Asn Gln Pro Phe
   485 490 495
   Pro Gln Ser Val Ser Glu Ser Cys Pro Gly Lys Phe Lys Ser Gly Phe
   500 505 510
   Pro Gln Val Ser Met Phe Phe Thr His Thr Phe Pro Lys
   515 520 525
<210> 47
   <211> 525
   <212> PRT
   <213> Homo sapiens
<400> 47
   Met Lys Ala Leu Ile Ala Ala Leu Leu Leu Ile Thr Leu Gln Tyr Ser
   1 5 10 15
   Cys Ala Val Ser Pro Thr Asp Cys Ser Ala Val Glu Pro Glu Ala Glu
   20 25 30
   Lys Ala Leu Asp Leu Ile Asn Lys Arg Arg Arg Asp Gly Tyr Leu Phe
   35 40 45
   Gln Leu Leu Arg Ile Ala Asp Ala His Leu Asp Arg Val Glu Asn Thr
   50 55 60
   Thr Val Tyr Tyr Leu Val Leu Asp Val Gln Glu Ser Asp Cys Ser Val
   65 70 75 80
   Leu Ser Arg Lys Tyr Trp Asn Asp Cys Glu Pro Pro Asp Ser Arg Arg
   85 90 95
   Pro Ser Glu Ile Val Ile Gly Gln Cys Lys Val Ile Ala Thr Arg His
   100 105 110
   Ser His Glu Ser Gln Asp Leu Arg Val Ile Asp Phe Asn Cys Thr Thr
   115 120 125
   Ser Ser Val Ser Ser Ala Leu Ala Asn Thr Lys Asp Ser Pro Val Leu
   130 135 140
   Ile Asp Phe Phe Glu Asp Thr Glu Arg Tyr Arg Lys Gln Ala Asn Lys
   145 150 155 160
   Ala Leu Glu Lys Tyr Lys Glu Glu Asn Asp Asp Phe Ala Ser Phe Arg
   165 170 175
   Val Asp Arg Ile Glu Arg Val Ala Arg Val Arg Gly Gly Glu Gly Thr
               180                 185                 190
   Gly Tyr Phe Val Asp Phe Ser Val Arg Asn Cys Pro Arg His His Phe
           195                 200                 205
   Pro Arg His Pro Asn Val Phe Gly Phe Cys Arg Ala Asp Leu Phe Tyr
       210                 215                 220
   Asp Val Glu Ala Leu Asp Leu Glu Ser Pro Lys Asn Leu Val Ile Asn
   225                 230                 235                 240
   Cys Glu Val Phe Asp Pro Gln Glu His Glu Asn Ile Asn Gly Val Pro
                   245                 250                 255
   Pro His Leu Gly His Pro Phe His Trp Gly Gly His Glu Arg Ser Ser
               260                 265                 270
   Thr Thr Lys Pro Pro Phe Lys Pro His Gly Ser Arg Asp His His His
           275                 280                 285
   Pro His Lys Pro His Glu His Gly Pro Pro Pro Pro Pro Asp Glu Arg
       290                 295                 300
   Asp His Ser His Gly Pro Pro Leu Pro Gln Gly Pro Pro Pro Leu Leu
   305                 310                 315                 320
   Pro Met Ser Cys Ser Ser Cys Gln His Ala Thr Phe Gly Thr Asn Gly
                   325                 330                 335
   Ala Gln Arg His Ser His Asn Asn Asn Ser Ser Asp Leu His Pro His
               340                 345                 350
   Lys His His Ser His Glu Gln His Pro His Gly His His Pro His Ala
           355                 360                 365
   His His Pro His Glu His Asp Thr His Arg Gln His Pro His Gly His
       370                 375                 380
   His Pro His Gly His His Pro His Gly His His Pro His Gly His His
   385                 390                 395                 400
   Pro His Gly His His Pro His Cys His Asp Phe Gln Asp Tyr Gly Pro
                   405                 410                 415
   Cys Asp Pro Pro Pro His Asn Gln Gly His Cys Cys His Gly His Gly
               420                 425                 430
   Pro Pro Pro Gly His Leu Arg Arg Arg Gly Pro Gly Lys Gly Pro Arg
           435                 440                 445
   Pro Phe His Cys Arg Gln Ile Gly Ser Val Tyr Arg Leu Pro Pro Leu
       450                 455                 460
   Arg Lys Gly Glu Val Leu Pro Leu Pro Glu Ala Asn Phe Pro Ser Phe
   465                 470                 475                 480
   Pro Leu Pro His His Lys His Pro Leu Lys Pro Asp Asn Gln Pro Phe
                   485                 490                 495
   Pro Gln Ser Val Ser Glu Ser Cys Pro Gly Lys Phe Lys Ser Gly Phe
               500                 505                 510
   Pro Gln Val Ser Met Phe Phe Thr His Thr Phe Pro Lys
           515                 520                 525
<210> 48
   <211> 698
   <212> PRT
   <213> Homo sapiens
<400> 48
   Met Arg Leu Ala Val Gly Ala Leu Leu Val Cys Ala Val Leu Gly Leu
   1               5                   10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
                   85                  90                  95
   Lys Pro Val Val Ala Glu Phe Tyr Gly Ser Lys Glu Asp Pro Gln Thr
               100                 105                 110
   Phe Tyr Tyr Ala Val Ala Val Val Lys Lys Asp Ser Gly Phe Gln Met
           115                 120                 125
   Asn Gln Leu Arg Gly Lys Lys Ser Cys His Thr Gly Leu Gly Arg Ser
       130                 135                 140
   Ala Gly Trp Asn Ile Pro Ile Gly Leu Leu Tyr Cys Asp Leu Pro Glu
   145                 150                 155                 160
   Pro Arg Lys Pro Leu Glu Lys Ala Val Ala Asn Phe Phe Ser Gly Ser
                   165                 170                 175
   Cys Ala Pro Cys Ala Asp Gly Thr Asp Phe Pro Gln Leu Cys Gln Leu
               180                 185                 190
   Cys Pro Gly Cys Gly Cys Ser Thr Leu Asn Gln Tyr Phe Gly Tyr Ser
           195                 200                 205
   Gly Ala Phe Lys Cys Leu Lys Asp Gly Ala Gly Asp Val Ala Phe Val
       210                 215                 220
   Lys His Ser Thr Ile Phe Glu Asn Leu Ala Asn Lys Ala Asp Arg Asp
   225                 230                 235                 240
   Gln Tyr Glu Leu Leu Cys Leu Asp Asn Thr Arg Lys Pro Val Asp Glu
                   245                 250                 255
   Tyr Lys Asp Cys His Leu Ala Gln Val Pro Ser His Thr Val Val Ala
               260                 265                 270
   Arg Ser Met Gly Gly Lys Glu Asp Leu Ile Trp Glu Leu Leu Asn Gln
           275                 280                 285
   Ala Gln Glu His Phe Gly Lys Asp Lys Ser Lys Glu Phe Gln Leu Phe
       290                 295                 300
   Ser Ser Pro His Gly Lys Asp Leu Leu Phe Lys Asp Ser Ala His Gly
   305                 310                 315                 320
   Phe Leu Lys Val Pro Pro Arg Met Asp Ala Lys Met Tyr Leu Gly Tyr
                   325                 330                 335
   Glu Tyr Val Thr Ala Ile Arg Asn Leu Arg Glu Gly Thr Cys Pro Glu
               340                 345                 350
   Ala Pro Thr Asp Glu Cys Lys Pro Val Lys Trp Cys Ala Leu Ser His
           355                 360                 365
   His Glu Arg Leu Lys Cys Asp Glu Trp Ser Val Asn Ser Val Gly Lys
       370                 375                 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Ile
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
           595                 600                 605
   Arg Lys Asp Lys Glu Ala Cys Val His Lys Ile Leu Arg Gln Gln Gln
       610                 615                 620
   His Leu Phe Gly Ser Asn Val Thr Asp Cys Ser Gly Asn Phe Cys Leu
   625                 630                 635                 640
   Phe Arg Ser Glu Thr Lys Asp Leu Leu Phe Arg Asp Asp Thr Val Cys
                   645                 650                 655
   Leu Ala Lys Leu His Asp Arg Asn Thr Tyr Glu Lys Tyr Leu Gly Glu
               660                 665                 670
   Glu Tyr Val Lys Ala Val Gly Asn Leu Arg Lys Cys Ser Thr Ser Ser
           675                 680                 685
   Leu Leu Glu Ala Cys Thr Phe Arg Arg Pro
       690                 695
<210> 49
   <211> 377
   <212> PRT
   <213> Homo sapiens
<400> 49
   Met Cys Asp Asp Glu Glu Thr Thr Ala Leu Val Cys Asp Asn Gly Ser
   1               5                   10                  15
   Gly Leu Val Lys Ala Gly Phe Ala Gly Asp Asp Ala Pro Arg Ala Val
               20                  25                  30
   Phe Pro Ser Ile Val Gly Arg Pro Arg His Gln Gly Val Met Val Gly
           35                  40                  45
   Met Gly Gln Lys Asp Ser Tyr Val Gly Asp Glu Ala Gln Ser Lys Arg
       50                  55                  60
   Gly Ile Leu Thr Leu Lys Tyr Pro Ile Glu His Gly Ile Ile Thr Asn
   65                  70                  75                  80
   Trp Asp Asp Met Glu Lys Ile Trp His His Thr Phe Tyr Asn Glu Leu
                   85                  90                  95
   Arg Val Ala Pro Glu Glu His Pro Thr Leu Leu Thr Glu Ala Pro Leu
               100                 105                 110
   Asn Pro Lys Ala Asn Arg Glu Lys Met Thr Gln Ile Met Phe Glu Thr
           115                 120                 125
   Phe Asn Val Pro Ala Met Tyr Val Ala Ile Gln Ala Val Leu Ser Leu
       130                 135                 140
   Tyr Ala Ser Gly Arg Thr Thr Gly Ile Val Leu Asp Ser Gly Asp Gly
   145 150 155 160
   Val Thr His Asn Val Pro Ile Tyr Glu Gly Tyr Ala Leu Pro His Ala
   165 170 175
   Ile Met Arg Leu Asp Leu Ala Gly Arg Asp Leu Thr Asp Tyr Leu Met
   180 185 190
   Lys Ile Leu Thr Glu Arg Gly Tyr Ser Phe Val Thr Thr Ala Glu Arg
   195 200 205
   Glu Ile Val Arg Asp Ile Lys Glu Lys Leu Cys Tyr Val Ala Leu Asp
   210 215 220
   Phe Glu Asn Glu Met Ala Thr Ala Ala Ser Ser Ser Ser Leu Glu Lys
   225 230 235 240
   Ser Tyr Glu Leu Pro Asp Gly Gln Val Ile Thr Ile Gly Asn Glu Arg
   245 250 255
   Phe Arg Cys Pro Glu Thr Leu Phe Gln Pro Ser Phe Ile Gly Met Glu
   260 265 270
   Ser Ala Gly Ile His Glu Thr Thr Tyr Asn Ser Ile Met Lys Cys Asp
   275 280 285
   Ile Asp Ile Arg Lys Asp Leu Tyr Ala Asn Asn Val Leu Ser Gly Gly
   290 295 300
   Thr Thr Met Tyr Pro Gly Ile Ala Asp Arg Met Gln Lys Glu Ile Thr
   305 310 315 320
   Ala Leu Ala Pro Ser Thr Met Lys Ile Lys Ile Ile Ala Pro Pro Glu
   325 330 335
   Arg Lys Tyr Ser Val Trp Ile Gly Gly Ser Ile Leu Ala Ser Leu Ser
   340 345 350
   Thr Phe Gln Gln Met Trp Ile Ser Lys Gln Glu Tyr Asp Glu Ala Gly
   355 360 365
   Pro Ser Ile Val His Arg Lys Cys Phe
   370 375
<210> 50
   <211> 267
   <212> PRT
   <213> Homo sapiens
<400> 50
<210> 51
   <211> 377
   <212> PRT
   <213> Homo sapiens
<400> 51
   Met Cys Asp Asp Glu Glu Thr Thr Ala Leu Val Cys Asp Asn Gly Ser
   1               5                   10                  15
   Gly Leu Val Lys Ala Gly Phe Ala Gly Asp Asp Ala Pro Arg Ala Val
               20                  25                  30
   Phe Pro Ser Ile Val Gly Arg Pro Arg His Gln Gly Val Met Val Gly
           35                  40                  45
   Met Gly Gln Lys Asp Ser Tyr Val Gly Asp Glu Ala Gln Ser Lys Arg
       50                  55                  60
   Gly Ile Leu Thr Leu Lys Tyr Pro Ile Glu His Gly Ile Ile Thr Asn
   65                  70                  75                  80
   Trp Asp Asp Met Glu Lys Ile Trp His His Thr Phe Tyr Asn Glu Leu
                   85                  90                  95
   Arg Val Ala Pro Glu Glu His Pro Thr Leu Leu Thr Glu Ala Pro Leu
               100                 105                 110
   Asn Pro Lys Ala Asn Arg Glu Lys Met Thr Gln Ile Met Phe Glu Thr
           115                 120                 125
   Phe Asn Val Pro Ala Met Tyr Val Ala Ile Gln Ala Val Leu Ser Leu
       130                 135                 140
   Tyr Ala Ser Gly Arg Thr Thr Gly Ile Val Leu Asp Ser Gly Asp Gly
   145                 150                 155                 160
   Val Thr His Asn Val Pro Ile Tyr Glu Gly Tyr Ala Leu Pro His Ala
                   165                 170                 175
   Ile Met Arg Leu Asp Leu Ala Gly Arg Asp Leu Thr Asp Tyr Leu Met
               180                 185                 190
   Lys Ile Leu Thr Glu Arg Gly Tyr Ser Phe Val Thr Thr Ala Glu Arg
           195                 200                 205
   Glu Ile Val Arg Asp Ile Lys Glu Lys Leu Cys Tyr Val Ala Leu Asp
       210                 215                 220
   Phe Glu Asn Glu Met Ala Thr Ala Ala Ser Ser Ser Ser Leu Glu Lys
   225                 230                 235                 240
   Ser Tyr Glu Leu Pro Asp Gly Gln Val Ile Thr Ile Gly Asn Glu Arg
                   245                 250                 255
   Phe Arg Cys Pro Glu Thr Leu Phe Gln Pro Ser Phe Ile Gly Met Glu
               260                 265                 270
   Ser Ala Gly Ile His Glu Thr Thr Tyr Asn Ser Ile Met Lys Cys Asp
           275                 280                 285
   Ile Asp Ile Arg Lys Asp Leu Tyr Ala Asn Asn Val Leu Ser Gly Gly
       290                 295                 300
   Thr Thr Met Tyr Pro Gly Ile Ala Asp Arg Met Gln Lys Glu Ile Thr
   305                 310                 315                 320
   Ala Leu Ala Pro Ser Thr Met Lys Ile Lys Ile Ile Ala Pro Pro Glu
                   325                 330                 335
   Arg Lys Tyr Ser Val Trp Ile Gly Gly Ser Ile Leu Ala Ser Leu Ser
               340                 345                 350
   Thr Phe Gln Gln Met Trp Ile Ser Lys Gln Glu Tyr Asp Glu Ala Gly
           355                 360                 365
   Pro Ser Ile Val His Arg Lys Cys Phe
       370                 375
<210> 52
   <211> 375
   <212> PRT
   <213> Homo sapiens
<400> 52
<210> 53
   <211> 471
   <212> PRT
   <213> Homo sapiens
<400> 53
<210> 54
   <211> 571
   <212> PRT
   <213> Homo sapiens
<400> 54